# EUROPEAN PATENT APPLICATION

(11) **EP 2 088 156 A1**
(43) Date of publication of application: **12.08.2009**
(21) Application number: 08300078.6
(22) Date of filing: 08.02.2008
(51) Int. Cl.: C07K 14/57

(54) **Use of adiponectin for the diagnosis and/or treatment of presbycusis**

(71) Applicant: INSTITUT PASTEUR, 75724 Paris Cédex 15 (FR)
(72) Inventor: Petit, Christine, 92350, LEe Plessis Robinson (FR); Lathrop, Mark, 75012, Paris (FR); Aubois, Anne, 92380, Garches (FR); Kubisch, Christian, 53113, Bonn (DE); Weil, Dominique, 75015, Paris (FR); Franco-Vidal, Valérie, 33700, Merignac (FR); Coez, Arnaud, 78600, Maisons Laffitte (FR); Sahel, José-Alain, 75013, Paris (FR)
(74) Representative: Blot, Philippe Robert Emile

(57) **Abstract**

The present invention relates to mutations located within the gene coding for adiponectin, said mutations being associated with presbycusis. The present invention further relates to adiponectin polynucleotides comprising such mutations, to adiponectin polypeptides encoded by such polynucleotides, and to methods of diagnosing and/or treating presbycusis using adiponectin polynucleotides, adiponectin polypeptides and/or ADIPOR2 polypeptides.

## Description

### FIELD OF THE INVENTION

The present invention is in the field of presbycusis. More specifically, the present invention relates to mutations located within the gene coding for adiponectin, said mutations being associated with presbycusis. The present invention further relates to adiponectin polynucleotides comprising such mutations, to adiponectin polypeptides encoded by such polynucleotides, and to methods of diagnosing and/or treating presbycusis using adiponectin polynucleotides, adiponectin polypeptides and/or receptors binding thereto.

### BACKGROUND OF THE INVENTION

### 1. Adiponectin

Adiponectin (Swiss-Prot entry Q15848), also referred to as ACRP30, apM-1 or ADIPOQ, is an important secreted protein involved in the control of fat metabolism and insulin sensitivity, with anti-diabetic, anti-atherogenic and anti-inflammatory activities. Adiponectin stimulates AMPK phosphorylation and activation in the liver and the skeletal muscle, enhancing glucose utilization and fatty-acid combustion. Adiponectin also antagonizes TNF-alpha by negatively regulating its expression in various tissues such as liver and macrophages, and also by counteracting its effects. Adiponectin further inhibits endothelial NF-kappa-B signaling through a cAMP-dependent pathway. Adiponectin may play a role in cell growth, angiogenesis and tissue remodeling by binding and sequestering various growth factors with distinct binding affinities, depending on the type of complex.

Adiponectin is a homomultimer forming trimers, hexamers and 12- to 18-mers. The trimers (low molecular weight complexes / LMW) are assembled via non-covalent interactions of the collagen-like domains in a triple helix and hydrophobic interactions within the globular C1q domain. Several trimers can associate to form disulfide-linked hexamers (middle molecular weight complexes / MMW) and larger complexes (higher molecular weight / HMW).

Adiponectin has been shown to bind Adiponectin receptor protein 2, Adiponectin receptor protein 1 and Cadherin-13. Predicted functional partners further include the Solute carrier family 2, the facilitated glucose transporter member 4, the Leptin precursor, the CNR1 Cannabinoid receptor 1, the TNF Tumor necrosis factor precursor, the APPL DCC-interacting protein 13 alpha, Interleukin-10 and angiotensinogen.

Defects in ADIPOQ are the cause of adiponectin deficiency. The result is a very low concentration of plasma adiponectin. Decreased adiponectin plasma levels are associated with obesity insulin resistance, and diabetes type 2.

Adiponectin has been shown to potentially play a role in diseases such as diabetes, obesity, cancer, cardiovascular diseases and neurogenerative disorders.

### 2. Presbycusis

Presbycusis, a sensorineural age-related hearing loss, affects one-third of the population after 65 years of age and 60% of the population between 70 and 80 years. Epidemiological data indicated environmental risk factors such as smoking habits, exposure to noise, to solvents and ototoxic medications. The impact of genetic factors has also been reported (Gates et al. 1999 Arch Otolaryngol Head Neck Surg. 125(6):654-9), the latter seeming to be more pronounced for individuals below 64 years.

Although there appears to be a genetic basis for the disease, very few genetic studies on presbycusis have been carried out. Genome-wide linkage studies resulted in a total of seven different candidate genomic regions for susceptibility to presbycusis, all with lod score inferior to 3, and no identified gene (DeStefano et al. Arch Otolaryngol Head Neck Surg. 2003 Mar;129(3):285-9.; Garringer et al. Arch Otolaryngol Head Neck Surg. 2006 May;132(5):506-10).

Association studies have also been carried out (Unal et al. Laryngoscope. 2005 Dec;115(12):2238-41; Van Eyken et al. Audiol Neurootol. 2007;12(6):345-58; Van Eyken et al. Hum Mutat. 2006 Oct;27(10):1007-16; O'Grady et al. Int J Audiol. 2007 Apr;46(4):183-6; Van Laer et al. Hum Mol Genet. 2008 Jan 15;17(2):159-69). NAT2, an enzyme thought to be involved in the detoxification of exogenic substrates by N- or O-acetylation, is the only gene which has been independently replicated so far.

There is thus a need to identify genes and mutations therein leading to a susceptibility to presbycusis, both in order to diagnose the disease and in order to develop powerful treatments.

### SUMMARY OF THE INVENTION

It has surprisingly been found that the chromosomal region located between 187910000 and 189120000 of human chromosome 3 and comprising mutations compared to the sequence found in normal subjects is associated with presbycusis with a highly significant lod score of 3.4. Three mutations have been found. These mutations are located within the gene coding for adiponectin, at positions 4988, 15361 and 16514 of SEQ ID NO: 1 respectively:

| Position on SEQ ID NO: 1 | Nucleotide found in normal subjects | Mutation found in individuals suffering from presbycusis |
|---|---|---|
| 4988 | T | C |
| 15361 | T | A |
| 16514 | G | A |

The alleles comprising these mutations code for novel, mutated adiponectin polypeptides. These mutations are predicted to lead to impaired multimerization and/or impaired secretion of adiponectin. Reduced levels of adiponectin are therefore believed to cause presbycusis.

The invention is directed to an isolated, purified or recombinant adiponectin polynucleotide comprising a sequence at least 80% identical to SEQ ID NO: 1, or to a fragment thereof, characterized in that said polynucleotide comprises a cytosine at position 4988 of SEQ ID NO: 1 and/or an adenosine at position 15361 of SEQ ID NO: 1.

The invention is further directed to an isolated, purified or recombinant adiponectin polypeptide comprising a sequence at least 80% identical to SEQ ID NO: 2, or to a fragment thereof, characterized in that said polypeptide comprises a glutamine at amino acid position 9 of SEQ ID NO: 2.

Another aspect of the invention is directed to the use of at least one adiponectin-related allelic marker for determining whether there is a genetic association between said allelic marker and presbycusis.

Still another aspect of the invention is directed to the use of at least one adiponectin-related allelic marker for diagnosing whether an individual suffers from or is at risk of suffering from presbycusis.

The invention is also directed to a method of genotyping comprising the steps of:
a) obtaining an isolated nucleic acid from a biological sample derived from an individual; and
b) detecting the nucleotide present at one or more of the adiponectin-related allelic markers of the invention shown in the table below:

| Allelic marker No. | Position on SEQ ID NO: 1 | Alternative alleles |
|---|---|---|
| 1 | 4988 | T/C |
| 2 | 15361 | T/A |
| 3 | 16514 | G/A |

The invention further pertains to probes comprising a fragment of the adiponectin gene, wherein said probe comprises allelic marker NO: 1 or 2 of the invention, and to primers comprising a fragment of the adiponectin gene, wherein the 3' end of said primer is located in the vicinity of, preferably within 500 nucleotides, of allelic marker NO: 1 or 2 of the table hereabove.

The invention also encompasses a pair of primers comprising a first and a second primer each comprising a fragment of an adiponectin gene, wherein:
- said first primer hybridizes to a first DNA strand of said adiponectin gene;
- said second primer hybridizes to the strand complementary to said first DNA strand; and
- the 3' ends of said first and second primers are located in the vicinity of allelic marker NO: 1 or 2 of the table hereabove.

The invention is further drawn to a method of identifying an ADIPOR2-related allelic marker likely to be involved in presbycusis comprising the steps of:
- determining the sequence of the ADIPOR2 gene present in biological samples from at least one individual suffering from presbycusis and from at least one unaffected individual; and
- comparing said sequences;
wherein a difference in said sequences is identified as an ADIPOR2-related allelic marker.

Another aspect of the invention is directed to an adiponectin polynucleotide of the invention, an adiponectin polypeptide of the invention or a fragment or an agonist thereof for use in the treatment of presbycusis.

The invention also relates to a method of determining whether an individual suffers from or is at risk of suffering from presbycusis comprising the steps of:
a) providing a biological sample from said individual; and
b) comparing:
   i. the amount of adiponectin polypeptide, or of an RNA species encoding said polypeptide, within said biological sample with an amount detected in or expected from a control sample; or
   ii. the activity of adiponectin polypeptide within said biological sample with an activity detected in or expected from a control sample;
wherein a reduced amount or activity of said adiponectin polypeptide or said RNA species within said biological sample compared to said level detected in, or expected from, said control sample indicates that said individual suffers from or is at risk of suffering from presbycusis.

### BRIEF DESCRIPTION OF THE SEQUENCES

SEQ ID NO: 1 - Nucleotide sequence of part of the gene coding for adiponectin. SEQ ID NO: 1 comprises the promoter, the introns and the exons of the adiponectin gene.

SEQ ID NO: 2 - Amino acid sequence of an adiponectin polypeptide.

SEQ ID NO: 3 - Amino acid sequence of the ADIPOR2 polypeptide.

SEQ ID NOs: 4-9 - Nucleotide sequences of primers used for amplifying fragments of the adiponectin gene.

SEQ ID NO: 10 - Nucleotide sequence of the gene coding for ADIPOR2.

SEQ ID NO: 11 - Nucleotide sequence of the regulatory region of the adiponectin gene (upstream of the ATG codon).

### DETAILED DESCRIPTION OF THE INVENTION

It has surprisingly been found that the chromosomal region located between 187910000 and 189120000 of chromosome 3 and comprising mutations compared to the sequence found in normal subjects is associated with presbycusis with a highly significant lod score of 3.4. The lod score is a statistical test used for linkage analysis. A lod score greater than 3.0 means that the likelihood of linkage between two allelic markers or a trait and a marker is 1000 times greater than no linkage.

Three mutations have been found. These mutations are located within the gene coding for adiponectin, at positions 4988, 15361 and 16514 of SEQ ID NO: 1 respectively:

| Position on SEQ ID NO: 1 | Nucleotide found in normal subjects | Mutation found in individuals suffering from presbycusis |
|---|---|---|
| 4988 | T | C |
| 15361 | T | A |
| 16514 | G | A |

The alleles comprising these mutations code for novel, mutated adiponectin polypeptides. These mutations are predicted to lead to impaired multimerization and/or impaired secretion of adiponectin. Reduced levels of adiponectin are therefore believed to cause presbycusis.

Therefore, a first aspect of the invention is directed to an isolated, purified or recombinant adiponectin polynucleotide comprising or consisting of a sequence at least 80%, 85%, 90%, 95%, 96%, 97%, 98% or 99% identical to SEQ ID NO: 1, or to a fragment of such a sequence, characterized in that said polynucleotide comprises a cytosine at position 4988 of SEQ ID NO: 1 and/or an adenosine at position 15361 of SEQ ID NO: 1. The adiponection polynucleotide may further comprise an adenosine at position 16514 of SEQ ID NO: 1.

As used herein, the term "adiponectin polynucleotide" refers to the adiponectin gene (including the 5' regulatory region, the promoter, the introns, the exons and the 3' regulatory region) and to fragments thereof. The adiponectin gene is located on chromosome 3 at 3q27, and is shown in Genbank accession number NC_000003.10 (188043157..188058946). SEQ ID NOs: 1 and 11 correspond to adiponectin polynucleotides. SEQ ID NO: 1 comprises the promoter, the introns and the exons of the adiponectin gene. SEQ ID NO: 11 corresponds to the regulatory region of the adiponectin gene (upstream of the ATG codon). The fragments may be of any length, e.g. at least 10, 25, 50, 100, 500 or 1000 nucleotides long. In one embodiment, the adiponectin polynucleotide encodes an adiponectin polypeptide. Adiponectin polynucleotides in accordance with the invention comprise a cytosine at position 4988 of SEQ ID NO: 1 and/or an adenosine at position 15361 of SEQ ID NO: 1.

In a preferred embodiment, the adiponectin polynucleotide comprises or consists of SEQ ID NO: 1, wherein said polynucleotide comprises a cytosine at position 4988 of SEQ ID NO: 1 and/or an adenosine at position 15361 of SEQ ID NO: 1.

In another preferred embodiment, the adiponectin polynucleotide further comprises SEQ ID NO: 11 or a fragment thereof, wherein said polynucleotide comprises a cytosine at position 4988 of SEQ ID NO: 1 and/or an adenosine at position 15361 of SEQ ID NO: 1.

A second aspect of the invention is directed to an isolated, purified or recombinant adiponectin polypeptide comprising or consisting of a sequence at least 80%, 85%, 90%, 95%, 96%, 97%, 98% or 99% identical to SEQ ID NO: 2, or to a fragment of such a sequence, characterized in that said polypeptide comprises a glutamine at amino acid position 9 of SEQ ID NO: 2. This polypeptide may further comprise a serine at amino acid position 90 of SEQ ID NO: 2.

The term "adiponectin polypeptide" refers to a polypeptide of SEQ ID NO: 2 and to splice variants, allelic variants, fragments, muteins and functional derivatives thereof having adiponectin biological activity. Specifically, the term "adiponectin polypeptide" includes polypeptides in which one or more of the amino acid residues of an adiponectin polypeptide of SEQ ID NO: 2 are replaced by different amino acid residues, or are deleted, or one or more amino acid residues are added to the wild-type sequence. As used herein, the term "wild type adiponectin polypeptide" refers to a polypeptide of SEQ ID NO: 2 wherein said polynucleotide comprises a leucine at position 9 of SEQ ID NO: 2 and a glycine at position 90 of SEQ ID NO: 2. Adiponectin polynucleotides in accordance with the invention comprise a glutamine at amino acid position 9 of SEQ ID NO: 2.

As used throughout this specification, the term "biological activity" of an adiponectin polypeptide refers to any of the known biological activities of adiponectin, such as e.g. an anti-diabetic, anti-obesity, anti-thrombotic, anticoagulant and/or anti-aggregant activity. An adiponectin polypeptide has biological activity as soon as it exhibits some biological activity as described hereabove, although the level may be reduced or impaired as compared to a wild-type adiponectin polypeptide. Methods of testing the biological activity of an adiponectin polypeptide are well-known in the art and include e.g. the methods disclosed in WO 01/51645 and WO 2007/147563.

A third aspect of the invention is directed to an expression vector comprising an adiponectin polynucleotide of the invention. Preferably, such an expression vector encodes an adiponectin polypeptide of the invention. Such expression vectors of the invention may be used e.g. to produce adiponectin polypeptides, or may correspond to vectors suitable for carrying out a gene therapy.

A fourth aspect of the invention is directed to a host cell comprising an expression vector of the invention. The host cell may correspond to any cell suitable for producing proteins or for maintaining viral vectors. Such cells may be e.g. a bacterial (e.g. *E. coli, S. lividans*)*,* a yeast (e.g. S. *cerevisiae*)*,* a fungal (e.g. *A. niger*), an insect, a mouse, a CHO, a monkey or a human cell.

A fifth aspect of the invention is directed to a method of making a polypeptide, said method comprising the steps of culturing a host cell of the invention under conditions suitable for the production of an adiponectin polypeptide of the invention within said host cell. Such conditions are well-known in the art. Preferably, the produced polypeptide is further purified. Most preferably, the polypeptide is further formulated into a pharmaceutical composition.

Since the chromosomal region located between 187910000 and 189120000 of chromosome 3 and comprising mutations compared to the wild-type sequence is associated with presbycusis with a highly significant lod score of 3.4, a sixth aspect of the invention is directed to the use of at least one adiponectin-related allelic marker for determining whether there is a genetic association between said allelic marker and presbycusis.

As used herein, the term "adiponectin-related allelic marker" refers to an allelic marker located within the adiponectin gene. Such an allelic marker may be located within the 5' or 3' regulatory gene regions, the promoter, the exons (including the 5' and the 3' UTRs) or the introns of the adiponectin gene. Preferably, such an allelic marker is located within SEQ ID NO: 1 or SEQ ID NO: 11. Most preferably, the adiponectin-related allelic marker is selected from the allelic markers shown in Table 1 herebelow:

**Table 1**

| Allelic marker No. | Position on SEQ ID NO: 1 | Alternative alleles |
|---|---|---|
| 1 | 4988 | T/C |
| 2 | 15361 | T/A |
| 3 | 16514 | G/A |

As used herein, the term "allelic marker" refers to a readily identifiable genetic element that exists either in a wild-type form or in a mutated form and is associated with a genetic trait of interest. In the frame of this invention, the genetic element corresponds to the existence of a mutation and encompasses e.g. substitutions, deletions and insertions of a nucleotide.

As used herein, there is a "genetic association" between an allelic marker or a combination of allelic markers and a disease when a particular allele, genotype or haplotype of said allelic marker or combination of allelic markers is seen more often than expected by chance in an individual carrying the disease.

As used herein, the term "presbycusis" refers to age-related hearing loss, and encompasses all conditions that lead to hearing loss in elderly people (see e.g. Gates and Mills, Lancet. 2005 Sep 24-30;366(9491):1111-20). The disorder is characterized by reduced hearing sensibility and speech understanding in noisy environment, slowed central processing of acoustic information, and/or impaired localisation of acoustic sources. An individual is considered to suffer from presbycusis when its hearing threshold is increased above the threshold values shown in figure 1. The presbycusis may correspond to a "familial case of presbycusis", i.e. to an inheritable form of the disease. Preferably, an individual being a familial case of presbycusis satisfies the criteria of having (i) at least one affected brother or sister; and (ii) one or none affected mother or father. Alternatively, the presbycusis may correspond to a sporadic case of presbycusis, i.e. none of the brothers and sisters of the affected individual suffers from presbycusis.

Methods for determining whether there is a genetic association between said allelic marker and presbycusis are well-known in the art.

A preferred embodiment is directed to a method of determining whether there is a genetic association between at least one adiponectin-related allelic marker and presbycusis comprising the steps of:
a) providing biological samples from individuals suffering from presbycusis and from unaffected individuals;
b) determining the identity of a nucleotide present at said allelic marker; and
c) comparing the allele frequency of said allelic marker in individuals suffering from presbycusis with the allele frequency of said allelic marker in unaffected individuals.
wherein a difference in the allele frequency of said allelic marker in individual suffering from presbycusis and in unaffected individuals is indicative of a genetic association between said allelic marker and presbycusis.

The expression "identity of a nucleotide present at a allelic marker" refers to the nucleotide present at said allelic marker and may correspond to A, C, G or T.

Preferably, biological samples are collected from at least 2, 5, 10, 25, 50, 75 or 100 individuals suffering from presbycusis. More preferably, biological samples are collected from at least 5, 10, 25, 50, 75 or 100 unaffected individuals. Most preferably, the allele frequencies are statistically compared in step (c).

As the adiponectin allele comprising mutations at positions 4988, 15361 and/or 16514 of SEQ ID NO: 1 is associated with presbycusis, the invention further relates to methods for diagnosing presbycusis.

A seventh aspect of the invention is directed to the use of at least one adiponectin-related allelic marker for diagnosing whether an individual suffers from or is at risk of suffering from presbycusis. Preferably, said adiponectin-related allelic marker is selected from the allelic markers shown in Table 1 hereabove. In a specific embodiment, the detection of a mutated adiponectin allele comprising a C at position 4988, an A at position 15361 and/or an A at position 16514 of SEQ ID NO: 1 in a biological sample collected from an individual indicates that said individual suffers from or is at risk of suffering from presbycusis.

An eight aspect of the invention is directed to method of genotyping comprising the steps of:
a) obtaining an isolated nucleic acid from a biological sample derived from an individual; and
b) detecting the nucleotide present at one or more of the adiponectin-related allelic markers shown in table 1 hereabove.

Preferably, said biological sample is derived from a single individual. It is preferred that the nucleotide present at one or more of said adiponectin-related allelic markers is detected for both copies of said allelic markers present in said individual's genome. In a preferred embodiment, the nucleotide present at one or more of said adiponectin-related allelic markers is detected by a sequencing assay. Preferably, a portion of a sequence comprising the allelic marker is amplified prior to the determination of the identity of the nucleotide. The amplification may preferably be performed by PCR.

Any well-known method of genotyping may be used in the frame of the present invention. Such methods include methods such as e.g. conventional dot blot analyzes, single strand conformational polymorphism analysis, denaturing gradient gel electrophoresis, heteroduplex analysis and mismatch cleavage detection. Another method for determining the identity of the nucleotide present at a particular polymorphic site employs a specialized exonuclease-resistant nucleotide derivative as described in U.S. Pat. No. 4,656,127. Oligonucleotide microarrays or solid-phase capturable dideoxynucleotides and mass spectrometry may also be used. Preferred methods involve directly determining the identity of the nucleotide present at an allelic marker site by sequencing assay, enzyme-based mismatch detection assay, or hybridization assay.

The term "genotyping" a sample or an individual for an allelic marker involves determining the specific allele or the specific nucleotide carried by an individual at an allelic marker.

In a preferred embodiment, the method of genotyping of the invention further comprises the step of correlating the result of the genotyping steps with a risk of suffering from presbycusis and/or the step of diagnosing presbycusis.

In a most preferred embodiment, the presence of:
a) a C at position 4988 of SEQ ID NO: 1;
b) an A at position 15361 of SEQ ID NO: 1; and/or
c) an A at position 16514 of SEQ ID NO: 1;
indicates that said individual suffers from or is at risk of suffering from presbycusis.

The present invention is also directed to a method of identifying an adiponectin-related allelic marker likely to be involved in presbycusis comprising the steps of:
- determining the sequence of an adiponectin gene or a fragment thereof present in said samples from at least one individual suffering from presbycusis and from at least one unaffected individual; and
- comparing said sequences;
wherein a difference in said sequences is identified as an adiponectin-related allelic marker.

The difference preferably corresponds to a mutation present in said at least one individual suffering from presbycusis. More preferably, the difference corresponds to a mutation that is more frequently found in individuals suffering from presbycusis than in unaffected individuals. Most preferably, said sequence is determined for the region corresponding to SEQ ID NO: 1 and/or SEQ ID NO: 11, and the identified adiponectin-related allelic marker is located within SEQ ID NO: 1 or SEQ ID NO: 11.

The invention further relates to probes and primers useful for detecting the mutated adiponectin allele of the invention.

A ninth aspect of the invention is therefore directed to an isolated, purified or recombinant primer or probe comprising a fragment of the adiponectin gene, wherein said primer or probe comprises allelic marker NO: 1 or 2 of table 1 hereabove. Said probe preferably comprises a cytosine at position 4988 of SEQ ID NO: 1 and/or an adenosine at position 15361 of SEQ ID NO: 1.

Said fragment of an adiponectin gene may consist of a contiguous span of at least 12, 15, 20, 25, 30, 35, 40, 45, 50, 60, 70, 80, 90 or 100 nucleotides of SEQ ID NO: 1 or the complementary sequence thereof. The length of said primer or probe may be within a range of 12-100, 15-90, 20-80, 25-70, 30-60, 35-50 and 15-50 nucleotides.

Such primers or probes are used e.g. in methods of determining whether an individual carries a C at position 4988 or an A at position 15361 of SEQ ID NO: 1. For example, the probe may be used in mismatch cleavage detection or in hybridization assays for genotyping an adiponectin-related allelic marker of the invention.

In a preferred embodiment, the contiguous span of said primer or probe is 12 to 50 nucleotides in length and said allelic marker is within 4 nucleotides of the centre of said primer or probe.

In another preferred embodiment, said primer or probe consists of said contiguous span and said contiguous span is 25 nucleotides in length and said allelic marker is at the centre of said primer or probe.

In still another preferred embodiment, said allelic marker is present at the 3' end of said primer or probe. In the context of this embodiment, the 3' end of said contiguous span is preferably located at the 3' end of said probe.

A tenth aspect of the invention is directed to an isolated, purified or recombinant primer comprising a fragment of the adiponectin gene. Preferably, the primer comprises or consists of a fragment of SEQ ID NO: 1 or SEQ ID NO: 11. Most preferably, the 3' end of said primer is located in the vicinity of allelic marker NO: 1 or 2 of table 1 hereabove.

The 3' end of a primer is located "in the vicinity" of an allelic marker if said 3' end is located within 4000, 3000, 2000, 1000, 500, 400, 300, 250, 200, 100, 50, 25, 10 or 1 nucleotides of said allelic marker. The 3' end of said primer is preferably located upstream (i.e. toward the 5' end) of said biallelic marker. Alternatively, the primer may comprise the biallelic marker.

Said fragment of an adiponectin gene may consist of a contiguous span of at least 12, 15, 20, 25, 30, 35, 40, 45, 50, 60, 70, 80, 90 or 100 nucleotides of SEQ ID NO: 1 or SEQ ID NO: 11 or the complementary sequence thereof. The length of said primer may be within a range of 12-100, 15-90, 20-80, 25-70, 30-60, 35-50 and 18-25 nucleotides.

Such primers are used e.g. in methods of determining whether an individual carries a C at position 4988 or an A at position 15361 of SEQ ID NO: 1. For example, the primer may be used in sequencing assays for genotyping an adiponectin-related allelic marker of the invention or in PCR reactions for amplifying a fragment of an adiponectin polynucleotide comprising an allelic marker of the invention.

In a preferred embodiment, the 3' end of said primer is located within 1 nucleotide of said allelic marker.

In another preferred embodiment, said primer is selected from the group consisting of SEQ ID NOs: 4-7. Such primers typically find use in PCR reactions for amplifying a fragment of an adiponectin polynucleotide comprising allelic marker NO:1 or 2 of the invention.

An eleventh aspect of the invention is directed to a pair of primers comprising a first and a second primer each comprising a fragment of an adiponectin gene, wherein:
- said first primer hybridizes to a first DNA strand of said adiponectin gene;
- said second primer hybridizes to the strand complementary to said first DNA strand; and
- the 3' ends of said first and second primers are located in the vicinity of allelic marker NO: 1 or 2 of the table set forth in claim 5.

In a preferred embodiment; said pair of primers comprises:
- a first primer comprising a contiguous span of at least 12, 15, 20, 25, 30, 35, 40, 45, 50, 60, 70, 80, 90 or 100 nucleotides of SEQ ID NO: 1; and
- a second primer comprising a contiguous span of at least 12, 15, 20, 25, 30, 35, 40, 45, 50, 60, 70, 80, 90 or 100 nucleotides of a sequence complementary SEQ ID NO: 1.

The 3' ends of said first and second primers are preferably located within 500, 400, 300, 250, 200, 100 or 50 nucleotides of the allelic marker. Said first and second primers are preferably located upstream of the biallelic marker. The length of said primer may be within a range of 12-100, 15-90, 20-80, 25-70, 30-60, 35-50 and 18-25 nucleotides.

In a preferred embodiment, said pair of primers consists of SEQ ID NOs: 4 and 5, or of SEQ ID NOs: 6 and 7.

Such pairs of primers are used e.g. in methods of determining whether an individual carries a C at position 4988 or an A at position 15361. Typically, such pairs of primers are used in PCR reactions for amplifying a fragment of an adiponectin polynucleotide comprising allelic marker NO: 1 or 2 of the invention.

The primers of the present invention may optionally comprise sequences in addition to the contiguous span of at least 12 nucleotides of SEQ ID NO: 1 or the sequence complementary thereto such as e.g. a restriction site. In this case, the contiguous span homologous to SEQ ID NO: 1 is located at the 3' end of said primer.

The primers are optionally labelled. They may be labelled either with a radioactive or with a non-radiaoctive compound such as e.g. biotin.

A twelfth aspect of the invention is directed to a microarray comprising at least one probe according to the invention or at least a primer according to the invention.

A thirteenth aspect of the invention is directed to a diagnostic kit comprising at least one probe according to the invention or at least a primer according to the invention.

ADIPOR2 being a receptor for adiponectin, a fourteenth aspect of the invention is directed to a method of identifying an ADIPOR2-related allelic marker likely to be involved in presbycusis comprising the steps of:
- providing biological samples from at least one individual suffering from presbycusis and from at least one unaffected individual;
- determining the sequence of an ADIPOR2 gene or a fragment thereof present in said samples; and
- comparing said sequences;
wherein a difference in said sequences is identified as an ADIPOR2-related allelic marker. The difference preferably corresponds to a mutation present in said at least one individual suffering from presbycusis. More preferably, the difference corresponds to a mutation that is more frequently found in individuals suffering from presbycusis than in unaffected individuals.

Preferably, said biological samples are collected from at least 5, 10, 25, 50, 75 or 100 individual suffering from presbycusis and from at least 5, 10, 25, 50, 75 or 100 unaffected individual.

As used herein, the term "ADIPOR2" refers to the Progestin and adipoQ receptor family member II, the sequence of which is shown as SEQ ID NO: 3. An "ADIPOR2-related allelic marker" refers to an allelic marker that is located within the ADIPOR2 gene. The term "ADIPOR2 gene" refers to the gene encoding ADIPOR2, including the 5' and 3' regulatory regions, the promoter, the exons and the introns. The ADIPOR2 gene is located on chromosome 12 at 12p13.31. The sequence of the ADIPOR2 gene is shown as SEQ ID NO: 10 and in Genbank accession number NC_000012.10 (1670508..1768106).

A further embodiment of the invention is directed to a method of determining whether there is a genetic association between at least one ADIPOR2-related allelic marker and presbycusis comprising the steps of:
a) providing biological samples from individuals suffering from presbycusis and from unaffected individuals;
b) determining the identity of a nucleotide present at said allelic marker; and
c) compare the allele frequency of said allelic marker in individual suffering from presbycusis with the allele frequency of said allelic marker in unaffected individual.
Wherein a difference in the allele frequency of said allelic marker in individual suffering from presbycusis and in unaffected individuals is indicative of a genetic association between said allelic marker and presbycusis.

Preferably, said biological samples are collected from at least 2, 5, 10, 25, 50, 75 or 100 individual suffering from presbycusis and from at least 2, 5, 10, 25, 50, 75 or 100 unaffected individual.

Since reduced adiponectin levels are believed to cause presbycusis, a fifteenth aspect of the invention is directed to an adiponectin polynucleotide of the invention for use in the treatment of presbycusis. In the context of this aspect, the adiponectin polynucleotide is typically present on a gene therapy vector, and is administrated to the individual in the frame of a gene therapy. Also in the context of this aspect, the adiponectin polynucleotide corresponds to a functional (wild-type) adiponectin polynucleotide. It preferably comprises a tyrosine at position 4988, a tyrosine at position 15361, and a guanosine at position 16514 of SEQ ID NO: 1.

A preferred embodiment is directed to a method of treating presbycusis comprising the step of administering an adiponectin polynucleotide to a patient suffering from presbycusis.

A sixteenth aspect of the invention is directed to an adiponectin polypeptide or a fragment or an agonist thereof of the invention for use in the treatment of presbycusis. In the frame of this aspect, the adiponectin polypeptide corresponds to a functional (wild-type) adiponectin polypeptide. It preferably comprises a leucine at position 9 and a glycine at position 90 of SEQ ID NO: 2.

An "agonist of adiponectin" refers to a compound that increases the activity of an adiponectin polypeptide in a cell or in an individual, a compound increasing adiponectin plasma levels in an individual, and/or a compound that increases the expression level of the adiponectin mRNA in a cell. The agonist may be e.g. a natural ligand, a small molecule, an agonistic antibody or an aptamer. Adiponectin agonists are known in the art and include e.g. thiazolidinediones (Iwaki et al. Diabetes. 2003 Jul;52(7):1655-63), gliclazide (Drzewoski and Zurawska-Klis, Curr Med Res Opin. 2006 Oct;22(10):1921-6), quinapril (Hermann et al. J Clin Endocrinol Metab. 2006 Mar;91(3):1001-8) and rosiglitazone (Yang et al. Diabetes Care. 2002 Feb;25(2):376-80),

A "fragment of adiponectin" refers to a fragment of an adiponectin polypeptide having adiponectin biological activity. Such fragments are known in the art and may e.g. correspond to a globular Adiponectin, i.e. to a fragment of Adiponectin comprising the C1q domain but not the collagen domain. The globular Adiponectin may for example correspond to any of the globular Adiponectins disclosed in Fruebis et al. (Proc Natl Acad Sci U S A. 2001 Feb 13;98(4):2005-10), WO 01/92330 or WO 01/51645.

A preferred embodiment is directed to a method of treating presbycusis comprising the step of administering an adiponectin polypeptide or a fragment or an agonist thereof to a patient suffering from presbycusis.

As ADIPOR2 is a receptor for adiponection, a seventeenth aspect of the invention is directed to an ADIPOR2 agonist for use in the treatment of presbycusis. The agonist may be e.g. a natural ligand, a small molecule, an agonistic antibody or an aptamer. One example of such an ADIPOR2 agonist is its natural ligand Adiponectin.

A preferred embodiment is directed to a method of treating presbycusis comprising the step of administering an ADIPOR2 agonist to a patient suffering from presbycusis.

An eighteenth aspect of the invention is directed to a method of determining whether an individual suffers from or is at risk of suffering from presbycusis comprising the steps of:
a) providing a biological sample from said individual;
b) comparing:
   i. the amount of adiponectin polypeptide, or of an RNA species encoding said polypeptide, within said biological sample with an amount detected in or expected from a control sample; or
   ii. the activity of adiponectin polypeptide within said biological sample with an activity detected in or expected from a control sample;
wherein a reduced amount or activity of said adiponectin polypeptide or said RNA species within said biological sample compared to said level detected in or expected from said control sample indicates that said individual suffers from or is at risk of suffering from presbycusis.

All references cited herein, including journal articles or abstracts, published or unpublished patent application, issued patents or any other references, are entirely incorporated by reference herein, including all data, tables, figures and text presented in the cited references.

Although having distinct meanings, the terms "comprising", "having" and "consisting of' have been used interchangeably throughout this specification and may be replaced with one another.

The invention will be further evaluated in view of the following examples and figures.

### EXAMPLES

### Example 1: Enrolment of patients suffering from presbycusis

The inclusion criteria for patients suffering from familial cases of presbycusis were the following:

### 1. Clinical criteria:

- 40 years old at least at the time of diagnosis - symmetrical sensorineural hearing loss with normal impedancemetry.
- Shape of the audiometric curve: slope on high frequencies.
- Hearing loss can be associated with tinnitus.
- Thresholds value for the patient to be considered affected are reported in Table 2 herebelow. These are calculated on the mean of hearing thresholds in bone conduction on 500, 1000, 2000 et 4000 Hz (function of age and gender) on the best ear.

### 2. Genetic criteria:

- The patient must have at least 1 brother and/or sister affected +/- his children. Father or mother can be affected but not both.

Non inclusion criteria were the following:
- Neurological disease at the origin of the hearing loss
- Syndromic deafness
- Diabetes mellitus treated by oral antidiabetics or insuline
- Bilateral chronic otitis, bilateral cholesteatoma
- Vestibular schwannoma
- Noise induced hearing loss
- Ischemic cardiovascular disease

Table 2 shows the tresholds value for the patient to be considered affected. The values correspond to the mean of hearing thresholds in bone conduction on 500, 1000, 2000 and 4000 Hz (function of age and gender). The hearing threshold of affected patients are increased above the values observed in the 25% of the individuals of the same age and gender. Only individuals older than 40 years of age at the time of the diagnosis have been included in the study.(European standard AFNOR 7029:2000-08).

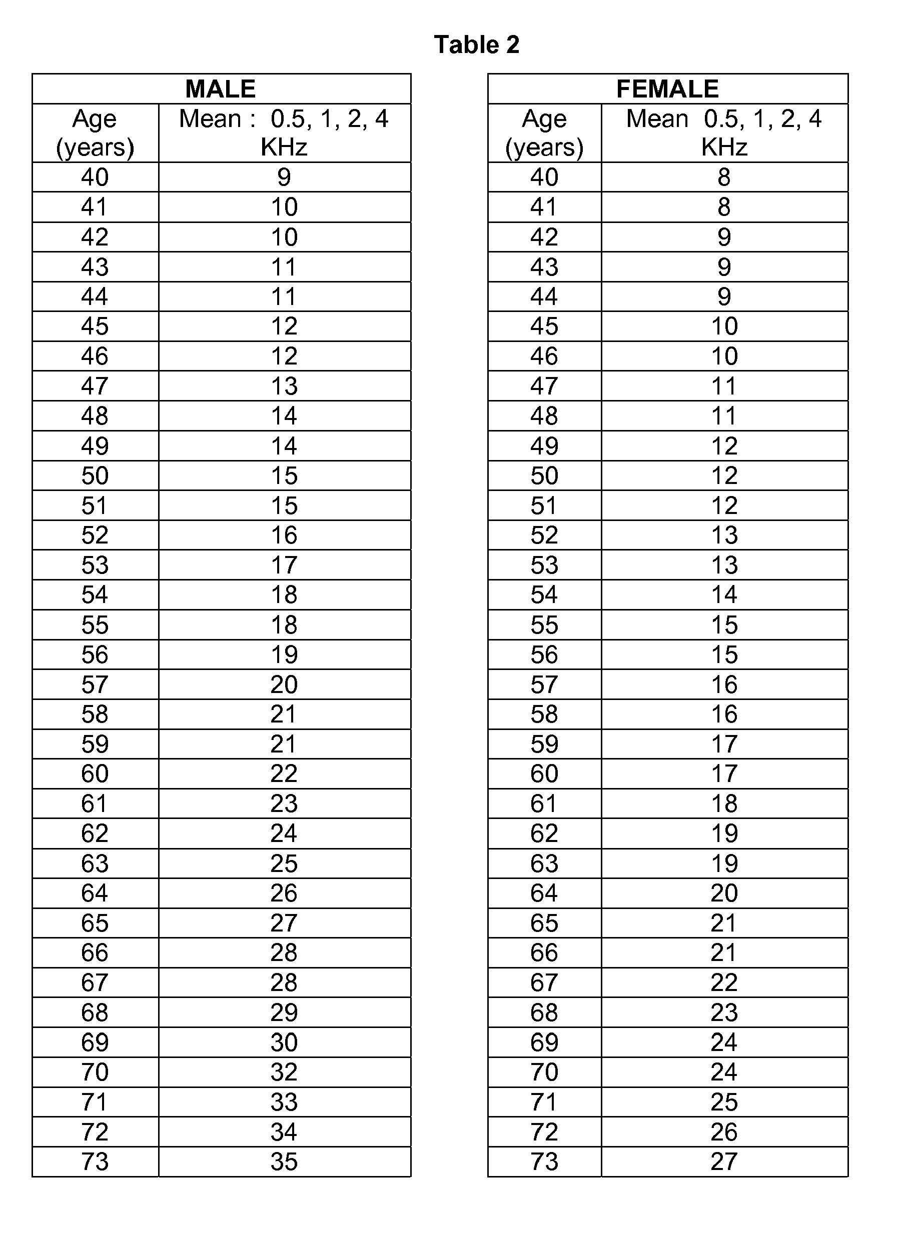

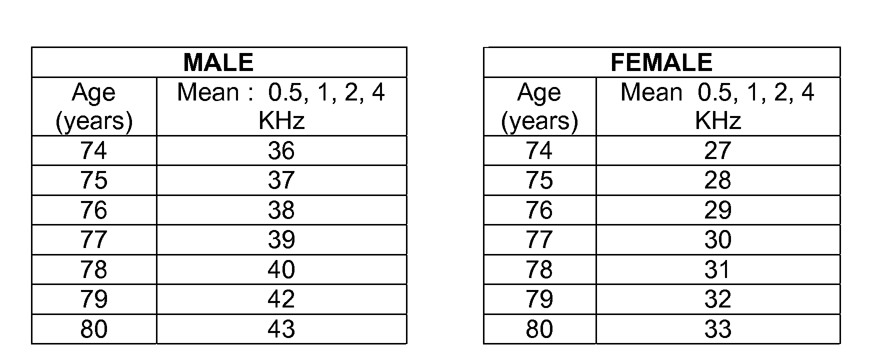

### Example 2: Linkage analysis

The lod score is a statistical test used for linkage analysis. A lod score greater than 3.0 means that the likelihood of linkage between two allelic markers or a trait and a marker is 1000 times greater than no linkage.

The linkage analysis was carried out using DNA samples collected from 85 families (180 individuals).

DNA was genotyped with the over 300,000 single nucleotide polymorphisms (SNPs or markers) using the Illumina Human Hap300 SNP array. Genotype data were subjected to standard QC procedures used at the CNG (National Genotyping Centre), and samples with less than 95% completion rate (less than 95% of successfully genotyped markers) were eliminated from further consideration. Similarly, markers with less than 95% success rate across the remaining samples, or that showed extreme Hardy-Weinberg distortion were eliminated from further analysis. Sex and pedigree relationships were verified against the data recorded in the medical files established.

For linkage analysis, a subset of markers was chosen that showed low pairwise linkage disequilibrium (LD) in order to eliminate or reduce the upwards bias in lod scores that can be produced by marker LD when not all parents have been genotyped. Non-parametric and parametric affected-only lod scores were calculated with the subset of markers with the recorded sex/pedigree data and the recorded affected status using standard methods (as implemented in the program MERLIN). Parametric affected-only lod scores were calculated under recessive and dominant models with incomplete penetrance.

In the two instances in which lod scores > 3 were detected with the non-parametric and dominant affected-only calculations, the 1-lod unit intervals was used to define the region likely to contain the putative susceptibility loci. These chromosomal regions were evaluated for association, taking into account the linkage results, by using standard methods (as implemented in the program LAMP).

### Example 3: Results

A lod score of 3.4 was found for the chromosomal region located between 187910000 and 189120000 of human chromosome 3 and comprising three mutations as indicated in table 3 below.

**Table 3**

| Position on SEQ ID NO: 1 | Alternative alleles | Allele most frequently found in affected individuals | Location within the adiponectin gene | Mutation in the corresponding protein |
|---|---|---|---|---|
| 4988 | T/C | C | 5' UTR | n/a |
| 15361 | T/A | A | Coding sequence | Leu9Gln |
| 16514 | G/A | A | Coding sequence | Gly90Ser |

The Gly90Ser mutation is known to cause impaired multimerization and/or the consequent impaired secretion of adiponectin (Waki et al. J Biol Chem. 2003 Oct 10;278(41):40352-63). The Leu9Gln mutation is located in the signal peptide, and may thus lead to impaired secretion of adiponectin as well. Therefore, it is believed that reduced levels of adiponectin cause presbycusis. Presbycusis may thus be treated by administering adiponectin or an agonist thereof. Alternatively, presbycusis may be treated by administering an agonist of ADIPOR2, which is one of the receptors of adiponectin.

## Claims

1. An isolated, purified or recombinant adiponectin polynucleotide comprising a sequence at least 80% identical to SEQ ID NO: 1 or a fragment of said sequence, **characterized in that** said polynucleotide comprises:
a) a cytosine at position 4988 of SEQ ID NO: 1; and/or
b) an adenosine at position 15361 of SEQ ID NO: 1.

2. An isolated, purified or recombinant adiponectin polypeptide comprising a sequence at least 80% identical to SEQ ID NO: 2 or a fragment of said sequence, **characterized in that** said polypeptide comprises a glutamine at amino acid position 9 of SEQ ID NO: 2.

3. Use of at least one adiponectin-related allelic marker for determining whether there is a genetic association between said allelic marker and presbycusis.

4. Use of at least one adiponectin-related allelic marker for diagnosing whether an individual suffers from or is at risk of suffering from presbycusis.

5. Use according to claim 3 or 4, wherein said adiponectin-related allelic marker is selected from the group consisting of the adiponectin-related allelic markers shown in the table below:
| Allelic marker No. | Position on SEQ ID NO: 1 | Alternative alleles |
|---|---|---|
| 1 | 4988 | T/C |
| 2 | 15361 | T/A |
| 3 | 16514 | G/A |

6. A method of genotyping comprising the steps of:
a) obtaining an isolated nucleic acid from a biological sample derived from a single individual; and
b) detecting the nucleotide present at one or more of the adiponectin-related allelic markers shown in the table set forth in claim 5.

7. The method of claim 6, further comprising the step of correlating the result of the genotyping steps with a risk of suffering from presbycusis and/or diagnosis of presbycusis.

8. The method of claim 7, wherein the presence of:
a) a C at position 4988 of SEQ ID NO: 1;
b) an A at position 15361 of SEQ ID NO: 1; and/or
c) an A at position 16514 of SEQ ID NO: 1.
indicates that said individual suffers from or is at risk of suffering from presbycusis.

9. An isolated, purified or recombinant probe comprising a fragment of an adiponectin gene, wherein said probe comprises a cytosine at position 4988 of SEQ ID NO: 1 and/or an adenosine at position 15361 of SEQ ID NO: 1.

10. The probe of claim 9, wherein said fragment consists of a contiguous span of at least 12 nucleotides of SEQ ID NO: 1.

11. An isolated, purified or recombinant primer comprising a fragment of an adiponectin gene.

12. The primer of claim 11, wherein the 3' end of said primer is located within 500 nucleotides of allelic marker NO: 1 or 2 of the table set forth in claim 5.

13. The primer of claim 11 or 12, wherein said fragment consists of a contiguous span of at least 12 nucleotides of SEQ ID NO: 1.

14. A primer according to any of claims 11 to 13, wherein the 3' end of said polynucleotide is located:
i) at said biallelic marker; or
ii) one nucleotide upstream of said allelic marker.

15. A pair of primers comprising a first and a second primer each comprising a fragment of an adiponectin gene, wherein:
a) said first primer hybridizes to a first DNA strand of said adiponectin gene;
b) said second primer hybridizes to the strand complementary to said first DNA strand; and
c) the 3' ends of said first and second primers are located in the vicinity of allelic marker NO: 1 or 2 of the table set forth in claim 5.

16. The pair of primers of claim 15, wherein:
a) said first primer comprises a contiguous span of at least 12 nucleotides of SEQ ID NO: 1; and
b) a second primer comprises a contiguous span of at least 12 nucleotides of a sequence complementary to SEQ ID NO: 1.

17. The pair of primers of claim 16, wherein said pair of primers consists of:
i) SEQ ID NOs: 4 and 5; or
ii) SEQ ID NOs: 6 and 7.

18. A method of identifying an ADIPOR2-related allelic marker likely to be associated with presbycusis comprising the steps of:
a) providing biological samples from at least one individual suffering from presbycusis and from at least one unaffected individual;
b) determining the sequence of an ADIPOR2 gene or a fragment thereof present in said samples; and
c) comparing said sequences;
wherein a difference in said sequences is identified as an ADIPOR2-related allelic marker.

19. The method of claim 18, further comprising the step of determining whether there is a genetic association between said ADIPOR2-related allelic marker and presbycusis.

20. An adiponectin polynucleotide for use in the treatment of presbycusis.

21. An adiponectin polypeptide or an agonist or a fragment thereof for use in the treatment of presbycusis.

22. An ADIPOR2 agonist for use in the treatment of presbycusis.

23. A method of determining whether an individual suffers from or is at risk of suffering from presbycusis comprising the steps of:
a) providing a biological sample from said individual; and
b) comparing:
ii. the amount of adiponectin polypeptide, or of an RNA species encoding said polypeptide, within said biological sample with an amount detected in or expected from a control sample; or
iii. the activity of adiponectin polypeptide within said biological sample with an activity detected in or expected from a control sample;
wherein a reduced amount or activity of said adiponectin polypeptide or said RNA species within said biological sample compared to said level detected in or expected from said control sample indicates that said individual suffers from or is at risk of suffering from presbycusis.


SEQUENCE LISTING

<110> INSTITUT PASTEUR

<120> USE OF ADIPONECTIN FOR THE DIAGNOSIS AND/OR TREATMENT OF
PRESBYCUSIS

<130> BET07P1548

<160> 11

<170> PatentIn version 3.4

<210> 1
<211> 20840
<212> DNA
<213> Homo sapiens

<220>
<221> misc_feature
<222> (4951)..(5026)
<223> exon 1

<220>
<221> allele
<222> (4988)..(4988)
<223> T/C polymorphism

<220>
<221> misc_feature
<222> (15328)..(15549)
<223> exon 2

<220>
<221> allele
<222> (15361)..(15361)
<223> T/A polymorphism

<220>
<221> misc_feature
<222> (16461)..(20740)
<223> exon 3

<220>
<221> allele
<222> (16514)..(16514)
<223> G/A polymorphism

<400> 1
tggcacaatc tcggctcact acaacctttg cctcctgggt tcaagtgatt ctcctgcctc 60

agcctcccaa gtagctggga ttacaagtgc gtgccaccac acctggccaa ttttgtattt 120

ttagtagaga tggggtttca ccatgttggc cgggctggtc tccaactcct gacctcagct 180

gatctgcctg cctcagcctc ccaaagtgct gtaattatag gcataagcca ctgtgcctgg 240

cctagtgttg tacattctgt gggttttgac aattgtatgc atctacatgt atgtaccatt 300

atagtattcc tgtttttaat tttagccatt ctagtaggca tgtagtgata tctcatggtg 360

attttaattt gcgtttccgt aatggttaat aatgctgaac atctttgcat gtgcttgttt 420
gtcatttgtg tttcctactt ggtgaaataa ttgttcatgt cctttgtcca ttttctaatt 480
gaattttttt ttaccattta gttttgagat ttctttatac aatctagatc caaatctctt 540
gtctcaaata tggtttgcaa atacattcct ctaattcata tattgccttt tcctcctctt 600
aacaggatgt ttcacagagc aaaagtttta gttttgttga aatctcactt ttcatttttt 660
tctttagtgg attgtgcttt tgttgtcata tgtaagaact cttcactggc cctagatcct 720
tgtattggtt tcctaagatt gccatagcaa atcaccatga acttagtgac aaaaagacag 780
aaatttattt tcacttccta ctgtgggcag actagacgtt aattattttc atgtatgctc 840
attcctatga catctttctg atataataat tatagttatt cttaagcttc accctttttt 900
ctattagctt tgttaccttg ggtgtcactt tttctttttt gacattgtga cctatgccag 960
atcatgtctg ttagtactta gccctccatt cacctctcca taatcccttt tgtattcctg 1020
gagcttgatg cctgaaatga cacatcctac attcctttgc cagatgggta ccagttagct 1080
tgtgcacatg ggagacaacc gtgaaaagac tgaagtgggg aagaagggag gagctgttgt 1140
gtttcagtga gcgcccttgg cagtggcgct gacagtggct cctgttcagt ggcaatggtg 1200
gagcagctag caagacatgc agtaagcgca ggctcatagg ctatggtcca ggagcagtca 1260
ccgattcctg gtctttaggc aatatcatct ccctttgctt ctccagcctt tctaaaatta 1320
ttgtaccttg actagtacaa ttttttagta ttgggggtag tccaaggaca caggctttaa 1380
aaagtatgaa ttcagggttg cctacctgca ttgactgcgc ttgaatcatg atggccttct 1440
ggtcggtggc aggaggtgac agtccaaatc atgcagtagc aaaccagata cttaaattat 1500
catctgagat acttcagaag tacagccgta gccatacctt cagaagagat aaagaaatgt 1560
tctcctggcc aggcgcggtg gctcacgcct gtcattccag cactttggga ggccgagggg 1620
gtggatcacc tgaggtcggg agttcgagac cagcctgacc aacatgggga aaccctgtct 1680
ctactaaaaa tacaaaatta gcggggcgtg gtggcacatg cccataatcc cagctactcg 1740
ggaggctaag gcaggataat cgcttgaacc tgagaggcag aggttgcggt gaactgagat 1800
catgccatag tactccagcc tgggcaacaa gagtgaaact ccatctcaaa aaaaaaaaaa 1860
aagaaaaaaa gataaagaaa tgttctcctt tcttgccatt tctaggggtt tggggatggc 1920
gtacattgct gcagggcgtg ctcactctac catcttgctc caatctttat ttttcaaaat 1980
acagtgctta tgcttggtta cttcagttaa gattattttt aaaaatcata attaagcaaa 2040
aatatatggc catgcttaaa catatttaag ataaattaag tgatttggcc tgtttcagta 2100
tcccaactca catgctaaca ggggcttgac ctgtagctac ggtaccctgg aggaaatgat 2160

cgcatttatt tggttatttc ggtctaagta gtaatagttc tgtcctggga aaaagactag 2220

cctcaaggca tttctgattg aatgtttttc aattacagtc tttaaaccag tatgccacag 2280

aactggctct ttccacatga cggcctttgt ggtgggtggc agattgccct gaggcctcgc 2340

aaaatgctag gctttcacaa tgtcactgac tgacagccag gcccagcaca gtcttggtgt 2400

gattgtgggg ctaaagttat tccaccttgt gcaatagcta cagctttctc taaccagctg 2460

cattcttata aagttagaag aaaatacttt tttttttttg agatggattc tcgctctgtt 2520

gcccaggctg gagtgcaatg gtgcgatctc ggctcgctgc aacctccgcc tcctgggttc 2580

aaacgattct cctccctcag acccccgagt agctgggatt gcaggtgcct gccaccacgc 2640

ccggctaact tttttgtatt tttagtggag acggggtttc accatcttcg tcaggctggt 2700

ctcagactcc tgacctcaag tgatctgccc gcctcagcct cccaaaatgc tgggattaca 2760

ggcatgagct actgtgcccg gccaaagaaa atacttttta tgccagccct gaaactaccc 2820

tgaagcacat acatcaacct tgaggcctca cactccatca agaggggtga agggcatgag 2880

gaattagaaa gcatagggat ttttagttag acagatctgg ttcaaatcct agacttgtgc 2940

cttgaacaaa ttatttaccc tcattgaact ctagattcat tatttgtaaa atgaaagaca 3000

ataatagtta tctccaaagg aaagttgaat atgatcattc atttattcat taattcaaca 3060

tttattattg cctactttgt gccaggttct attctaggaa ctaagggata caactttgaa 3120

taggcaaaat ctctgctctc ctgaagttta cttttttttt tttttttgag acagagtttc 3180

actcttgtca cccaggctag agcgcaatgg tgctcttggc tcactgcaac ctccacctcc 3240

tgggttcaag tgattctctt gtctcagcct cccaagtagc tgggactaca ggtatgtgcc 3300

accacgcccg gctatttctg catttttagt agagatgggg tttcaccatg ttggccagac 3360

tggtctcaaa ctcctgatct caggtgatat gcctgtcttg gccttccaaa gtactgggat 3420

tacaggcctg agccactgca cctgacctga agtttatgtt ctattaaata gcaacagaca 3480

gtaacataaa ccaaaaataa ataggaaaac accataacaa aaatcaaaca gtgatataat 3540

tgagagttgc ttctatttct ttttgttgtc ttcttggttc aatcagcctg ctaaactata 3600

tggaacctca ttttcatggg ccacttattt aagccggggg accttggaaa gtctctcatg 3660

tctctcatct caacggccta atgtgacttc tcttgaaata tttggacatt agcaggaagc 3720

tgaggcttta catcagatct ttactttaat ggtggacttg actttactgg tagattttta 3780

ggctctgtgt ggactgtgga gatgatatct ggggggcagg cagacacttg ccctgcctct 3840

gtctgagaaa attctgtttt ggatgtcttg ttgaagttgg tgctggcatc ctaagccctt 3900

gctggggtcg taatttaatt catcagaatg tgtggcttgc aagaaccggc tcagatcctg 3960

cccttcaaaa acaaaacatg agcgtgccaa gaaagtccaa ggtgttgaat gttgccactt 4020

caagcctaaa ctttctagga acacctaagt gggtggcagc ttccagttct ccaggctgct 4080
tctaggccag agctgggttc cacaagagac agaataggca tatatatgct taaggaactg 4140
gaaaaacagg ctctctctct ctcacaaaca cacacacaca cataccaagg tagctgtcaa 4200
aatgttatcc gaaattttgg aaccaaaaaa tcttgaaaga tggtattcca atatcacatt 4260
ttatgtaagt tttctattat attagattca aattacgatt cgaggccaca agctttaaga 4320
attcagggcc tttttaactt gccaagcccc acaccactcc aggaacttcc ccacacccca 4380
gttctcagaa ttcatgtgca aggtctttcc taaatccagg gtccaggtca gagagtggag 4440
gatgtgctct atttcttacc tgattgcaga cccctctgac agtgctccct tctgaagcac 4500
tcactgtctg aacgtacaca gtctcagact taatcatgca cagtgagcaa gactgtggtg 4560
tgataattgg cgtccctgac ttattagggc aaatctatgg gagggggaga cctcctggac 4620
cactgagcaa ttaattcatt tacattagga agtttctccg tcagatgcag gaaaaaaatc 4680
ttgttttcct gctgtggttt tgacttttgc cccatcttct gttgctgttg taggaggcaa 4740
aataagggtc aaggcctgga aacacaagtg ctttgactga agctccactt ggcttccgaa 4800
gcccaagctg ggttgtacca ggttccctag ggtgcaggct gtgggcaact gccagggaca 4860
tgtgcctgcc caccggcctc tggccctcac tgagttggcc aatgggaaat gacaattgtg 4920
aggtggggac tgcctgcccc cgtgagtacc aggctgttga ggctgggcca tctcctcctc 4980
acttccaytc tgactgcagt ctgtggttct gattccatac cagagggtaa gagcaattct 5040
gtgaagttcc aggctgggtg ggggatgcat gcatagcctc tggctgggat cacccaggct 5100
ctcccgtccg tagtagtgtg ggagtggata caggtggata ctctggtcag agcagcactg 5160
gtggaggcag atatgcactg ggcttcttcc tccgttctcc cacagcccca agagagaaag 5220
ggttatttca gacattcctt ctaagatgca tggaaccatt ctgaattttg cccagttcgc 5280
tctgtagcag gatacctatt gagaaaaagt tagggtcagt aaggtggaag ggtctgtcca 5340
cagatgaagt ccaattcgat taagggggat aagggaatac attgcctctt agcttgacca 5400
ggtagggcaa aggaagaagc atatatgaag gcagcttcag aaaagtcaag ctgagcactg 5460
acttcagact ggaattagga atccagctct gccactttat tctactcagc aaatatttac 5520
tgagcaaatt ctatgggcta gacagtggat tgggttcaca agatacaatg agtgtgacat 5580
ggttgttgtc tatggatttg gggatatatg taggtatagg gatatcttac aaggtaatca 5640
agaggttcta atgaggccag ccatggtggc tcacacctgt aatcccagca atttgggaga 5700
ccgaggcggg tggatcacct gaggtcagga gttccagact agcctgacca acatggtgaa 5760
accccgcctc taccaaaaat acaaaaatta gttgggcgtg atggcaggtg cctgtaatcc 5820

cagcttctcg ggaggctgag gcaggagaat tgtctgaacc tgggaggcag aggttgcagt 5880

gagccgagat tgttgccact gcattccagc ctgggtgaca gagcgagact ttgtgtcaaa 5940

aaaaaaaaaa aaagaaagaa aagaaaaaga ggctctaatg agataaaatg agaaaagcct 6000

ggcatatagt ggcaacttat gaaaaattgt aattaaaaaa aaacattttc tgacagaaga 6060

aactggatct acctggtttt tctgaagcct aatcctgctc gccccagtga gtgctgtttc 6120

tgaggcatcc tggttgtttt gagctgtgga tgctgaaggt tagagtggga gggattttag 6180

aggttaggtc tgcccctctt gtgttagagg acatggatcc ctggtctgga gaggttctgg 6240

tttttggatc aagcctcaca aggggtggca ccaactcact cctaggaact ccgctagaag 6300

gaaggccagc tctgcctaat tcggttgggg agatgggggt ccctttatgc tagcagaata 6360

tgtccgaagg agcatgatgg tgtcagcttt gttcatgaag gccagtggta cacagggagc 6420

ccggcagctt cctcagcagt ccctgctgcc actcttcctt aagtcttgag gagtcttttt 6480

ttggcacaat ctcagctcac tgcaacctcc gcctcccagg ttcaagcgat tctcctgcct 6540

cagtctccca agtagctgag actacaggca tgcgccacca cgcccagcta atttttatat 6600

ttttagtaga gatggggttc accatattgg ccaggatggt ctcgatctct tgacctcata 6660

ttccacctgc ctcggcctcc caaagtgctg gtattacagg tgtgagccac tgcgcctggc 6720

cgaggagtct taagctgaga tcacagcatt gcactccagc ctgggcaaaa aaagcaaaac 6780

tccatctcaa aaaaaaaaaa aaaaaaatag acacaagact ggctccttgt cttttttggg 6840

gacagggtct cactctatca cccaggctgg agtgcagtgg tgcaatcaca gctcactgca 6900

gcctcgattt cccaggctca agtgaccctc ccatcttagc ctcctgagta gctgggacta 6960

caggtgtgtg caaccatgcc tggctaattt ttaaaaattt tttgtagaga tgaggtctca 7020

ctatattggc tggggggcct caaactcctg ggctcagcag tcctcccacc tcagcctccc 7080

aaaaggctgg gattatatgc ttgctctttt taaggtggct gtagggacaa actttccacc 7140

tactccttgt caagccagtg gaccggtggt cccagacata cggctaaagt caagaggtga 7200

tgtcttttgg agagatactt tcaatcagga atttcaatca gaaattcaat catgtggaga 7260

gagacttatc ctaaaaatgt ggtggtgcgt gggatgctct gttttattag ttccttgaca 7320

gtatgtatgt gtgtgagtgt gtgtgtgcgc gcgcgcacac tcatttggat gggtgtgtat 7380

gtgtgggggg gggtggtgcg tacgtatgtg gatgtgtgga tgtggtgtgt gggtgtgcgc 7440

gtgcataggt ggaggtgtgt gtatgggtgc gggtatgtgt gtgtgttggg catggagata 7500

ttgacagctc tcccagggct gagtgaaggc tttcgggcaa agctcctggg agctaggcaa 7560

agctgagttg attcctggtt atgccattta ttattgggtt gcaccgtgtg aaactgccaa 7620

tattctacac tttgactttt atttattttt atttttattt tttttgagac agagtttcac 7680

acctgtcacc taggctggag tgcagtggcg cgatctcagc tcactgcaac ctctgcctca 7740

tggattcaag tgattctcct gcctcagcct cccaagtagc tggaattaca ggtgcccgct 7800

accacgcctg actaattttt gtatttttgg tagagacggg atttcaccat gttgtccagg 7860

ctggtctgaa actcctgaca tcaggtaatc cacccacctc agcctcccaa agtgctggga 7920

ttacaggcat gagccactgc gcccggccca ttttgacttt taaaaatggg agtttgatat 7980

aattcaatcc agtggttgaa ttagctagca tcgttccctc tccaagtctc aggttctcct 8040

acacgttaga gtcaaaagca gggctatggg aagattaagt aaaataaatt ttgaaaatgc 8100

cttatgaaaa ttacactcca aagaactcgc gccagtgtca gtgttctcat gttcctcatc 8160

tcacatgatc acatttcgcg gattaggaag ctgagtctga gaagctccgt gtagtgcttt 8220

ttcggaggca ccgtgatgtg atggaaggct cactcgttag gaagtcagaa cagagtctct 8280

gagggatcat ctccttaatc tgtcagtttc ctcatctctg aagttgggct catttccttc 8340

cttcatggag ttattgtaaa gatgaagata aataacgtgt aaaatctagc atgggaactg 8400

gcttctataa ggttctaata agtgcattcc tactccttcc cctcagcctt cccatttgta 8460

aaagcaaggc aggggtgagg tgatttctgg ggctcctttt ggctctgaca tttgaggatt 8520

ttgtatactt tttttttttt gagacagagt cttgctctgt cacccaggtt ggagtgcagc 8580

tcaatgcaaa ttccgcctcc caggctcaag caattcttat gtctcagcct cctgagtacc 8640

tgggattaca ggcaggcacc accaccccca gctaattttt ttgtattttc agtagagacg 8700

gggttttgcc atattggcca ggctggtctt gaactcctga cttcatgtga cccacccatc 8760

tcagcctccc aaagtgctga gatgacaggt gtgagctacc gtgcctggcc aattttgtgt 8820

gctttaatgc ccttttctgc tggaagagtt ggcaccaggt ttggtgatct ctttccccca 8880

cacggctctg cctcctgcca gtcccagagg ggaccctgtc cttgcatttc acaggattct 8940

gctgttgcaa ctgaaattcc agtaggtcaa agtgaaattt ctcatacact ttaacatgaa 9000

gataaatgat cacagtatgg ccctttagga tcctgagaac atcacggtca tcccctggta 9060

taattttaaa agcagatgaa tccatgcctg tgcgaggttt gccaggaaag ccagtgctgg 9120

gattacagtg gaagtctttt tatgctactt ttttcttgta tccctcaccc catggggtgg 9180

catattgaaa ggcaggatgt gtgaccacga tacttttctc ctcctggact atgtctaaga 9240

gtctgttatt gggttctgaa gatcagagtt taatttccga ctcctctctg tgtagctctg 9300

ggatcttgga aagccactta acctttctga agtccccttt cctcatctct aaaatgcata 9360

cactcatcac taacatttac tgagcactga catgtgccag acaccattct aagcatttta 9420

cacagactac accatttgat cttccaacaa acagaacact gaaacgcatt acaggtcaga 9480

acaaatgatt tgtgcctaag caccaagacc gtagagcccg tgctccctat tctaccctat 9540

cctgtctctc aaaatgattg tgagaatcga atgagacact aggtgagaaa agggttttat 9600

aaatagcatt ttaaaaattt tttaaagtcc acaaaatttt taattttaat acagataaaa 9660

tagatccctt tgttttataa aaagtaacaa aatttgttat acaacaacta tgttatttat 9720

taattttgcc tttttgtatg ctgccaggaa agaaacatta agaaatctta aattgattat 9780

ggtgaatcag aaggtctgcc tggacttttt attgctctaa ctgtacagct gatcatacta 9840

cctcattttt ttttatgaca cttcaagggt gcgcttagct tcatcactcc ttcgttgcca 9900

aaagctttgt gaccaaaaac aattaagcag attcctgagt cactaaatga cacataacca 9960

gagttgagac ttaggaactt ttagtgccat gctaagccca cagggacaca acaaatagca 10020

ttttacaaag gcaaagaatt gtgacacttg agatttagct tgttgatcct tgtaaaagtt 10080

ttctttttag gcataattga gttttagatc atagtactca ctattactta gtaataattt 10140

ttttctgata gaaatacagt gtaacaggcc gggcgcagtg gctcatgcct gtaatcccag 10200

cactttggga ggccgaggcg ggcggatcac ttgaggtcag gagtttgaga ccagcccggc 10260

caacatggtg aaatcccatc tctactaaaa atacaaaaaa ttagccaggt gtggtcgtgg 10320

attcctgtga tcccagctac ttgggagggt gaggcaggag catcagttga acccaggagg 10380

cggaggttgc agtgagccaa gatggtgcca ttgcactcca gcctgggcca caaagcgaga 10440

ctccacttca gaaacaaaaa aaaaagagag agagaaaaga aggaaggaag gaaggaagga 10500

aggaaagaag gaaggaagga aggaaagaag gaaggaagga aggaaggaaa gaaggaagga 10560

aggaaagaag gaaggaagga aagaaggaag gaaggaagga agggtaacaa gcaaagtgta 10620

acaatggcaa tatctaaaaa aataggtatt tttatatgtt tgtcgtttta tatatatgac 10680

ccccacttta gagatgagga aactgagaga ttaaggaaac gatccctgag agactctgtt 10740

ctgacttcca aatcggtgag ctttccatcg catcacggtg cctccgaaag catgacacgg 10800

agcttctcag actcagcttc ctaatccgct aaacgggatt atgtgagatg aggaacatga 10860

gaacgctgac atgggtgagg gttccttgga gtatcatttt catgtggcat tttcaaaact 10920

tattttacct aatcttccca aagccctgct tttgactcta atgtgtctcc tgagacttgg 10980

agagcgcaag atgctagcga cagagcaaga ctccatctcc agataaataa ataagtaaaa 11040

taaaaaagaa cacaaataat tttgaaaatt tttttgaaaa ttaggcacgt ttgcactgac 11100

cttcaattgt tattaattgc tggtttccca cccagaatta agttggaatg caactttctt 11160

ttacaatcag agtccgttct tggtcttgga aacttctgag gctcctgtgc taatcacact 11220

cttgtatttt tggcacctct accccgtgcc actgtcatgg aacccaggct gatcgcacct 11280

attagtggag aaatctgtcc ataatactga agtttgggga caaacagtgt tcccttaggg 11340

taggagaaag agatctttat ttttgacaaa gggggaggag ccagaaaact ccagagaccc 11400
ctgagtttgc cctctctcca aggtttgggg taagcccccc gtcacccttt atctctgggg 11460
ctttcacata ttctggattc tctcctcctg tttcccagca gaaaaggatg gagcctcaca 11520
gattcttccc atttctggag aaaaacatgc atggagctca aagttcttct caggagtttt 11580
attgccaaag ccataataag aaagggtgga ggtgacaagc agtgaggaag tttaaagatg 11640
catgaaatct gtaaagtctc agaacaagaa ttctcctaaa atgcaaaagg ggctttgctg 11700
gtctcccctt ggcttctcat gtagctcacc tctttttctt tatcttgaga ctagtcaaac 11760
ctaagctgtt tctcatttta tttccagaag ctattgagaa cactctcctg aattcttcaa 11820
attcagtaga gggcgacaaa tgtacatata aatgatggta gtgggtctta aataaagact 11880
catgacacct aaaggggcag cacctgagtc tgattgcacc tgtttctgtt gctgtttctg 11940
tctctcttct ctctgtctgc catttcatta tcaatggtta ctttacttat aagatcatat 12000
tagaacctga tatttgataa atgatgcatc agatctatag tgagagaaaa aattaatgca 12060
attaaaggtg ttgtaacagc tagtcttcaa gtggggagaa atcatttgag taccttaggt 12120
cacagcttac atcaaaacaa aaaatcagag ctacattaaa aagtgaaatt ttaactatat 12180
caaacaatag aaaaaaacag aagaaaattg aatacttact aaatcttagc atgaataaga 12240
actgtttaac acttagaggc aaggactggg cgtggtggct catgctttta atcccaggac 12300
tttgggagcc caaggcgggc ggatcacctg aggtcaggag tttgagacta gcctggccaa 12360
catggtgaaa ccccgtctct actaaaaaat gcaaaaatta gctgcgtgtg gtggtgcatg 12420
cctgtaatct cagctacttg ggaggctaag gcatgagaat cgcttgaacc tgggaggtgg 12480
aggctgtagt gagccgagat tgtgccactg cactacagcc tgggtgacag tgtgaaatcc 12540
tctctctcaa aaaaaaaaaa aaaaaaaagc aaactagagc agtgaggtac cattatttcc 12600
tttgctcact aaactgacaa cacacaaatg ttttttataa tacccaaagc tgatgagggt 12660
agttaaggta tgccctttta tacacacact aatgatgtac tactggttgg cagtataaca 12720
tatgctgcca tgtggggata tgtatcagga gacttaaaaa tgtgcatacc ttttggtcca 12780
gtaatttact tctgggaatc tgtcataaca gaataataat cttggggaaa gctacatgcc 12840
taaggatatt taaaatatta tttaaaaatc aaagtataat ttcttacaga atataaaata 12900
atattttaaa atgaaaatat gctaaaagtt tgatgaaata taaatggtca aatatatatt 12960
gattatatcc acttactaga ctagcactca ctctgagacg ttaaaaatag tcattataaa 13020
aactagaaaa tgccaaagac aaaataaagg aataaagttt tacataaagt atgattccac 13080
tatgtttaaa aataaacaga gacattcttg gagttgagta ttgttttctt ttctgtcatg 13140

tccaaagaac tatataacta ttatttttaa tgaactatat atgtaatata tacatatagt 13200
ttatatgtat atacaaaatt tatctcatat atatgataaa gatgaaagat gagttggatg 13260
tgccacgtga agagggtagt atagaacccc aggtaatggg gcataggagt gggattccag 13320
ataccaggcc catgtttttg gggtgagatt gccaatcacg gtctttcttc catccctcac 13380
agaggagtag gtttgtcttc aacaaacctt cagttgtcct gaagacaaac ctaattctgg 13440
agacttcata taatctagaa gagacaagca aactgatgaa aaatagtgaa tttttaaggt 13500
aaaataaagt acatggacta cactttgttt agaatcagat tcttgggatt aaccacatta 13560
acccacagag ggtcttagtg atgcctctaa tccaggatcc taggacctat ttctctctgt 13620
gagatgcttt ctcccaactc cttggtgaga gtgggaagac taagacctca gcaatctgag 13680
gtggaggcct aagatccccc taagatcgga ggcagaatct gagaggggat aaaagtccct 13740
atacctgtat tgggcccttt tctgggaggg ggatatcaaa gaatgatttt gagacaggga 13800
ggcttttgac tacctgtgcc acttgagctc tttgctaggg ctccagaata catatttcaa 13860
atacattccc cctccctcct tccttccctc ttccactctt cctttttatc ttcctttctt 13920
cttttccttc ctccttccct tcctttctct ggctctctca tgatttcttt tcctcattat 13980
aaaagtgctt atttagtccc tactctgcta ttagtgtgtt agtctttgtc ccctggtact 14040
tgctgtttaa tggagaaatg ggtgagcaaa acagaaatta cagcagagtg caatgataga 14100
gctaagccag gtgtataaat ccattctcac actgctgtaa aaaactactt gagactgggt 14160
aatttataaa gaaaagaggt ttaattgact cacagttcca caggctgtac aggaagcatg 14220
gctggggagg cctcagaaaa cttacaatca tggtggaaga aagagcgaag gggaagcaag 14280
cacatcacac agcagcagga gagagagaga gaaagagaga gagagagaat ataggggaag 14340
tgctacacac tttcaaccag atcttgtgag aattcaccta ctatcatgag aacagcaagg 14400
gataagtctg cctccatgat tcagtcacct cctaccaggc cccttctcca acacatgtcg 14460
acgtgctatt tgggtgggga cacagaccca aaccatatta ccagggcact ggagaaacac 14520
agaggggaaa gaaccagcca aggagtgaga tggagaacaa ggaggacttc ttgaaacaga 14580
tgacatccaa actgggtcct gaaagctgaa tagagattag acaggggagg aggggcagct 14640
aaagatggct caggcaaaca aagggccagg ggatatgttc atgggatgat gtctctctcg 14700
ttgtctgctt aacacaaggt gagtctctcc ctccctctct ctctcttttt ctctgtgtgt 14760
gtttgtgtgt gtgcatgtgt gcaaatgtaa tatacccaat agtcaaacat gtgccccagg 14820
agaggggtag aggaagaaag agaatgagag agtaagaagg aggaatagac acagaaaatg 14880
agagagaagg ggggaaagaa aaagaagaaa ggagccagag gagagaagct ggttagcatt 14940
gaatggagca atctgtgtca tcgtacttgg gaaacccaag gatggattct tggcaagtcg 15000

actcttggag ctttccctgt gcttggtcct gtgctcagac atgggaaaat tagaggagtg 15060
tcatctgtgc aatcactgaa ttcataatct tggtgaggaa aggagactac acacagggaa 15120
taatgctaag tattacagat ttcagggcag aaagagatca aggtgggctg caatattcag 15180
aaaagtcttc ctggaaaagt tgaatactta gaaagcagct cctagaagta gactctgctg 15240
agatggacgg agtcctttgt aggtcccaac tgggtgtgtg tgtggggtct gtctctccat 15300
ggctgacagt gcacatgtgg attccagggc tcaggatgct gttgctggga gctgttctac 15360
wgctattagc tctgcccggt catgaccagg aaaccacgac tcaagggccc ggagtcctgc 15420
ttcccctgcc caaggggccc tgcacaggtt ggatggcggg catcccaggg catccgggcc 15480
ataatggggc cccaggccgt gatggcagag atggcacccc tggtgagaag ggtgagaaag 15540
gagatccagg taagaatgtt tctggcctct ttcatcacag acctcctaca ctgatataaa 15600
ctatatgaag gcattcatta ttaactaagg cctagacaca gggagaaagc aaagcttttt 15660
tatgttaacc ataagcaacc tgaagtgatt tggggttggt cttccaagga tgagtgtaga 15720
tggtgcctct ataaccaaga ctttggcttt gctgcatctg cagctccttt tccatcccct 15780
ttcccatctt caccctcatc cctattccca gtacattcat attctgattc ctctttctgt 15840
ctgcttaact tccatttcac ccagtggcat tcaaccacat ttactgcaca ccccctgaaa 15900
ggctcagtcc tgcctttggg gaactcttga tctaggtaag atgtctaatg tgcaaggctc 15960
tgttggtggt tactacaaga aagtctactc taaaaatgtc aaactgaatg tgaacaagta 16020
ttcaaagtat ggagcataga gaaaatatac tcaccgtgga cctgatgaag aatgaaggct 16080
tcaaggagga ggcagagctt cagctaggcc ttgaatgatg ggtaggcaga atagaggagg 16140
agagacatcc tagatggagg gggtagaatt gcaaaaccag ggttgatggt gccagcacat 16200
aaagggctgg cagggtggag ggtctatgat agagacctat aggagataaa gatagagttg 16260
aaattatggg agcctccatg tctgtgggag atatagaagg aggaggtaac acctctctcc 16320
ttttgggagc tcttattggt ttcttgatct ataagtcaag aaggttgtga gtgggagcca 16380
cagggatggt aatttaggct gtaaccaacc taggcaggag ttctgttctt tgtagtcact 16440
gaggtcttct cattccttag gtcttattgg tcctaaggga gacatcggtg aaaccggagt 16500
acccggggct gaargtcccc gaggctttcc gggaatccaa ggcaggaaag gagaacctgg 16560
agaaggtgcc tatgtatacc gctcagcatt cagtgtggga ttggagactt acgttactat 16620
ccccaacatg cccattcgct ttaccaagat cttctacaat cagcaaaacc actatgatgg 16680
ctccactggt aaattccact gcaacattcc tgggctgtac tactttgcct accacatcac 16740
agtctatatg aaggatgtga aggtcagcct cttcaagaag gacaaggcta tgctcttcac 16800

ctatgatcag taccaggaaa ataatgtgga ccaggcctcc ggctctgtgc tcctgcatct 16860

ggaggtgggc gaccaagtct ggctccaggt gtatggggaa ggagagcgta atggactcta 16920

tgctgataat gacaatgact ccaccttcac aggctttctt ctctaccatg acaccaactg 16980

atcaccacta actcagagcc tcctccaggc caaacagccc caaagtcaat taaaggcttt 17040

cagtacggtt aggaagttga ttattattta gttggaggcc tttagatatt attcattcat 17100

ttactcattc atttattcat tcattcatcg agtaacttta aaaaaatcat atgctatgtt 17160

cccagtcctg gggagcttca caaacatgac cagataactg actagaaaga agtagttgac 17220

agtgctattt tgtgcccact gtctctcctg atgctcatat caatcctata aggcacaggg 17280

aacaagcatt ctcctgtttt tacagattgt atcctgaggc tgagagagtt aagtgaatgt 17340

ctaaggtcac acagtattaa gtgacagtgc tagaaatcaa acccagagct gtggactttg 17400

ttcactagac tgtgcccttt tatagaggta catgttctct ttggagtgtt ggtaggtgtc 17460

tgtttcccac ctcacctgag agccattgaa tttgccttcc tcatgaatta aaacctcccc 17520

caagcagagc ttcctcagag aaagtggttc tatgatgacg tcctgtcttg gaaggactac 17580

tactcaatgg cccctgcact actctacttc ctcttaccta tgtcccttct catgcctttc 17640

cctccaacgg ggaaagccaa ctccatctct aagtgccgaa ctcatccctg ttcctcaagg 17700

ccacctggcc aggagcttct ctgatgtgat atccactttt tttttttttg agatggagtc 17760

tcactctgtc acccaggctg gagtacagtg acacgacctc ggctcactgc agcctccttc 17820

tcctgggtcc aagcaattat tgtgcctcag cctcccgagt agctgagact tcaggtgcat 17880

tccaccacac atggctaatt tttgtatttt tagtagaaat ggggtttcgt catgttggcc 17940

aggctggtct cgaactcctg gcctaggtga tccacccgcc tcgacctccc aaagtgctgg 18000

gattacaggc atgagccacc atgcccagtc gatatctcac tttttatttt gccatggatg 18060

agagtcctgg gtgtgaggaa cacctcccac caggctagag gcaactgccc aggaaggact 18120

gtgcttccgt cacctctaaa tcccttgcag atccttgata aatgcctcat gaagaccaat 18180

ctcttgaatc ccatatctac ccagaattaa ctccattcca gtctctgcat gtaatcagtt 18240

ttatccacag aaacattttc attttaggaa atccctggtt ttaagtatca atccttgttc 18300

agctggacaa tatgaatctt ttccactgaa gttagggatg actgtgattt tcagaacacg 18360

tccagaattt ttcatcaaga aggtagcttg agcctgaaat gcaaaaccca tggaggaatt 18420

ctgaagccat tgtctccttg agtaccaaca gggtcaggga agactgggcc tcctgaattt 18480

attattgttc tttaagaatt acaggttgag gtagttgatg gtggtaaaca ttctctcagg 18540

agacaataac tccagtgatg ttcttcaaag attttagcaa aaacagagta aatagcattc 18600

tctatcaata tataaattta aaaaactatc tttttgctta cagttttaaa ttctgaacaa 18660

ttctctctta tatgtgtatt gctaatcatt aaggtattat tttttccaca tataaagctt 18720
tgtctttttg ttgttgttgt tgtttttaag atggagtttc cctctgttgc caggctagag 18780
tgcagtggca tgatctcggc ttactgcaac ctttgcctcc caggttcaag cgattcttct 18840
gcctcagcct cccgagtagc tgggaccaca ggtgcctacc accatgccag gctaattttt 18900
gtatttttag taaagacagg gtttcaccat attggccagg ctggtctcga actcctgacc 18960
ttgtgatctg cccgcctcca tttttgttgt tattttttga gaaagataga tatgaggttt 19020
agagagggat gaagaggtga gagtaagcct tgtgttagtc agaactctgt gttgtgaatg 19080
tcattcacaa cagaaaaccc aaaatattat gcaaactact gtaagcaaga aaaataaagg 19140
aaaaatggaa acatttattc ctttgcataa tagaaattac cagagttgtt ctgtctttag 19200
ataaggtttg aaccaaagct caaaacaatc aagacccttt tctgtatgtc cttctgttct 19260
gccttccgca gtgtaggctt taccctcagg tgctacacag tatagttcta gggtttccct 19320
cccgatatca aaaagactgt ggcctgccca gctctcgtat ccccaagcca caccatctgg 19380
ctaaatggac atcatgtttt ctggtgatgc ccaaagagga gagaggaagc tctctttccc 19440
agatgcccca gcaagtgtaa ccttgcatct cattgctctg gctgagttgt gtgcctgttt 19500
ctgaccaatc actgagtcag gaggatgaaa tattcatatt gacttaattg cagcttaagt 19560
taggggtatg tagaggtatt ttccctaaag caaaattggg acactgttat cagaaatagg 19620
agagtggatg atagatgcaa aataatacct gtccacaaca aactcttaat gctgtgtttg 19680
agctttcatg agtttcccag agagacatag ctggaaaatt cctattgatt ttctctaaaa 19740
tttcaacaag tagctaaagt ctggctatgc tcacagtctc acatctggtt ggggtgggct 19800
ccttacagaa cacgctttca cagttaccct aaactctctg gggcagggtt attcctttgt 19860
ggaaccagag gcacagagag agtcaactga ggccaaaaga ggcctgagag aaactgaggt 19920
caagatttca ggattaatgg tcctgtgatg ctttgaagta caattgtgga tttgtccaat 19980
tctctttagt tctgtcagct tttgcttcat atattttagc gctctattat tagatatata 20040
catgtttagt attatgtctt attggtgcat ttactctctt atcattatgt aatgtccttc 20100
tttatctgtg ataattttct gtgttctgaa gtctactttg tctaaaaata acatacgcac 20160
tcaacttcct tttctttctt ccttcctttc tttcttcctt cctttctttc tctctctctc 20220
tctttccttc cttccttcct ccttttcttt ctctctctct ctctctctct ttttttgaca 20280
gactctcgtt ctgtggccct ggctggagtt cagtggtgtg atcttggctc actgctacct 20340
ctaccatgag caattctcct gcctcagcct cccaagtagc tggaactaca ggctcatgcc 20400
actgcgccca gctaattttt gtatttttcg tagagacggg gtttcaccac attcgtcagg 20460

ttggtttcaa actcctgact ttgtgatcca cccgcctcgg cctcccaaag tgctgggatt 20520

acaggcatga gccatcacac ctggtcaact ttcttttgat tagtgttttt gtggtatatc 20580

tttttccatc atgttacttt aaatatatct atattattgt atttaaaatg tgtttcttac 20640

agactgcatg tagttgggta taatttttat ccagtctaaa aatatctgtc ttttaattgg 20700

tgtttagaca atttatattt aataaaattg ttgaatttaa gatggatgac tgttttattt 20760

gtttgctgtt caccacttct gttttattct ctttccagaa ttcttttgga ttgtttaaat 20820

atttcataat attttatctt 20840

<210> 2
<211> 244
<212> PRT
<213> Homo sapiens

<220>
<221> VARIANT
<222> (9)..(9)
<223> Xaa = Leu or Gln

<220>
<221> VARIANT
<222> (90)..(90)
<223> Xaa = Gly or Ser

<400> 2

Met Leu Leu Leu Gly Ala Val Leu Xaa Leu Leu Ala Leu Pro Gly His
1               5                   10                  15

Asp Gln Glu Thr Thr Thr Gln Gly Pro Gly Val Leu Leu Pro Leu Pro
            20                  25                  30

Lys Gly Ala Cys Thr Gly Trp Met Ala Gly Ile Pro Gly His Pro Gly
        35                  40                  45

His Asn Gly Ala Pro Gly Arg Asp Gly Arg Asp Gly Thr Pro Gly Glu
    50                  55                  60

Lys Gly Glu Lys Gly Asp Pro Gly Leu Ile Gly Pro Lys Gly Asp Ile
65                  70                  75                  80

Gly Glu Thr Gly Val Pro Gly Ala Glu Xaa Pro Arg Gly Phe Pro Gly
                85                  90                  95

Ile Gln Gly Arg Lys Gly Glu Pro Gly Glu Gly Ala Tyr Val Tyr Arg
            100                 105                 110

Ser Ala Phe Ser Val Gly Leu Glu Thr Tyr Val Thr Ile Pro Asn Met
        115                 120                 125

Pro Ile Arg Phe Thr Lys Ile Phe Tyr Asn Gln Gln Asn His Tyr Asp
    130                 135                 140

Gly Ser Thr Gly Lys Phe His Cys Asn Ile Pro Gly Leu Tyr Tyr Phe
145                 150                 155                 160

Ala Tyr His Ile Thr Val Tyr Met Lys Asp Val Lys Val Ser Leu Phe
                165                 170                 175

Lys Lys Asp Lys Ala Met Leu Phe Thr Tyr Asp Gln Tyr Gln Glu Asn
            180                 185                 190

Asn Val Asp Gln Ala Ser Gly Ser Val Leu Leu His Leu Glu Val Gly
        195                 200                 205

Asp Gln Val Trp Leu Gln Val Tyr Gly Glu Gly Glu Arg Asn Gly Leu
    210                 215                 220

Tyr Ala Asp Asn Asp Asn Asp Ser Thr Phe Thr Gly Phe Leu Leu Tyr
225                 230                 235                 240

His Asp Thr Asn

<210>  3
<211>  386
<212>  PRT
<213>  Homo sapiens

<400>  3

Met Asn Glu Pro Thr Glu Asn Arg Leu Gly Cys Ser Arg Thr Pro Glu
1               5                   10                  15

Pro Asp Ile Arg Leu Arg Lys Gly His Gln Leu Asp Gly Thr Arg Arg
            20                  25                  30

Gly Asp Asn Asp Ser His Gln Gly Asp Leu Glu Pro Ile Leu Glu Ala
        35                  40                  45

Ser Val Leu Ser Ser His His Lys Lys Ser Ser Glu Glu His Glu Tyr
    50                  55                  60

Ser Asp Glu Ala Pro Gln Glu Asp Glu Gly Phe Met Gly Met Ser Pro
65                  70                  75                  80

Leu Leu Gln Ala His His Ala Met Glu Lys Met Glu Glu Phe Val Cys
                85                  90                  95

Lys Val Trp Glu Gly Arg Trp Arg Val Ile Pro His Asp Val Leu Pro
            100                 105                 110

Asp Trp Leu Lys Asp Asn Asp Phe Leu Leu His Gly His Arg Pro Pro
        115                 120                 125

Met Pro Ser Phe Arg Ala Cys Phe Lys Ser Ile Phe Arg Ile His Thr
    130                 135                 140

Glu Thr Gly Asn Ile Trp Thr His Leu Leu Gly Cys Val Phe Phe Leu
145                 150                 155                 160

Cys Leu Gly Ile Phe Tyr Met Phe Arg Pro Asn Ile Ser Phe Val Ala
                165                 170                 175

Pro Leu Gln Glu Lys Val Val Phe Gly Leu Phe Phe Leu Gly Ala Ile
            180                 185                 190

Leu Cys Leu Ser Phe Ser Trp Leu Phe His Thr Val Tyr Cys His Ser
        195                 200                 205

Glu Gly Val Ser Arg Leu Phe Ser Lys Leu Asp Tyr Ser Gly Ile Ala
    210                 215                 220

Leu Leu Ile Met Gly Ser Phe Val Pro Trp Leu Tyr Tyr Ser Phe Tyr
225                 230                 235                 240

Cys Asn Pro Gln Pro Cys Phe Ile Tyr Leu Ile Val Ile Cys Val Leu
                245                 250                 255

Gly Ile Ala Ala Ile Ile Val Ser Gln Trp Asp Met Phe Ala Thr Pro
            260                 265                 270

Gln Tyr Arg Gly Val Arg Ala Gly Val Phe Leu Gly Leu Gly Leu Ser
        275                 280                 285

Gly Ile Ile Pro Thr Leu His Tyr Val Ile Ser Glu Gly Phe Leu Lys
    290                 295                 300

Ala Ala Thr Ile Gly Gln Ile Gly Trp Leu Met Leu Met Ala Ser Leu
305                 310                 315                 320

Tyr Ile Thr Gly Ala Ala Leu Tyr Ala Ala Arg Ile Pro Glu Arg Phe
                325                 330                 335

Phe Pro Gly Lys Cys Asp Ile Trp Phe His Ser His Gln Leu Phe His
            340                 345                 350

Ile Phe Val Val Ala Gly Ala Phe Val His Phe His Gly Val Ser Asn
        355                 360                 365

Leu Gln Glu Phe Arg Phe Met Ile Gly Gly Gly Cys Ser Glu Glu Asp
    370                 375                 380

Ala Leu
385

<210>  4
<211>  21
<212>  DNA
<213>  Artificial

<220>
<223>  primer

<400>  4
tttgactttt gccccatctt c                                                21

<210>  5
<211>  20
<212>  DNA
<213>  artificial

<220>
<223>  primer

<400>  5
tgcatatctg cctccaccag                                                  20

<210>  6
<211>  21
<212>  DNA
<213>  artificial

<220>
<223>  primer

<400>  6
gggcagaaag agatcaaggt g                                                21

<210>  7
<211>  20
<212>  DNA
<213>  artificial

<220>
<223> primer

<400> 7
tggaagacca accccaaatc 20

<210> 8
<211> 26
<212> DNA
<213> Artificial

<220>
<223> primer

<400> 8
ctataagtca agaacgttgt gagtgg 26

<210> 9
<211> 23
<212> DNA
<213> Artificial

<220>
<223> primer

<400> 9
atctggtcat gtttgtgaag ctc 23

<210> 10
<211> 97799
<212> DNA
<213> Homo sapiens

<400> 10
gtgcgatgcg cgtcacggcg acgtgcggtc gtgggggcgg ggagacgcgg cggcggcggt 60
cgctgtcgcg gcggcgagag cgcgattcca gaagcggcat cgcggcggcg gcagcggcgg 120
cggctacacc gggcttggcc ccctccctcc tccgttcccc cctcctcccc cctcccctca 180
gcggtggctc ccaagaagtc cgagacacgc ggtgaggcgg cgacgggctc cgggtgaggg 240
tctctggagt gtgggcgccg tttcctgggc gcagagggag agcttggggt cggggggagg 300
tgagagctga ggggcgaatg tccctggcgc gggcgggaga gcgcgctctc ggcccggccc 360
gcaacttgat tcctaactct gacctgccgc ccggcgcgcc ttggcctgag ggtcggccgt 420
gtggggacct ggagccgccc tccttgcgac cgctgtcgga ggagtccggc gcccgctgtt 480
cccctcgcct cgcctttcgc ggcggagggt cggagcctgc tgccctctgc tcaccttcgg 540
gtgcagttgc tccagctccc tgcccgcgga gaggatctgc ccgcccctct tcgtctcttg 600
tctgttttta tcacttctag gaatttgttt ggtggaggct tgggatgggc tctctcagcg 660
ctactttcac agtgactggg gtggggaaga gttccctcac ctttttcagg tggcgcgtca 720

ggcagcctgc ttggtacctg gagtcctgtt ttcccaagcc cccgtccgtc agtgttcgct 780
tcttctaaaa ttaccgatga cagggttata aatcagtttt tagggggctc ggagagagtt 840
tgggttggaa actgttagct cttttttcct cctggctttt gatcttgatt taagcgtttg 900
cctctgaaat tttgacaact tcataagatg gtccaaaagt gaattttggt agaaagattt 960
aaatgcacag ttgggatttt atgggggtac ggtgacttca ttgcaggttg ttgacttttt 1020
ttttaaaata agatgttctt ttaatttttt gactctgggt ttctgagcct tggttattgt 1080
cccccatttc tgttccaaaa gcagactttt ttttttttta tcaccccttc ccctcaccca 1140
gagaatccct ttaaacgcca agaaagctgt cataacattt agcattattt gatcacagaa 1200
cttttccaaa tgaaatgcta aaaattcctt cagaaaaaag catccatcta cccatagcat 1260
tattttttgg ggggaggcta tacgtcttgt cagttctgaa agtttgaagt gacctcttat 1320
caaaggccac ccttatgtct ccttaaacga ctcttaggta attcctagat gttttcatcc 1380
gttctgataa atttgttgtc agagtgttga ttgtgatgat ctgcaactaa aattatttca 1440
tcagtaaaga tttccacaca tgtggcagct gcttgttggt gaattatgaa atttgaaaaa 1500
attgcaaatt aaatgtgaaa ggcatatttc ttttaacttc tgattgtaac tgtctgtcct 1560
ccagtatacg ttcctatcta gactttggtc tgcttagcag atttcctctt ttcacttgac 1620
actaggaatg ggagtaggat ttccagagcc taaaagagag tttgtttttt ttttctatcc 1680
ctaggctcct tgtatgaatt tataaataaa tggttttttc tatcaatagt atcaatttag 1740
ctggtgcact tttttttttt agtataaatc acattttgtt tcctaaaatg aaaataattt 1800
tgttttattc tttatttcag ggattgacag attttaaagc ctgcaggccc agtcctgcca 1860
gttgcctctt tttgtaaata aagatttgat taggcctggt gcgatcgctc acgctggtaa 1920
tcccagcact ttgggaggtc gaggcgggca gataacctga gatcaggagt ttgagaccag 1980
cttggccaac atggtgaaac cccgtcccta ttaaaactac aaaaattagc cgggtgtggt 2040
ggcgcactct tgttatccca gctgcccggg aggctgaggc aggagaatcg tttaaacccg 2100
ggaggtagag gttgcagtga gccaagattg caccattgca ctccagtctg gacaacaaga 2160
gcgaaactcc gtcttaaaaa aaagaggaag aaaaaagttt ttattagaac atagccatat 2220
gcagtcgttt aggtattgtc tgtagtttct tttggggtac agcagcagag ttgagtagtt 2280
gtgccagaga gccatgtggc ctgtaacacc ttatatttat ttactatgtg gaccttaata 2340
taaaaagttt gccgagctcc attaaaaatg atacatagtc aatgttgaaa atttaaatga 2400
gtcagaaaat tgtaaaggag aaagtaaaag ttatttacaa gtacaccact tggggctttt 2460
tttctgtgat tatagaattt gttggtgatt attttttata taaatgaact caaattgata 2520

aatgttggtt tgtaatctgc tttttttttt ttttgttcat tttgtttttt gagacggagt 2580
ttcgttcttg ttgcccaggc tggagtgcaa tggcacgatc ttggctcact gcaacctctg 2640
cctcctgggt tcaagcaatt ctcctgcctc agcctcccta gtagctggga ttacaggtgt 2700
gcgccaccaa gcccggccaa tttttttttt gtatttttag tagagacggg gtttcaccat 2760
gttggccagg ctggtctcga actcctcatg tgatccacct gcctcacccc tcaaagtgct 2820
gggattacag gtgtgagcaa ccgtgcccag ccctgtaatc tgctttttta agaagtattt 2880
cattatttgt atttggattt tgattgcttt tgtattttct gcattggagg tctttctaga 2940
aaggggctta tgaaccgggt tttcatgatt agtcttacca gtcaagattt aaaaagaaaa 3000
tccaggaata ataaatgctt ggtaatgaag atgctggtta acatttattg agcacttact 3060
atgtactaga caatttccac tactttacgt gtatgaaatt atttaattgc tgtggcagct 3120
ctgtgagtgt atactatgat ttctgtttta caggtgagga aactaaggca aggagaaaaa 3180
ttaagtaact gttggaagtt tatcagctag caaatggtgg agctaggatt tgatttcatg 3240
cagtctgatt tgagaaaccc aaattcttaa caatatgcta cagagcctct acttacatta 3300
ttgctcagtg tagaaagttt agacagtcca gagatatact gtcatccctt ggtgtctgta 3360
gggactgctt ctaggacctt tggtgaatat caagatgcac agatgctcaa atctctgata 3420
taaatggcat agtatttgca tataacctgt gcatatcctc ccaaatactt tatatcattt 3480
ctagattact tataatacct aatagaatat aaatgctatg taaatagttg ttatactgta 3540
ttgtttgggg aataaagaca agaaaaaaaa gtctgcatgt ttcaaataca gatgctatat 3600
taaacttttt ggatccaagc ttggttgaat ccacagacat aagagcccat ggatatggag 3660
ggccaactgt ataatgtaaa aaaattcctt ctcatgcgct agtttccagt tttcatcctt 3720
tgtagataac caggggcaac agtttgatat aggtcttttc acctctcttt gtgtgcatat 3780
acaagtaatt atttgcagat ttgtctgcat attcagaatt taaaaattac actttttttt 3840
agcagcttta gatgtacaga aatttgagcc gatagcacag aaagttccca tattcccttc 3900
ccttccctcg tttctcctat tgcaaacatc ttgcattaat ttggcacatt tattgcattt 3960
gatgagccca tatcaatatg ttgttattta ttggtctata gtttacatag ggcttattct 4020
ttgtgttgca gttttttttt tttttttgtt ttgttttttt aagatctcac tctgtatctc 4080
aggctggagt gcactggtgc cgtcatggct ctgcagcctc gacctcccgg gctcaagcga 4140
tcctcccacc ttagcctccc aaagtgttag ggttacagac atgagccact gcacttggcc 4200
gagagttatt ttttacaaaa atgggatcaa gcccagtggt ggcttgcgcc tgtagttcca 4260
gctacttggg aggttgaggt gggaggattg cttgaggcta ggagtttgag accagtctgg 4320
gcaacacagc atgactctga ccagtctggg caacacagca tgactctgtc tcttaaaaaa 4380

aaaaaaaagt gggctgaggg tggtggctaa cgtcggtaat ccttgcactt tgggaggcca 4440

aggcaggtgg atcacctgag gtcaggagtt cgggaccagc ctggccaaca tgaatgaaac 4500

cccctctcta ccaaaaatat attaaaaaaa aaattatctg ggtatggtgg cgcgcgcctg 4560

taatcccaac tactcaggag gctgaggcag gagaatcgct tgaaactggg aggtggaggt 4620

tgcagtgaac cgagattgca ccactgcgct ctagcctggg caacaggagc aaaactccat 4680

ctcaaaaaaa aaaaaaaaaa atgacgaaac ccgtcattca gcagtcattc accttttttg 4740

gtggttttca ggatatagtg cttttggact ttctaatgta aacattcttg atttactaat 4800

tttcagatta atgacaattt attttgggaa tggaaggctg gcttggatgt tactttttct 4860

ctctttttat ttttctattt ttttttttaa gtgtatctca tagtggcagc agcttttccc 4920

caaaggaatg cttatttttt ttgacgggac attctggcta tacctaatca tcaaagttct 4980

tcttttttct ctttatctct ggatttggaa aatgattcat tttggttttc ttcctcataa 5040

attctttttg gtctgtccag ccattgcagt tgctagtcat gtctccttgt cagactttgg 5100

ctttttatga ctgtcatcaa tgttagaaaa aattacttgc tccttatcac tatttcaata 5160

catttcctct tgggctttcc ctgggggcag cactgaattt tttccttagt tgagccatgc 5220

agattttcta gaagtcatcg tttagtttgg cttttcactc ctccatgggt gctgttcagc 5280

ttctaggaga tttcttgcag tttctacttg tcaacagatt caccagcagc accctcctcc 5340

tcactgagac tggtgcttcc ccacccatct ttatcatttt cagccttctc tttttctttc 5400

tcttttcttt tttccttctt tttttttttt ttggttacag cgtcttactc tgttgcccag 5460

actggagtgc agtggtgcaa tcgtagctta ctgcaacctc cgcttcatgg gctcaagtga 5520

tcctcccact tcagcttccc aagtagccgg gattacaggc atgcaccacc atgcctggct 5580

aatgtttgta ttttttgtag aaacagggtt ttgccatgtt gtccaggctg gtcttgaact 5640

cctggcctcg agcgatcctc ctccctcagc ctcttgaata gctggattta acaggcagga 5700

gccattgtcc ctggccatat tcttgcactg ttgattttac tggggcctct gtcagcttac 5760

cctggaattc tgcatcatct gaggatttgg aagaactgaa tcacagttat agtagacagc 5820

atcatattct tatccttgtg tgttttatac tgggacaggt cttgaggaag ttcttctgtc 5880

atggctaggg gacattgagc tattacctct tatagcactg atttggactg tcatgtcttc 5940

cctaaagttc ttgtccataa cataatggat ggtcacagtc atgagcaata actgagtgat 6000

ctctgggact aacaggtgat gaaatgcatg tttagcaaac agtgatttct gttacttctc 6060

tgtggtgcag cagcagaaac ttttgcccaa aggcatagaa gttagagggc ttatgaattt 6120

ctgccaatct ggagataaga tttatgagca tcatgttaac tcaaacctct gcttagagat 6180

ttctagctgt gtgcttcagg agtgttttgg taaaactttg aggatcatga ataaagctaa 6240
tagcttaaaa gttaaacact gtgagttttt tcttcttttg tttcctgggc acttttcata 6300
accttttcag [?]gttttgtgt ttggagccac tcttcccagt gacatactgc acttgtaggt 6360
ctcttgtcac [?]ttatcctca gattctgagt ctgtcctatt tgtttctctt cttcatctta 6420
tcctaggaag aatgtcaagg tggcaaccaa tatctcaaaa catgcagttg tgatgacatt 6480
gacagttttg gcatcagtcc ggatg[?]ttct tgtagagttc agtcatcaca tccaaagata 6540
tcttggctgc agttacattg ctgtc[?]ctta acacgatgta tatgaaattc tgcagtacta 6600
tatccacttt a[?]tcttgtgt tttacagtta tattcttgct atcagtcaca acatgagtat 6660
gtaaagtctc aaaagcttat tatggcaact tggaagttca aaaaagagct ctagcaaact 6720
tgatggatgg atatgatact tctcagcaag atcaaagctt tgtaaaatgt tattagatga 6780
ttttgattca agacagtaca gtataattgt aggaaagtag atcttttagc tcttgaggaa 6840
agttacttag atgttctggg taacagtgac caatctgtgc cataaatatc accagctctg 6900
tcaattcttt gccaggttta ttggttttaa tttgagaatc tccatattgg atttgtagtg 6960
tatatagtac tgctgtagta accctttaat gtcagccagt ggctccactt tattagattc 7020
tataactgct gcttgttact gggcagctag tttctgcagg acatgttggc tgtagactgg 7080
atccacagtg agtagttctc agagaacccg tactgctgac atttgcaaac ccatttcagc 7140
tgtggtgtgg agctgtggcg ttgtacttct ggatgtgacc agaaacagtg tttgggagct 7200
cagcaggtgg tgctgtcttg gctgattgtg tgtgtgtgtg tgtgtgtgtg tgtgtgtg[?]a 7260
ttttatttaa tttctgagac agggtcttgc tctgtcaccc aggctggagt gccatggcac 7320
aatcacggct cactgccctc tcgaactcaa acagtcctcc caactgagac tcccacgtag 7380
ctgggactat agatgcacgc caggctaatt tttgcatttt taatggagac gaggttttgc 7440
catgttgccc aggccggcct tgaactcctg gactcaagtg atcctctttg ccttagcttc 7500
tcaaagtgct gggattatag gcttgagcca ccatgacagg ccctgtgtgt gtgtttttaa 7560
aattaatatt tgtggcttac tttacatctg catctactct cttcttttta gtagttctct 7620
agtgtagtat tattaaatgg atatgctata acttaatact gctcttactg atggatatt[?] 7680
agattatata aatggattgt aaattgccca tacattttat gtatatttat tgcaagctaa 7740
ttccccttta gtaattttgt aatagttgaa gaaaacacat ttttcatttt gatgtgtgtt 7800
atcacattgc cacccagaaa ggtcacagca atgtaaactc tcaccagaaa tgtgggtgga 7860
gtctagcttc tctgtgtcat tacaaattaa aaaaaaaaat ctttgtttat ctgataggag 7920
gttaagcatt tgcagtatat tctatctaac tgggagatat gctttgtttc aatagcattc 7980
ttttaaagtt gatttacgat tacttcaggt ggagcatgca ttctgttccc gggcctccct 8040

taggtggagc tgcttcacct atttacattc tctgcttggc tcttgaaagc atatggtttt 8100

gttatcactg atcaaaaaga atctgggtgc ctgcctaata gactatatat ttcttgaggg 8160

taaggaacgt gtaactttct tgataatcta ctggcagttc cggtatatag caggtggtaa 8220

gtaaatggtc ttttaaaaat ctaaattggt tcatgaacca gtaactgttg gggttactgc 8280

tgagtgaagt gctttaattt tgttaaaaat aatattttca aattgtgaaa tacaaacttg 8340

aaaaactgca gtgcatgctg ggtgcggtcg ctcaggcctg taatcccagc actttgggac 8400

gccaaggtgg gcggatcacc tgaggttggg agtttgagac cagcctggcc aacatggcga 8460

aacccccgtc tctactgaaa atactaaaac aaaacaaaac aaaaaaaacc aaaaacaaaa 8520

attagccagg catcatggcc actgcctgta atccctgcta cttgggagac cgaggcagga 8580

taatcacttg aacccgggag gcagaggttg cggtgagccg agatagcacc attgcactcc 8640

agcctgggca acaagagcga aactccgtct taaaaagaaa agaaaaactt cattataaaa 8700

ttgtcacttt ggcatatgtt gtctctcatc tttcaactct aagataagtt aaataggacg 8760

aactgatatc ttaggacctg ttaggaaata attagcctat tttataagta catttggtat 8820

ttagaaattg gaagttgagg aatttattaa aaaggggatt tgtttaacat tacttatggt 8880

tttgagtttt ctggattgga gtataccatg tgttagaagg ggatatatgt tggccaatga 8940

tattatagat tggcttaatt agacaagtgt ccagacagaa ggaaaaggga agctaaattc 9000

tgagaattgc cagattatct tcaggcatgg gaaaaataac tggaaaagga cacttaagtt 9060

ttttaatgga agactacaac aaaatgatat tttctttata actgatcata ttacacattc 9120

ttactggatt tattgaatat aagaatattc ttatttccac aatgtctgtg tactagagaa 9180

caaaacacaa agcatatggt cttaaaaggg gcaaaattag ttagcttatt acactttggc 9240

catttaggcc tttcttttgt atgtttgttg ttagtatatc agctgattat ttttcattga 9300

cagcatattc actttaatct tgtatgtttt gaaattaagt ttagtgaaca tgtatgccta 9360

gttgtatcca tgtatccatt cctttggtta aggaggatgt ctttgtggta ggggttacat 9420

tatgaaaact tcagcagaat ggcacagaag attgacttta ttttcttgaa atttatttta 9480

tcctatttct tccagaaagg atatctggta acttaaaata aaagatctgg aagtgtaata 9540

aaatatgtgt aaaaagaaga aatcagggaa aggacaaagc atatattctt ttcccaagtg 9600

acagcataat tcaatgacta agaataaaaa ttgggtttga gtttgcttgg cagcgagggt 9660

aaaaatggaa gtatggtaaa atacatagca ctaaactaaa aggaggaaca agaccaattt 9720

ttcaggggtg aaatcctctc ttcacagatt tctaagaaac tatttaaact ggacattata 9780

taaaagctgt tttgttgttg ttagtggccc tagatttaaa aaaaaaaaaa aggtgagggc 9840

taagcattaa gatttcaagc caatgaaata cattttaggg gttgatttga atttaggctg 9900
cagatgtatg ttttatttgg ccaatatatg ttttttttcc ttaaaatatt tcaaacatac 9960
atgagactac agagaccaat ataattaaca ttcatatatt aaccatacag ctgtagcata 10020
tcttagcatg ttcccatatt tctcattttt ttgaggaaaa aaaatcatgt acagttgaag 10080
caacttcttg gattacagtt tactttcttc ccagaagtta actattattc tgagtatggt 10140
gttgatcttt tttttttttt ttttttggaa acagggtctc actctgtcac ccaggctaga 10200
gtacaggggt atgatcatgg ctcactgcag cccatctcct ttgcttgagc agtcctccca 10260
cttcagcctt ccaagtagct gggaactcag gtttacacca ctgtgcccag ctaatatttg 10320
tatattttgt agagattggg tttcgcctgt tgcccaggct ggtctcctgt gctcaagcag 10380
tccatccacc tcggcctccc aaaagtgctg ggattacagg tgtgagccac cactcctggc 10440
ctgatcattt ttaatgcatg tttttatgta attactacat gggtatatat tcacaaatca 10500
tagtattgct ttgcatattt tcagatttga tgtaaatgat atattctgct tattctataa 10560
tttgtctttc atgaaaattt ttttgattta tatgaaactg ctgatacgtt accattttga 10620
cctacaaaaa tgacagtttc atatggccca ccctaatagt catactttta tctgctgtaa 10680
aattattttc tattatatga atatagccag ataaaattta tttattctca tgttgatagg 10740
catttagatt gtttccaggt tttcatgtat taaaaataat gttacattga acattcctta 10800
ttaggaatca ttgtgcttac atttaagatt tttggcaagt tttaaaaact gcatgttgtg 10860
gctgggcact gtggcttgag cctgtaatat cagcactttg ggaggctgag gtgggcggat 10920
cacctgaggt caggagtttg agaccagcct gaccaacatg gtgaaactcc atctctccta 10980
aaaatacaaa cattagctgg gcggggtggc gcgtgcctgt aatcccagct actcaggagg 11040
ctgaggcagg agaatcgctt gaacctggga ggcagaggtt tcagtgagcc gagatcacgc 11100
cattgcactc cagcttgggt gatgagcgaa actccatctt aaaaaacaaa tgaacaaaca 11160
gaaaaaaccc cacatgtcgc gactccctaa tggattgtga aatcaattca ggaattagaa 11220
aaatatcagg ttgtattgca aacaataagg ataaaatgtt ttgtgaaatt tttatttctg 11280
tgtgtatgta tgtggttatt aagtttgaaa tgaagccctc tatggtgaat tacccagaag 11340
tgcaattatt gggctgaaga ttttggaggt tatgggaatt tttttagctg tattggatat 11400
tgtcaaattt gctgtcactt tgcatatttt agcactagag tatgagaatt tgccatcttc 11460
cgattgttat gattaatggc attgtcagat tttttacatg tttacaaatc agatgagtgt 11520
gaaatgttac cacattgtct taatttttat ttttgattat ggggttgagt atgttttcaa 11580
atgttagatg actattcaag tgtttgtttc ttgaattggc tgttcttttc cttccacagc 11640
catttgggtg gtctttctaa attgatttta gaaagtttta agttcattac atacttcgat 11700

tatgtttgtt aagtgaattg caactgtctt ttctcagtct gtgtggctta tctcttaact 11760

ttatatggtt tgttttgtta aatagaaatg attattataa aatatatcac tattttcttg 11820

tttgtgtttt atttaagaaa cctggggtga tcactctgtg gttctcctca tgcacattaa 11880

caagtttgtt acttcatttc ttcagttgat ctcccttttg tgaggtggtt tttaagtgaa 11940

tcattaaaga gcaaaggaga agttttcccg tggctcccac gctgctattc aaaacttcct 12000

gggttagtaa gccttcttgt gaattgtctt taaaatgttt attaaaaact cttaggaact 12060

ctcatataaa gtcttaaatt tgtgtacaaa gatgctctct gacattgctt gactagtgaa 12120

gaaagttaaa acaatctaaa tgttaaaaat atattatatg tatccagact tcagaatttt 12180

tttgtagaca ttaagagaat aagctagagc tatacgttgt gacatgaaaa tgcctactat 12240

atactagtta agttttttaa aaagtatttt gcagagaaaa tatgcacagt ataatcttac 12300

agtgcaaaat ttttgcaggc acatatgtat atgatatatg caatctatat atgcatatag 12360

aagggtctga aaatatataa tagttgccac tggagtggta tgtgtgtttg tgttgcttta 12420

atttttttca gtacacattt aaacagaagt aaaaaacaat ttaaaagaaa tttggctggg 12480

tgtggtggct catggctgta atctcagtac tttgggaagc tgagatagga agtttgcttg 12540

agcccaaggg ttcgagacca acctgggcaa cagagtgaga cctgtctcta caaaaaactt 12600

aaaaaattag ccaagtgtgg tagtgtgtgc cgggactgag gtgggaggat agctcgaacc 12660

tgggaggtca aggctgcagt gagctgtgat tgagccactg cactccagtc tgggtgacag 12720

agtgaaactc tgtctcacga ataaataaat aaataaataa ataaatggga gaatcttaat 12780

aaccttggag tatggaatag atttgtttgt ttttttgttt gcgacagggc tcaagtaatc 12840

ccaccacccc agcctctgta gtagctaaga ctagaggcat gtgccaccac acatgattaa 12900

tttaatttag ttttgtgagg acaggatctc actatgttgc ccaggctggt ctcaaactcc 12960

tggcctgact gatcctcctg cctcagcctc ccaaagtgct gggattacag atgtgaaccg 13020

ctatgccccc accagttttt tttttttttt ttttgggtct tggttctacc tggaattttt 13080

tttttttttt tttttttggt gtgtgatgtg aggcagggat caaatttaat tttattatat 13140

gtatacccaa taaccatatt actgttaaca gagtgttttg aatttttgag ttaataattt 13200

ttaaaagcag ctcatggttt cttgttaagc acaattgcag tctttttgtc cttcaccagg 13260

ctgcttcata tattcaagtt tgtgatctct gtgaccagag tgatatgatt ggtggatgac 13320

ttgatccaga aatagaacct agaagagtag atacagagta tattttgtag gtctcactca 13380

catgtcattt ctcttactat ttaaatggaa attagattgc actttctttc ttctctaata 13440

aacttgcttt cacttaaaaa agaaattgga ttgcagacaa tttgaggata tacattaatg 13500

aggaaacaga ttgctggttt tttgcattgc tggctacaga ccttggaaac tagagaagca 13560
catggttgat tgattgatat cttgttctaa aacttgagca tacttaaatg tatttggtca 13620
tccagctatt atcacatata ctaacgtttg agacttttaa ttttgacttt agggtacatg 13680
tatcaataca gttgtcttaa ctttctaaga tgtttgatat aaaacactgt aaatcactta 13740
gtttaagtat gaagaccatt tttgtttcct cttaagaggg tttttattac acttttagtc 13800
taagaagtgt tgctgctgag gccttcagat gtatattcta aatttttctt ttttcttttt 13860
attccttttc tttctcatac tggtggtcag aagaaggcct tatgatttag tagaattgag 13920
caatattgat aaagattgta gatacaagtt tatttttaga tttcttagac ttgtaaaatt 13980
gagaaagata attaaccact tccttgaatt ttccatttgc agcattataa agcaaaacaa 14040
acaaacagac aaaacagact tgtgctctgg agggtaatat gtgagatgag atagagcccc 14100
gaatgattgt tatttaataa tgggagaaag gtgctttatc acttcaaagt agggttttga 14160
ttttgcctga aaggttaact gtacaggcca catgttttat ctgaacagat tttatttttg 14220
aagtagttta aacagtgggc tatgtggtat tatagtacag atgatttatg gaaggaagtc 14280
tttacatatt tgatatattt tggcacaaaa tatataatcc tagatttttc aggatttccc 14340
tgactttaat atttagaact gttgttcctt gtattagcat ttttatgtac agattgagca 14400
tctctaatcc aaaaatctga aatctaaaat gctcccaaac ctgaaactat tcgacgccac 14460
aagtggaaaa ttccatacct gacttccagt gacaagttgc agtcagaact ttgtttcatg 14520
cacacaatta caaaacattg tataaaatta ccttcaggct atgaaacaaa tatattttgt 14580
gtttagactt gggttatatc cccaagatat tttattatgt atatacaaat attccagaat 14640
ctgaaaaaat tccaaatcca aaacacttct gggtactaat ctttgtttca aaaacaatag 14700
tcattattat agatcttggg aaatgaggat aaccgttgta atatggctgt aaaagagttg 14760
tagaaagact tttctccttt aaagacattg tttttgtagc tttgaccact accagtggct 14820
ataatgcatt gtttgtggct ttattgaagt cctataagat ggtgataaaa gcatggactt 14880
tgaagtcaga tagataaaga tttgaattca gggtcttcca cttaagtttg tgttttgatg 14940
aattgcttga tctccgtgaa cctcagtttc ttcatctata aaatgggacc ccacttcata 15000
aggattgttg gagatgatag gtgaagggtc aactactggg ccttgttcaa agttgtcttc 15060
agtagtaaag taacaattca ccttctccta cagagagata acatcatgat gtgggaagaa 15120
gggaactact tacttttgtg ggaggcagtg ttgtataata ggccatttgt tgcttctctg 15180
ttttgtacgt tgtgtaacct tagggagatt atccagaaag tttaccctca gtcttctcat 15240
tggtaagtag agataatgtt acctatgtaa cagcagagtt ttaaagctaa atgagaatat 15300
ttataaaagc tcactttatc tggcacatgg taacatggta acttctatta ataaaatttt 15360

atggtaataa gcaatattat gcttctgaag taggtcacaa gaagggcttt tggttggaga 15420

aaccagaatt gttatgaatt agtccgtgtt tactttgagg gagtaaagac tagggagtat 15480

tcattgaagg aagactgttt aggaggctac tattacttgg ctaaaataca aataatgtga 15540

ctcaatagtt tctcaaaatt ttgtgtaatc taggcttatg taaataaact ttactgtttt 15600

attaggattt aaaatcaact ttattgaaat ataatttaaa caatgaaata tactcatttt 15660

aggggtgcag ttagatgact tttgataatg tacataccca cccagataac catcacccta 15720

atcaaggtaa tggaatttct gttatctcca aaaccttcct cgtgtccctc acagtcagtg 15780

caccacaccc ctcccctcct ctggttcaag gaaaccactg atttgcatac attcattttg 15840

tctcttagaa tttcatgtaa gtgggctggg caaggtggct catgcccata tttccatcac 15900

tttgagaggc tgaggtggga ggatctcttg agccaggagt tcaaggctgc agtgggccag 15960

gatcacacca ctgcagtcca gcttagtgac agtgtgagac ccggtctcaa gaaaaaaact 16020

ttgtgtgtgt aaatggaatc atactgtaca tattctttaa tgcctggctg ctttcactcc 16080

gcatgttttt gagagtcctc catgttgctt tgtgttccag tagtttgttg ccttttattg 16140

ctgagtagta ttcactgtgg tatggctata ccacaatttg tttcttcatt cacctgatat 16200

ttgggtttcc agtttttggc tattatgatt aaagtggcaa tgaacattga tgtacaagtc 16260

tttttgtgga cttaagtttt tatttctctt gggcaaatag gagtagaata gctgagtcat 16320

atggtgtata ttttgcagtt tcttaataag ttaaacagtt tttcaaactg attgtgccat 16380

tttatcttcc tgtaatctca tttacattat atgctgtgtt tttaatttta actttttttt 16440

ttttagtgtg aattgatatc ttattgtggt ttttatttac atttttcttt ttaaaagttt 16500

tgttttttgt tgtgagacag ggtcttgtct gttgtccagg ttgatgtgat cctggctcac 16560

tgcagtcttg acttcccagg ctcaaggaat ctttccaccc cagccttcca agtagctggg 16620

actacaggtg catgccacta catccagcta attttatttt gtaaaaattt ttatgtagag 16680

atgagatctt gctgtgtgcc caggctggtc tttaaatctg gggctcaagt gatcttcccg 16740

cctcagtctc ccaaagtgct gggattatag gtgtcagcca ccatgccttg ccttatttgc 16800

atttttctga tagcaaatga tgttgaacac ttcttcatgt gttcattggc catttgtata 16860

tccttttatt agatgaggtg tctaattctt aattcatttt taaaaaagct tttaaaaatt 16920

actgagttac aagttcttta tatgttgtgg atacaagtac tttgtcagct gtgtgaattt 16980

tgaatataca gtcagcgctc cttgtctgag ggttccacat ccacagattt aacgaatcta 17040

agatggaaaa tgtttggtaa aaaaataaca atatgcgaat aaaaaataat acaaataaaa 17100

aatacagtat aacagctatt tatatagcat ttaatcatat taggtattat aaataatcta 17160

gagattgatt taaagtataa ggaagatgtg catatgcaaa tatgggtttt tttttttttt 17220

aaataaggga cttgagcatc ctcgaatttt ggtgttcagt tttgggtgga ttaggagggt 17280

agagaggggt tcttgaaacc agtccaccag ggataccgag agatgactgt attcttttgt 17340

tctgtggctt gcctttcatt ttcttaacag tctttggaag agcagatgtt tcttaatttt 17400

gttgagtcca gtttatcact ttttacttat ggttagtgct ttttgtgtct taagaagtat 17460

ttagctacac caaggttgta aagattttca cctgtgttta ttttggaagc cttacagttt 17520

tagacattac atttaagcct atgatctatt tcaagttaat ttttgtgtag gatgtgaggt 17580

aaaagttcag gttctcctct cccatatgaa tatccagttg ttcaagcacc atttggtaaa 17640

aagactatcc tttcctcatt taattacttt gacctttgtt gaaaatcagt tgtccataaa 17700

tgagtgggtc tttttattat tgcattgatt tgtataccta tacttttttt tttttttgag 17760

acagagtctt gttctgtcat ccaggctgga gtgcagtggc acagtcttgg gtcactgcaa 17820

catccgcctc ccaggttcaa gtgattctcc tgcctcagcc tcccgagtag ctgggattac 17880

aggtgtgcac caccacgccc ggctaatttt tgtattttta gtagagatgg ggtttcacca 17940

tgttggccag gctggtctcg aactcttgac ttcaggtgat ccacctgcct atacctccca 18000

aagtgctgga ttacatgtgt gagccactgt gcctggccct gcatatctat acttatgcca 18060

gtactacact tgattactgt agctttatgg taaattttgg ttactctaga ttctttgcat 18120

ttccataaat tttggtatta atagtgtcag tttctatcaa aaagcctgct ggaatttcag 18180

tttgtgttgt attaaatata cagatcatta gggagagaat tgtcgtttaa aaaatattga 18240

gtcttctggt ccttgaaaat ggtgtatctt attatttttt aaggttcttt agtttctctc 18300

agcagtgttt tgtagttttc agtgtaggaa gtgttggaca tcttttattg gatttattcc 18360

taattatttt aagtgcttac atgggaattt ttttaaagtt tcattttttc caatttggtg 18420

ctagtatgta gattacaatt gattggtggt ctgtctagca tgtagaaatt ccttaataag 18480

tagtcagatg gaatttaatt tgttctcatc taactttctg gagaaaataa tcatctaaaa 18540

gaaaaaagat tatctctgaa tttgaatggg ttttaaattt gtatgactgt ggcttatttt 18600

aatttctctg aatttgtaac gtagaagtac atttggattt tatggtaaaa tatacgatat 18660

tggatataag tggagacatg tagagatcat tcaacagatg agaggatata gctatagaaa 18720

agcagagttg aaaattttta gatggtaaaa tattcaattt tatattaaat ataatatttt 18780

aaagtagaaa gtgcttgagt agtatgatta aaaagaggaa acttgttttc atttgaatac 18840

attttggcct acaagaatgt ctgttactgg cttaagtatt tttgtttgtg tataccgtca 18900

gatgatttac catttcctag ccaaacctga tttgttggcc cagtacaact ctcttctctc 18960

ttttaagctg tgtctttgat agtatttctg ttcatcctgc ctagaagatt gtgttctcac 19020

acagttttct gttcttggtt ctaaaacatg gtgtaaatga gaggattgta aaatgcgcca 19080

atcagcactc tgtaaaaatg caccagtcag tgctctgtgg ctagctagag gtttgtaaaa 19140

tggacccctc agcacactgt aaaatggacc aatcagtgct ttgtaaaatg gaccaatcag 19200

caggatatgg gcggggataa ataagggaat aaaagctggg caccccagcc atcagctgca 19260

acccagtcag gtccccttcc atgctgtgga agcttttttc tttcgctctt cacaataaat 19320

cttgctgctg ctcactcttt gggtccgtgc cacctttaag agctgtaaca ctcaccgtga 19380

aggtctgcgg cttcattctt gaagttagcg agaccacgag cccactggaa ggaaccaact 19440

ctggacacat cttggtgacc cagacgggac cattggaccc cttttgcttg ctattctgtc 19500

ctatttttcc ttacaattca ggggctaaac accagggcac ctgtcggcca gttaaaagca 19560

actagcgcgg ccaccggact aaagacacgg gtgttaggct ttctgggaaa gggctctcta 19620

acaacccccg actcttcaga gttgggagcg ttggtttgcc tggaaccagc ttcctctttg 19680

cctgtacttc tgggctgagc caagggttga caagagggga aagccattca gctgcggggt 19740

cccaacaaaa agttggttga ccctgtagcc atgaggggaa ctctgtaggt cagccaaagg 19800

ttcagtgggt cggtccaggg gtccttggta gaagttgtta gttgaactca tttggggttc 19860

catttgtaag accatctata gcttgatggc atcgatcctg gaggaaacaa atttgacaag 19920

gagtttaaaa atacgggcct gaaggcgagt aatagcaaga tggctgttac gggacctaga 19980

aaggggagaa gccatgttgc ccagctccag aggttggtat aagagtttga aaggcgttgt 20040

ctgatttcag aagccttttc ctgtaaacgc caggtggcgt ctcatactat ccctcactgg 20100

ttagtgtaaa agcaacattc ttcccctaag aaggtgcata gtcctccttt ctcagcagtg 20160

aggaagtcta ggccttggcg gttttggaga gtcactgctg ccaaagagtc tatctgggat 20220

tgtagaggaa ggatagattt tgttatttct tgcaagctgt ctgagaaatc ctttgagagt 20280

gtgtggtagt aggataatga agtagataaa tctgctattc caattcctgt agcagtggcc 20340

attcctaacc ctataagtag gggtattagt tgtatggccc tgtgctgatg gacttgagct 20400

ttgaaggggc actgatagag tctgatttcc tggggcaatg ttaatgttgg gacttaggaa 20460

gactaaggtg taggtgcctg tccagttggt ggggaggcag atacaggttg aagttccact 20520

taagaagaat atgccttggc tgggtagaca gaaatttacc ctggctttta aaggaatagg 20580

gtacactgtt ttttctttac tacttctatc tctgtgtctc tctttgtctc ttcctctctc 20640

tctctctctc tctctctctc tttctctctt tctgacttct tttctgtctc ttcctctctc 20700

tgtttctctc tttctgactc cctctttgtc tgtctcttcc tctctctctc tttcctctct 20760

gctggtcttt ccctgcctct gccagctgct tatgctgctg ttctcccctc tcctttcagc 20820

agtgtaagac tgccacctcc ttgggttttt gcactgtgtg caataactcc gtgatttcct 20880
tatggtattt aatggggttc acccagaggt taggaactcc ctttctttcc atattgcatc 20940
atgggcacga aggattagat aagcatactt gttatctata cacatttatt ctttttccct 21000
ttcccagttc taaggcttgg gtaagtgcca ctagttctgc tatctggccg ctggtccctg 21060
ggggaggagg cttactttca agtactgtta catcactaac tatggcataa cctgcccttt 21120
gtatcccatt cttcacagat gaactgccat cagtgtatag gttaaggtca ggattagcta 21180
aggggacttc taagagatcc tcttgggtgg cataagtctg gactataatt tgttggcagt 21240
catgctcgat tggttcccca tcctctggga gaaaagtggc acggttgaga gccgcacaga 21300
tgcatatttg aagcatcggt ccctcaagga gtagcacctg gtatccaagc aggcggttgt 21360
ctgatagcca taaacttcct ttggcaccta gtgtgccatt tacatcatga gtagtccaga 21420
cagtgagatc ctttcctagt attattttga tagcctctga tataagacgg ccactgctgc 21480
aactacacgt aaacagtgag gctagccttt tgccactggt tgtagggttg tcccgtgagt 21540
ctgagtaagg acttcaagag ctattcctgc tctctctctg acgtataaag agaagttttg 21600
tcttgtggga aggcttaagg ctggagcttg tactagggcc tgctttaagg atttgaaggc 21660
tgtttctgcc tctggttccc attctactag atgagtattt gccctctggg tctcctttat 21720
tagagtatag agtggcctgg ccatcttgct gtatccaggg atctgtagtc ggcaaaagct 21780
ggtgattcca aggaaccccc gcaactgttt taatgtctta gggcgaggat aagccagtat 21840
aggctgtatt cgtttcttgc tgagggccct ggttcctctg gctaagatta ggcctagata 21900
tttgacttgt tgtaggcaga gctgggcctt tgatttagat gccttgtacc cttgattagc 21960
tagaaagttc aagagatctc gagtagcttg ctggcatgag gcttccgaac tggcagccaa 22020
aagtaaatca tccacaaact gaaggaccag agtgcctgga cttgagaagt ggcctagatc 22080
ttggaccagt gcctgaccaa acaggtgagg gctatccctg aacctttggg gcaagactgt 22140
ccacgtaagt tgggacgtgt gatctgtagg atcctcaaag gcaaagagaa actgggagtc 22200
agagtgcagg ggaatacaga agaaggcatc cttgaggtcc agaactgtga acgattctgc 22260
ttcctttggt atttgagaga acagggtata gggcttgggt acaactggat atagaggaat 22320
tactgcttca ttgatgagtc taagatcttg cactagtctc cactgactgg tttttgtact 22380
cctagaattg ggtgttgcca gggactgctg cattttctta ctaggccttg agcttttaaa 22440
tgtctaacaa tatcctgtaa tcctttatga gcttcaggcc ttaagggata ttgcctttga 22500
taagaaaaag tggtgggatc ttttagcctt atttggagtg ggtgggcata ttttgccctt 22560
ctgaattgtc cttccaatgc ccaggcttca gggttgattc cctcctcaag caggggacaa 22620
caaattggta acttgttccc catattcatg tagataatag ctccagcttt ggctaatatg 22680

ttcctcccta ataagggtat gggactttca ggcataacaa gaaaggcatg tgaaaagagc 22740

aaagtctccc aattaccact gagaaggtgg gagaaatacc tggttacaga ctgtcccagg 22800

attcctcgga tggtaatgga ccttgaggac agctgtccgg gacaggagat taacactgag 22860

cctcggatgg taacggacct tgaggacagc cgtctgggac aggagattaa cactgataaa 22920

gctgtaccgg tgtccaggag gaagtcaatt tcctggccct caatggttaa acatacccgg 22980

ggttcagtga gggtgatgac atgagctggc gcttgccccg ggcaccctca gtcctgttgg 23040

atcatctggt tgggggcttc tggcccagag aacctttgtc ctttggggca gtgcaccttc 23100

cagtgattgc cttggcatag tggacatggg cgagggggca gcttgtttct cgttggacaa 23160

tctttttaa agtgtccttg caaaccaaat tgataacaag cctaaccggg tgattggcct 23220

gctccatttt ctgtcctctc tgaaccacca aggtctgttt gtctgagggc catgactaag 23280

gctgtagcct ttctcttatc tcacttttcc ttttcagcct gttc[?]tctcg atccctactg 23340

tagaacacgg aggttgccag gtttaataat gcgtccaaat tttg[?]tcagg ggccagggct 23400

agcttttgga gctttatcct gatatctgca gctgactggg taa[?]aaactt atcttttagg 23460

atcaattgac cctcgagtga gtcgggtaac agggaagtat attttcttaa ggcctcctgt 23520

agccgctcga ggaaggcggt aggattttct tcctttccct gagttatggt ggatgtcatt 23580

gaataattca tgggcttttt cctaattctg cttagtcctt ctaggacaca ggttaacaga 23640

tgttcacgac tccagtcccc atgatctgag tcgaggaccc ggtggggatc cacactgaga 23700

atggcttgct gaccagtagg gaatttgtcc ctttcttcga ctgtcattct gtcatttacg 23760

tgactgagat gccaggtatc tccaaactct tgggctgcaa ctaaagctgc attcttttca 23820

ttaaaggcca gggtttgatc taacaatagc atgacatctc tccaagtgag gttgaaggtt 23880

tgccctagac cctataggac atctatatac ctatcaggat catctgaaaa cttccccagg 23940

tctactgtca tctgctttaa atcagagaga gagagaaggg gacatgtacc tgggttgggc 24000

caaattcccc tccccctaca gcttgaaggg gacataaccg atagcctggg agtttttgtg 24060

gtcctttgga gatttctttg cttgtttcct tctgggtggt ggagattaga ggaggcttat 24120

cattaatagg aaggggagct gtaggaaggc taggatatgg gggtaagctg agaggtcctc 24180

ctccccaata ttgcaagctt tgcatagttg tggattatcc ttcagtgaaa agaaagcttg 24240

gacataaggt atttcactcc gtttgtcttc cctcttacag aaaaggtcaa gctgcaggat 24300

agtattgtaa tttatatttc cctcaggtgg ccatttttga ccatcagaga gagaatat[?]g 24360

gggccaggcc atagtgcaga aaaaaatgag ccgcctcttt ttcagggttt gcaggtcaaa 24420

ttggtcccag tggcttagga tgcatttcaa gggtgagcct gttgatgcct gtgtgttccc 24480

catctgaaag aaaaaacctc ctgtggtttt ggtttgttcc ccgctaccct ctcaaccccg 24540
agttatcgtg gctttaaaaa ccggaccatt gtcgctttgg ctggcctata gaattcccct 24600
ctggaggaca ctaccactgc agggcccctt ctttacccct atccagcagg aagtagctag 24660
agtgggcatc gcccaattcc tagcagcagt tggggtgtcc tgtttagagg ggggattgag 24720
aggtgaagcc agctcgactt ctgggttggg tggggacttg gagaactttg atatcttaca 24780
agaggattgt aaaatgcgcc agtcagcgct ctgtaaaaat gcaccagtca gtgctctgtg 24840
gctagctaga ggtttgtaaa atggaccagt cagcaccctg taaaatggac caatcagtgc 24900
cctgtaaaat ggaccaatca gcaggacatg gagggggaca aataagggaa taaaagctgg 24960
ccaccccagc cagcagcggc aacctggtcg ggtctccttc cacaccgtag aagctttgtt 25020
ctttcgctct tcacagtaaa tcttgctgct gctcactctt tgggtccgtg ccacctttaa 25080
gagctgtaac actcaccacg aagttctgcg gcttcattct tgaagtcagc gagtccatga 25140
acccactgga aggaaccaac tccgcacaca taaagatgag gaaaattgtt cttttggttt 25200
tgacgacttt actatgtgca gttagcattg ctgaatgtgg tggtctctga gagcagtgta 25260
attctgatga tacatacagg catttgtcta ttaaaaatgg gatgaaattg aagatggttt 25320
attgcctttt atatcatctt ttggatttgt gataaagaca aacctttgaa aatgtttgta 25380
gttattaata tgaccagaaa ctgaaactgt ttcagtttgg cacaggagag attagtagta 25440
gtattgctgt tagtcattat gtaagagatg ataatgtttg gatttaacct tcgattgttt 25500
gtaatatgat attcctaaca ttcctgacta aaaggctagt gaataaaaaa taatgcattt 25560
acaactatat taaaactgca ttgcagaaag catatgaaat ttcaagttta ggaaatttcc 25620
tgctttgaaa cttttttctt cacacttcac gctttcagca cttatgtgtg atgcatctag 25680
aagatgcatc tggttttgcc aaatacagta ctgcacctga attacatttt cggtatttat 25740
tcagtattta ttttcagtct ttatttccta aaggttaatt ttctttaaaa ccttagcaat 25800
ttttcatcca gcccttttca tgttataatc atttgcttgt gtgattttac cacacactgt 25860
atccaactag gtagcattac tggaaaactt atttgatttt ttgccaaatg ggagatggct 25920
ggaatatttt ttgtagtaga gaatatgtcg agaaaatgct ttaattgatc cctgaccaaa 25980
tacagcaaga aaaacttaag taaaccttaa taagcaggca atagaagtta aggtgataat 26040
attctgactt tggatataaa cactcaaaca tgagctctgc tactggaaag taaaaaataa 26100
aattagtgtt aagttgagat gagtcaactt gttgatcatc tgcctagcct actgtgatag 26160
gaatggttgt aagacttgga agtttttcct tcctaagttg ataagcattt gagtggaaag 26220
aaagtcactt ttctcttgat gttttttcac tttggaattt tcagactgtt taaagtcttt 26280
taaaattttc tgagtcactt ctttattttc taggctgctc atattaagag aagtattgcc 26340

tttttatcct ttaaaatgta ataccctcat ctttagtggt tgtccctgct taggttatat 26400
tgcttagaac ataacgttta tgtttggtta aaatttcact gagccaccgg gcacggaggc 26460
tcacacctgt aatcccagca ctttgggagg caaggcaggt ggatcacctg aggtcgggag 26520
ttcgagacca gcctgatgaa catggagaaa ccctgtctct gctaaaaata caaaattagc 26580
caggcgtggt ggcacatgcc tgtaatccca gctacttggg aggctgaggc aggagaattg 26640
cttgaacctg ggaggcggag gttgtggtga gctgagatcg tgccattgta ctccaacctg 26700
ggcaatgaga atgaaattcc atcttaagga aaaaaaaaaa attcagcttc tgaaaagcac 26760
tagggactaa gcaagttgac tatgtaggct ttattattag atttgtgtgt ttgtattata 26820
aggaaaacaa atttttagta aaatataatg tattttgttc attgtttagt tcattgctgg 26880
attgctggtc tctagaactt ttcctggtac agaataaaca tataataagt attttttaaa 26940
tgatgtactt aattttatta ccctaccatg cttgaaactg tgactttata ttatagtttc 27000
ctttagcaaa aggtgaaact atagtcaata tatagattat aagtaaattt ttaaaaatct 27060
tcatttaccg taatagcatc attttacatg tatacctgct tttcagattt gtaactcatt 27120
tggtttaagt ttgcggcatt tggatgtttc tgatgttgaa tgataaaatg taggcttaaa 27180
aaaattatcc ttcgagtttt atgctgctgt attgtgtata gcctctcttt cataatgata 27240
cttggactgt gatggtataa agtgactctt attttaatct gaatgcattc gagatatttc 27300
aagttcttga aagactgtta cacttcttgg gatggtcatc atcatatttt tgaagcagag 27360
atcctaaaac ttaaaaacct cagagaaagt ccagcatcta ttttcaactc cttgtttttt 27420
aaagctttag aacttgactt tgtactcttt ggtgaggtga caaaacaaat tcagattttt 27480
ttttttaact gaggacttca aaaaattttt gtgatgttta tagtattttg tctcttttta 27540
gaaagtcaaa tttgttgagg tatagtttac actcagtgaa attcacccat tttagatgta 27600
tggttctgtg aattttgaca aacattactg aaatggctaa agtcgactgc tatctagcag 27660
acagtgctaa aatagcaact tagcagttaa aatgtcatct ttttatcact ttgggaagat 27720
tctcattggt atctttccat gggtacaata agagaccagc agtgtggcag gctttcactg 27780
tctttttccc tcaactctac ctcacataaa ggtgagattt tcctattttc ttaggaaagt 27840
cagctagtgg ggtacggtga taagctagga cgggttcctt gtgattttat ttaaaggttg 27900
gtctacttaa tgctcgggga aaaaagaagt gtagtgatac aacatgttgc ctacctttac 27960
attttcaaaa taattttaag gaaataaact gctgtttgat tcgaaatgat tctttgtgtt 28020
atggcagtta agattttatt aacaccaggg ctcgagaatg cctgttaaaa atctaggtgt 28080
tgctccttct cagctaccaa ctatatcttt ttttaagggc catattagta acatatgttg 28140

ggaacttttg ccatgttacc aattatgctt tttcagtttt ttttttgaga cttttctact 28200

caaaaaggtt ctgagtattt agaaagtact ccaattgtaa tcattctttt tattgcttct 28260

ggaatgtagc agggctccat gttcttcccc atttcttgac agtttcttaa tatcttggaa 28320

cattgttgta gagtataatg tcctatctgt gttgaaatac ttggctttgg catttaagtg 28380

ctatcatgca tttatttgag aacagtttat ggctcattca gacattgtgg ataagagcat 28440

tgacttaggt gtcaggcaga ataaatttaa tctgactctg ccatttagta actatatgaa 28500

catggataag ttaattataa ctgctctagc tgttttgtca tctgccaaat ggggaaaata 28560

atcatatgta tcttccagga ttgttgtaag gattaaagag ataatccttg ggaaatattt 28620

tattgcatgt agtaaacact cagagcatgt tagctgctcc tgtcatcctc actcagcaga 28680

agtttttttt tgttttcatc tttttttttt ttttggagat agtcttgctc tgtccccagg 28740

ctggagtgca gtggtgcagt ctcagctcac tgcaacctcc gcctcctggg ttcaagcagt 28800

tctcctgcga cagcctgtgg agtagctggg attacaggta tgtaccacca tgcccagcta 28860

attgttatag ttttttagca gagatggggt tttaccatgt tggccaggct ggtctcgacc 28920

tcctgacctc aagtgatctg tctgccccgg cctcccaaag tgttggaatt acaggcttga 28980

gccaccgtgt ctgtcctctt ttcatcccag accatgcttg gtgttgtgga tacaaagtga 29040

attctaaaga aggcctcccc atcttaagaa gtttcatagt tcaagaaggg aagacaggca 29100

tgtaaacaga taaatgcaat aagttgtgac atatgctata tcagaaacaa atgaaaagaa 29160

taaaggagtt agtatcataa gcctggggag gtcagggaaa gacatttcta tccctgactt 29220

atttttgatg tgtgaatagt agtttcctgt tgacttattt gacagatatt tactgaatac 29280

actttacttt tgccaggcac tattttaggt gctgggaata ccactggttt catggaagac 29340

aatttttcca tggactggtg ggcagggggt ggaggggatg gttttgggat gatccaagca 29400

cattacattt attgtgcact ttatttctat tattactaca ttgtaatata taatgaaata 29460

attatataac tcaccataat gtggaatcag tgggagccct gagcttgttc tttcacagct 29520

agactgtccc atatgggagt gatgggagac agtggcagat catcaggcat tagattctca 29580

taaggagcgt gcaacctaga tcacaatagg gttcaggctt ctatgagaat ctaatgccac 29640

cgctgatttt acaggaggcg gagctcaggt ggtaatgcaa gcagtgggga gcagctgtaa 29700

acacagatga agcttttgag cccctcctgc tgtgaggcca ggttcctaac aggctatgga 29760

attggcttgt agcctagggg ttggggaccc ctgctgtata ggagttgtga aaggaaatta 29820

aatcttgggg ccctcaatcc ctaaactaaa ggaaaaagtc aagtggggaa ctgcttaggg 29880

ctaacctgcc ttccattcta ttcaaaatca tccctctgct cactgagata gatacatatc 29940

tgattgcctc ccttggaaag gctaatcaga aatgcagcca ttggtctctc acctaacttt 30000

aacctggaag ctccctccct gatttgagtc ttcttgcctt tgctttaagt tgtcctgcct 30060
ttccagacca aaccaatgta cttcttacat atattgatcg gtgtctcaag tctccccgaa 30120
atgtataaaa ctaaactttg ccccggccac cttgagcaca tgttgtcagg acttcctgag 30180
gctgtgtcat gggagcgtcc tcaacctagg caaaataaaa taaacttttt tttttttttt 30240
tttttttttt tttttgaggt ggagtctcac tgtcatccag gctggagtgc agtggcacga 30300
tctcggctca ctgcaacctc cacctcccgg gttcaagtga ttctcttccc tcagcctcct 30360
gagtagctgg gattacaggc acatgccacc acacctggct aattcttgta tttttagtac 30420
agatggggtt tcaccatgtt ggccaggctg gtcttgaatt cctgacctca actgatccgc 30480
ttgccttggc ctgccacagt gctgggatta caggcgtgag ccactgtgcc cagcctgcaa 30540
aataaacttt ctaaattaac tgagacctgt ctcaaatttt cagggttcac agagtaaaga 30600
agacagataa tgtctctact ctcaggagct ttcagtctag tgggaaaaga tcagtagtaa 30660
acaaaaatat cagagacaag tgctatgaaa gagactaaaa ggtgatgtaa taaggagaga 30720
ctgaggggtt gctttacatt gcattgtcag ggaaggatcc tttgagtagg taacctttga 30780
gatcttccta caaaagagtc agccatggca aagattagag ggatgagcat tctaccatag 30840
ggaaccatgt tacacaaagc cccttgagct cttggagtat ttaaggagca gacaggtcag 30900
tgtggctgga atgtaatggg caaggggtag agtggtgcaa aatgagatca gagatagtca 30960
gggtctaaat catttaaggc tttgtaagcc agagtaagga gtttctaggt gtaaacgaaa 31020
gccagtggag ggctgtagca gggggaacgc cttttgaaaa agattactgt cgctgccgtg 31080
tgaagactgg attctgagga taagggtaac aagggtggaa tttaggagac ctctggaggc 31140
tgctgtatta gtataggtga gaggtggtgg tggcttgggc cagagagtaa attagtggag 31200
aaaaataggt tgatttgggc tgtgttttga agattgaatc aacaggactt gctgactgca 31260
tgtgatcaag gcaaatccta ggacttgagc acttagaaga ttggtggtgc agtttactga 31320
aatggataag actggagggg agagcaagtt tggtgggaca atcatgagtt ctgttttgtt 31380
aagtttgagg tgccaattag acatctatgt agagatttca tacttggtgg taggtaatag 31440
tagtactgtg gaattcagtg agagtttagg gccagggata aatatttagg agtaactgat 31500
gtatgtctgc tagatttggt aagtgacctt gacaagtgtg gtcttagtgg gaatggaaac 31560
tagattgtag agaagtatca ggtaaaggat ggaatgagaa tatgaagttg gggaatggag 31620
acagtgacaa tagactccta agaaattttc ctgtgaaaag ggaagcagaa aaatggttgt 31680
gaccacaagt aaggcccaga ttcgaatctt acttttatac ttgcctggat acagttatta 31740
tttgagtgca ttctaaagtt taagatattt tatgtaccct acgcagtcct ctctacaaat 31800

attgaggtag atgatacttt tccttctgtg acatgagagt ccttagttta gggaagttaa 31860

ctgacagtcc aggtagaacc tgtatttgga ctcactcact ttgactgtag atgcagagta 31920

tttttcatta taccaaaatg ctccttaaac ttttattatc ttggtcaaat ccaatctacc 31980

ttagagtttt tggaaggatt acaagatgag agatgtgaaa attctttgca agctctaaat 32040

tgttacacaa atatagaaat tcctttcttt aaaatgcccc attatgtctt ctttgtgcat 32100

gactttttag gatatagtag gtagtggcct tgaagttcat tttggatcat agcctatctc 32160

attagagccc agagaatgta agcagattgt taaagacata gaacctgtca aagctatcta 32220

tggcatatga atcgaacatg gatcatgttt aattgaacat ttttaccctg gtttaatgat 32280

cttgtctcct tcgcatgttc catgaggtct cataatcctc cccaaaggag ctcttcccaa 32340

aaataattcc ttctaaaaat gtggaagggc tgggcacagt ggctcatgcc tgtaatccca 32400

gcactttggg atgctgaggc aggtggatca cctgaggtca ggaattggag cctggccaac 32460

atagtgaaac cccgtctcta ctgaaaaaaa aaaaaaaaaa aaaaaaatta gccaggcgtg 32520

gtggtgggtg cctgtgcctg tactcccagt tactcaggag actgtcggaa gaattgcttg 32580

aagcgagagg tggaggttgc agtgagccaa gatcgtgtca ctgcattcca gtctgggtga 32640

cagagcaaga ctccatctca aaaacaaaac aaaacaaaac aaaacaacaa caacaaaagt 32700

ggaagtaatt ttaaaaaaac tgcaaaaaag ccaatcctga aaggttacaa actatatcat 32760

tccaaaagcc gtggaaaaca gtaacgtgag tcctcaaaga attaaaaata gaattaccct 32820

gtgatccagc aattctactt ctgggtatat actcaaaaga actgaaagca gcagggtctt 32880

gaaggggtat ttctacaccc atgctcatag cagcattatt gacaaatgct ggaagtagtc 32940

cagctgtccc caaatggagg aacagataag caaaatgtta ctggtataca tacaaaatgg 33000

aatctttttt tttttttggg agacagagtc ttgcactgtt gcccgggctg aagtgcaatg 33060

gtgtgatctt ggctcaccgc aacctctgcc tcccgggttc aagcgattct cctgcctcag 33120

cctccctagt ggctgggatt acaggtgccc gccacgacgc ccagctaatt ttttgtattt 33180

ttagtagaga cggggtttca ctgtgttggc caggctggtc ttgaactcct gacctcgtga 33240

tccacctgcc ttggcctccc aaagtgctgg gattacagat gtgagccacc gcacccggcc 33300

acaaagtgga atcttatttc acctttcaaa ggagagaaat tctgacatgc tacaacctgg 33360

atgaatcttg agaacaatac actaagtgaa actggccagt gaaaccagtc acaaaaagac 33420

aaatactgta tgattccact ttaatgaggt acttagagta gtacaaatca cagagacaga 33480

aagaatggta attgccaggg gttgaggaga agagggaatg gagagttact gtttaacggg 33540

tatagtttca gttttgcaag ttgtagagtt ctagagctag atggttgtaa gggtcacaca 33600

acaatataaa tgtactcagt aacattaact gtgcacttaa agataattta gatggtaaat 33660

tttatgttat gtgtatttta ctatgccatc atgcctggct aatttttttg ttttgttttg 33720

attttctggt gacagggtct cactctgtcg cccaggctgg aatgcggtgg cacgatcacg 33780

gctcactgta gcctcgactt cctggactca agtgatcctc ccatcacagc ctcccaagta 33840

gctgggacta caggtgtacg ccaccactcc tggctaattt ttactaactc actccctttt 33900

ctgcctcctt tcagtattca ttcattcatt taacagataa tgactgaaca actgttaggt 33960

attattaggt attatatatt attcctggga gcagactatt aagagtcact ctataccatc 34020

acagtcccaa gtgtcattat gaaagagggg ctatacacaa tacagcagta taaaactgta 34080

aacagatttt ttaagcctac attttatcag tcaacatcag aaacatccaa ataccgcaaa 34140

ctttcttttt tttttttttt gagacagttt cactcttgtc acccaggctg gagtgcaatg 34200

gcgcgatctt ggctcactgc agcctccgcc tcctgggttc aagtgattct cctgcctcag 34260

tctcccgagt agttgggatt ataggcatgc gccaccatgc ctggttaatt tttgtatagt 34320

agagacgggg tttcactata tgttggccag gctggtcttg aactactgcc cccaggtgat 34380

ccacccgtct tggcctccca aagtgctggg attacaggtg tgaaccacca cacctggcca 34440

aatgtcccaa acttaaacca aatggactat aaatcaattc cctgaaaaac tggtatacat 34500

gtaaaatgat gctattacat tacagtaaat gatctttaaa aatttattta tagtctatat 34560

gttgactaca gttttacctt ttgctaaagg aaactataaa gtcgcagttt caagtatggt 34620

agggtaataa aactaagtac atcatttaaa aatacttatt aaatgtttat ctttgccagg 34680

caatgttcta gagaccagca gtccagcaat gaactaaaca aaatgaacaa aatacattat 34740

attttactaa aaatttgttt tccatataat acaaatacac aaattaataa taaagcctac 34800

gttaatgcat tgtactaatc tgtcttggtg agaagttcaa tttatgaaga tacagtagaa 34860

atgattttaa tttctttttt ttcccctaga gaatgtgatg tttattcatt aatttattca 34920

ttcactcaag aaatcttcat tgaatgtcta ctgtgtatca gttgcagttt gggattcagc 34980

agtgaacaaa acataaatgc ttgcccttat ggaacctaca atctgttggt tttaaaaaaa 35040

atctctcctg gattagtgtt agatttctag accagaagtg tcaattaagt ttaacaattt 35100

cttcaggcag agaaaattgc ttttgaggta tcacttaagc aaccttagtg cttctaagca 35160

acataactgc cttttgaggt atgaactgaa gggaagtgaa gtataagcaa tagagttggg 35220

attttttttt tcttgagacg gagtttcatt cttgtcaccc aggctggagt gcaatggcac 35280

catcttggct cactgcaacc tctgcttcct gggttcaggg gattctcctg cctcaccctc 35340

ctgagtagct gggattacag gtgcatgcca ccacagccag ctaatttttg tatttttagt 35400

agagacgggg tttcaccatc ttggccaggc tagtctcgaa ctcctgccct caggttatct 35460

gcctgtgttg gcctcccaaa gtgctgggat tacaggtgtg aaccaccacc cctggctgag 35520
ttgggatttt ttaattttcc atagatttcc cattaggaag gcaacctggt gtgacggtat 35580
ttgagagtca agagatctgg gttctaatcc tcatttcatc atgtaatact ttgtgtgttt 35640
cagaattctt cacagtgatc cttgcttgtc ctcactcctt tgtacagctt ttttttttaa 35700
aggattttat ccactgttgt gccagtaact atgctagtgt cactttttat gctagtaata 35760
gttctatttt caagttcatt ctgtttcctg agtttaaatt tgtgtttcct agtgccaact 35820
gaacatttct gtcatttcat actccatttc caaaaagaaa ctcattattg ttccgctttg 35880
aatttgcttc ccttctttat gatcttttca tctcggcaaa tagcattgct atgtacccag 35940
ctgcctaagg aaaaaccctg agtcgtcatc tttctatatt tcattgaccc tcattcagtc 36000
attcaaatat tgcttctctt gagtatatct tgaatctatc ctttatgctc taatttctga 36060
acagaactga cactctttgt cctgcatgtt ctactttctt tgcacagaat accttctctt 36120
tagttggcaa actccaactc attggttaat gtccagctca aatgttaact cctgtaaaac 36180
cttcaccatt tcttttaggc agaattagtg attcctgagt ctttgctctt agagcttgta 36240
ggtcaagtgt gtactgtatt tctgttcttt cacagttttt tattcgattg tctgtagaat 36300
agactttatt attgtcaagg ggaagacagc cttagcaaaa ttttgaaata agtcattgtg 36360
tgcctaagca taaattagtt tgtgagaaca tactttttaa agagaaatgt agtagggtgt 36420
atagtatgtt tgagtatctt tttccaataa gcattgcatg tataatcatt cactcattgc 36480
atgtataatt attatttaaa tattctttat taattaaaga cctctctggt ctctccagaa 36540
gtcttccatg gctgccctca tccccaccct ccagaggaat ctgaagccac atgtgctccc 36600
tggcacccac agcccctgcc tctcccagag cagcagtacc tgagcctcag tggattccaa 36660
gaatcgaaac ccttggtctg ctgcccctcc ctcccagaat gtttctgtct cattcttacc 36720
tactcaaggc cctttcagta gccccttggg gtattctctt cctatgcacc agggcaactt 36780
ctgaactcac cactttcctg gaaagacccc caacatcttc ggggagcggt cccctacagg 36840
cctggctgca gatgcccgtg cacacaggaa cactgtttaa aaaaaaaaaa aaaggatact 36900
ggtgggaggg gctttgtaac tgaggaggtt taggaattga aaactcatgt gtattttctt 36960
tctttcctca ttgtcacttc ccagctaccc agagatattg aaggacagct gagctcccct 37020
acttgctgaa aatgcattga tcaaatttgt gtaggatttc cacatctatt tatgggctca 37080
gaacactttc tagttacttg ttccttccta tcagctgttt tctgtttgtg ttagattttt 37140
tttttttctg agatgaagtc tcgctcttgt cccccaggct ggagtgcagt ggcgcgatct 37200
cagctcactg caacttccgc ctcccggttt caagtgattc tcctgcctca gcctcccaag 37260
cagctgggat tgcaggcacc tgccaccacg cccggctaat tttgtatttt ttagtagaga 37320

tggggtttca ccatgttgat caggctggtc tcgaactcct gacctcaaat ccgcccacct 37380

cagcctttca aagtgctggg attacaggca tgaacaccac acctggcctc atgcagagct 37440

ttaaaaaaaa atgttaacac ggctgggcgc agtggctcac gcctgtgatc ccagcacttt 37500

gagaggccga ggtgggtgga tcacttgagg tcaggagttc cagaccagcc tggccaatat 37560

ggtgaaaccc cgtctctact aaagatacaa aaaattagcc gggcgtggtg gtgggcgcct 37620

gtaatcccag ctactccgga ggctgaagca ggagaattgc ttgaacctgg gaggcggagg 37680

ttacagtgag ccaagatggc gccattgcac tctaggctgg gcgacagggc aagactccgt 37740

cttaaaaaaa acaaaaataa ataaataaat aaaagaaagc tctgcatgat acaatacagt 37800

attcatagaa ttttggccag tgaaaatttt ctgtgttagt agaataaaaa tgaactcttc 37860

tgatttacag atattgatag tctggtcttt ccatcttcca tcttgactct cttttactgt 37920

taaaaaatct tagaacaaag taaagccagt tccaaatact tttcctagtt ctggactgtt 37980

tttttttttt tttttttttt taacaagaca gtcatttgtc cgagtacaac agaacagttc 38040

attgttaata ctttggttct gtgtgcttta aatctttttt ccccctctac ccataatctc 38100

cctttgtaaa ctttgaatgt tttgtcagta gatcatcatc ataggtaaag atactgcaca 38160

tcaagactgt tggtcttccc cattcttcct ggaatatact ggaagctgtc aggactgagg 38220

ttaatttctc cattcctaga actatattgt aataaatatt ggaatactgg cttttgtgta 38280

tttttttgtg ttacttagtt tttggagaaa ggaaatattt ctagactttc ttgacttgtc 38340

ataacttttt attgttaaag aatatagttt aaaaattatt aaatattagg gagccttctg 38400

cttcattggg actttaagga atgatagcca taggaaggga tatatcttat attatcataa 38460

agcttttaaa aaataatata tttattacaa ttttttaaat ttatttttta tttttagaaa 38520

cagggtctta ctctattgcc caggctggtc tcaaacttct gggctcaagt gattctcctg 38580

cattgacctc ccaaagtgtt gggattacag gcatgagcca atgagctcag ccagttgttt 38640

tttttttttt tttttttttt tgagtgtttt tttttgataa cttctaatta ttttcttatc 38700

ctatccctaa tagttggcat cagttaatga actagttttg attccccaaa agccctctct 38760

taatctcctg tattttgctt tactttctat ctcttttgtt tatttcttag agaaatgtca 38820

tttatgtaat cagttcttat tttgtaatac tgggccgtaa gaaagcaggg aaaagcacaa 38880

aacttggcac atagtaaatt ttgttgcttg aacaaattaa tgaatgaatg aacttgcagc 38940

ataagagtgc accctattta gtggtactgt ttaattattt catgatcctt agccaagaac 39000

gaaggcatac cggaagggaa cacacaacaa aaaaaggtga gggaaaagtg ttaatatatc 39060

atttaagtga ctgtattaca acaatattac attatgttgt cactcctgaa catgagatgt 39120

gattctcatt tacatttcaa gctaatttgg ttagctgggg gaattcttag gcatctcaag 39180

agataattag atatacccct taggacctca cataattata atcactatta ggggtattaa 39240

agtttttttt tttttttaaa caaaaactta gggggaaaag ttgtgattgg taagcgtttt 39300

tttctcttag tgttctcaga atcttaggag atacgttatt tgaaacagtt gttcaggccg 39360

ggcgcggtgg ctcacacctg taatctcagc actttgggag gccaaggcga gtggatcatg 39420

aggtcaggag atcgagacca tcctgactaa cacggtgaaa cctcgtctct actaaaaata 39480

caaaaaatta gctgggcgcg gtggcgggcg cctgtagtcc cagctacacg ggaagctgag 39540

gcaggagaat ggtgtgaacc caggaggcgg agcttgcagt gagccgagat agtgctactg 39600

cactccagcc tggctgacag agcaagactc tgtctcaaaa aaaaaaaaca aaaaaaacca 39660

aacaacactt gttcaatcaa ttctacttta gtagaaacca gctctctata ttgttctcta 39720

acattttgtt atcttggctt ttagatgaaa tctttagcat cagttgggct cattagcacc 39780

tctactaaga gaagcaagca cagaatggaa gggagagcaa atcataactt tttaaaaagt 39840

tatttctcct tagttaaaag tttggaagag aagcaagttg aaactgtagg ctgattatgt 39900

aaaagtttaa gttatcagct ctcttatatg taatgcaggt tgattataag tttctgggct 39960

gttgtgacaa caagaaaatt atgtttatta ttaaaaactt gtttatactg catatatgtg 40020

aagggcatta tactgtgaca aagctaacaa taaaacatac attttaatat ttcagattcc 40080

ttattttatt tgtttgttta gagacagggt cttaatgttg cccaggctgg agtgcagtga 40140

tgcgatcatg gcttactgaa gcctcaaact cctgggctca agcgatcctc ctccctcact 40200

ctcccaagta gctgggacta caggcatgtg acactgcgtg gctgatgttt tgtagaggcg 40260

gcatcttatt atgttgtcta ggctggtctc gaactttctc ccgcttcagc ctcccaaagt 40320

tctgggagcc actgtgctgg gccagattcc tttttttaaa ttgtgggaaa atacacgtaa 40380

cataaaattt actatattaa ccattttaaa gggtgcagtt cagtggcatt aggtatattc 40440

acaatgttgt acaatcatca cactacctag ttccagaact actttatgat cccaatcaga 40500

aactatgcgc attatacaat aactcctcat tgttctcttc tcccagttcc tggtaagttc 40560

tattacactt tctgacgcta tcagtttgcc tgttctaagt atcatataaa tggaatcata 40620

tagcatttgt ctttttgtgt ctggtttatt tcacatagcg tacatgttat agcatatatc 40680

aaaattttat tccttttggc cgggttcggt ggctcatccc tgtaatccca gccctttagg 40740

aggccgagct gggcagatca cctgaggtca ggagtttgag accagcctgg ccaacatggt 40800

gaaaccctgt ctttactaaa aatacaaaaa ttagccgggc gtggtggctg gcgtgtgctt 40860

gtaatcccag ctacttgaga ggctgaggca ggagaatcgc gggaacccgc ggggcagagg 40920

ttgcagtgag ccgagatcgt gcagctgcac tctagcctgc atgatagagt gagacttggt 40980

ctgaaaacaa aaacaaaaac aaaaaacacc aagtttattt aaaaaaaaaa aaagcttaga 41040

ctttttttga gcatttttat gttatagaaa aattgagtga aaactagagt tcccatatat 41100

ctacccgctg cttggtacca ccagtttccc gttactaaca tcttgcatta gtgtggtaca 41160

tttgttacag tttatgaact gatgttaata catttttaat taaattgcat agttttgcat 41220

tagggttcac tctgtgtgtt gtacattctg tgggttttga cacatgtatg aaaggtatcc 41280

actattacag ttatcacaga atagtttcac tgccctaaat actcagtgtt tcatctactc 41340

atccctaaat ccctgccaac cactgatctt tttactgtct ccttagtttt gtcttttcca 41400

aaatgtcata taattggagt catataatat gtaatctttt cagattgatt tctttccatt 41460

ggcaatatac acttaaggtt ctccatggct tttcatggct tcatagctca tttcttttta 41520

ttgctgaata gtacactgtt gtatggatgt gtaccacagt ttgtttattc attgacctat 41580

taaagactat cttggttact tcccagtttt ggcagttatg aataaagcta ctataaacac 41640

ttgtatgcaa gtttttgtgt gcacataggt ttttaactca tttgggtaaa ttgtatgcct 41700

ttctaacttt tgtatttatg catacacttt tatatagttg taaaacttag ttttatctag 41760

ttgtaataat tttgttttat gttttaagat aacataaaca atcttcatgc tgtttttgaa 41820

tgctccctaa tattaaaatg agaaaatgaa tttattatgt actttatttt tctgttactc 41880

attaattgct tccattttcc cctgccttaa taaatacttt tgtgtgtgta gtttttccct 41940

tttgtattgc tttaggataa aattcaagga gaaacagtaa agagtacaat tattgggatc 42000

aaggccattt tagataattc ttgatatgca gtaaagtttc acaatgggaa accttgttag 42060

atttacagtg gaggtttgct gcagtttatt ttgtggcttt ctagacaaga gtagcaccag 42120

ttatctgtgt aattttactt agaaaaaaac aaagaacttt tcactggttc tttgaagtgg 42180

tagtaagctt ccctactgtt ggttttgttc aaaaatcact gtttgctatc tgaatcttct 42240

tgattacttc agaactttat gtaatagttt attgaggttc ttaggtacag gtagctaaca 42300

cgggcttgac tttatcttac tatggtacaa tctaaatata tatttagtgt tgttaattaa 42360

ggttatcatt cacagatgct cccttatata taggtggaga aactgaggct tcccttgaga 42420

cagcatgctc ctggatataa ccgtgagagc agttaaactc ttgagacctg cattctttat 42480

ttttcttaat tatcagtaca attattggat cagagtttct tctatctttt ttgtgagtca 42540

ttaaattcca tttttaaaag gtatcttttg aatacataca tttacaggtt tggaaaatat 42600

atttctagtt aaaccatgtg ttcttattta tttatttatt ttttattcta agttttaggg 42660

tacatgtgca caacatgcag gtttgttaca tatgtgtaca tgtgccatgt tggtgtgctg 42720

catctattaa ctaatcattt aacgttaggt atatctctta atgctatccc tcccccctcc 42780

ctcaacccca caacaggccc tggtgtgtga tgttcccctt cctgtgtcca tgtgttctca 42840
ttgttcaatt cccacctatg agtgagaaca tgtggtgttt ggttttttgt ccttggcgat 42900
agtttgctga gaatgatgat ttccagcttc atccatgtcc ctacaaagga catgaacaca 42960
tcctttttta tggctgcata gtattccatg gtgtatatgt gccacatttt cttaattcag 43020
tctgtcattg ttggacattt gggttggttc caagtctttg ctattatgaa tagagtgccg 43080
caataaacat acgtttgcgt gtgtctttat agcagcatgt tctctaatcc tttgggtata 43140
tacccagtaa tgggatggct gggtcaaatg gtatttctag ttctagatcc ttgaggaatc 43200
gccacactga cttccacaat ggttgaacta gtttacagtc cccccaacag tgtaaaagtg 43260
ttcctatttc tccacattct ctccagtacc tgttgtttcc tgacttttta atgattgcca 43320
ttctaactgg tgtgagatgg tatctcattg tggttttgat ttgcatttct ctgatggcca 43380
gtgatgatga gcattttttc atgtgtcttt tggctgcata aatgtcttct tttgagaag[?] 43440
gtctgttcat atcctttgcc cactttttga tggggtcgtt tgtttttttc ttgtaaatt[?] 43500
gtttgagttc attgtagatt ctggatatta gccctttgtc agatgagtag attgcaaaag 43560
ttttctctca ttctgtaggt tgcctgttca ctctgatggt agtttctttt gctgtgcaga 43620
agctctttag tttaattaga tcccatttgt caattttggc cattgctttt ggtgttttag 43680
acatgaagtc cttgcccatg cctatgtcct gaatggtatt acctaggttt tcttctaggg 43740
tttttaggtt tttaggtgta acatttaagt ctttaatcca tcttgaatta atttttgta[?] 43800
aaggtgtaag gaagggatcc agtttcagct ttctacatat ggctagccag ttttcccagc 43860
accatttatt acatagggaa tcctttcccc atttcttgtt tttgtcaggt ttgtcaaaga 43920
tcagatagtt gtagatatgc ggcactattt ctgagggctc tgttctggtc tatatctc[?]g 43980
ttttggtacc agtaccatgc tgttttggtt actgtagcct tgtagtacag tttgaagtca 44040
ggtagcgtga tgcctccagc tttgttcttt tggcttagga ttgacttggc aatgtgggct 44100
cttttttgct tccatatgaa ctttaaagta gttttttcga attctgtgaa gaaagtcatt 44160
ggtagcttga tggggatggc attgaatcta taaattacct tgggcagtac ggccattttc 44220
acgatactga ttcttcctac ccatgagcat ggaatgttct tccatttgtt tgtgtcctct 44280
tttacttcat tgagcagcgg tttgtagttc tccttgaaga ggtccttcac gtcccttgta 44340
agttggattc ctaggtattt tattctcttt gaagcaattg tgaatgggag ttcactcatg 44400
atttggctct c[?]gtttgtct gttattggtt tataagaatg cttgtgattt ttgcacgttg 44460
attttgtatc c[?]gagacttt gctgaagttg cctatcagct taaggagatt ttgggctgag 44520
acaatggggt tttctagata tacaa[?]catg tcatctgcaa acagggacag tttgacttct 44580
tcttttccta attgaatacc ctttatttcc ttctcctgcc taattgccct ggccagaact 44640

tccaacacta tgttgaatag gagtggtgag agagggcatc cctgtcttat gccagttttc 44700
aaaggcaatg cttccagttt ttgcccattc agtatgatat tggctgtggg tttgtcataa 44760
atagctctta ttattttgac atacgtccca tcaataccta atttattgag agttttagca 44820
tgaagcattg ttgaattttg tcgaaggcct tttctgcatc tattgagata atcatatggt 44880
ttttgttgtt ggttctgttt gtatgctgga ttacgtttat tgatttgcgt atgttgaacc 44940
agccttgcat cccagggatg aagcccactt gatcatggtg gataagcttt ttgatgtgct 45000
gctggattcg gtttgccagt attttattga ggatctttgc atcgatgttc atcagggata 45060
ttggtctaaa attctctttt ttgttttgtc tctgccaggc tttggtatca ggatgatgct 45120
ggcctcataa aatgagttag ggaggattcc ctctttttct attgattgga atagtttcag 45180
aaggaatggt accagctcct ccttgtacct ctggtagaat tcggctgtga atccatctgg 45240
tcctggactt tttttggttg gtaagctatt aattattgcc tcaatttcag agcctgttat 45300
tggtttattc agagattcaa cttcttcctg gtttagtctt gggagggtgt atgtgtcgag 45360
gaatttatcc atttctccta gattttctaa tttatttgcg tagaggtgtt tatagtattc 45420
tctgatggta ctttgtattt ctgtgggatt ggtggtatca ccacccctgt atcacaatcc 45480
cctgtatcat tttttattgt gtctatttga ttcttctctc ttttcttctt tattagtctt 45540
gctagtggtc tatcaatttt gttgatcttt tcagaaaacc agctcctgga ttcattaatt 45600
ttttgaaggg tttttttgtg tctctatccc cttcagttct gctctgatct tagttatttc 45660
ttgttttctg ctagcttttg aatgtgtttg ctcttgcttc tctagttctt ttaattgtga 45720
tgttagggtg tcgattttag atctttcctg ctttctcttg tgggcattta gtgctataaa 45780
tttccctcta cacactgcct tgaatgtgtc ccagagaatc tggtatgttg tgtctttgtt 45840
ctcattggtt tcaaaaaaca tctttatttc tgccttcatt tcgttatgta cccagtagtc 45900
attcaggagg aggttgttca gtttccatat agtttgtttt tgattcagaa aatttggtca 45960
gaattagtgt ctacacaatg ggcctgtttt catgtccacc aggcattatg gctgagtgaa 46020
aagccagaag ggcccgattt tgaacttttc ttccacttac cagccatgag actgagcaac 46080
ctgcacaaac tgtgagcctt gaattcttca gttgcagaaa tgaggtgttg aatccttcag 46140
gttgttatga agaataaatg agataatggg cacctgacag catatattta tgttaggaat 46200
atataattat tgctgtttga aattgtagcc tgtatatatt tgcagagtgc ccaaatactt 46260
gttcctcaga tacagagttt atgtacatct caatgtacct tttatagttg aagttgtttt 46320
gttttgagag aagactgaag ttatttgtgg ggttttttgt tgttgttttt tttcttacag 46380
tattgtcatg tggaaaatac atgttaatta tcagggttta ggttttctgc tgagtgtgtt 46440

tcaaattaag tcatttccct ctagaattgg tagtttgttg tcttcatgtt atttataatc 46500
atttccaagt ttaccagata gaaaaaccct taatgaaatt ttggaaaaga agtacaccag 46560
tgagttgttt gctaatcttc ctgtagttgt cacacagact gactgtttat atattttttg 46620
gagacagagt ctcactctgt cacccaggct ggagtgccgt ggtgtgatct tggctcactg 46680
tgacctctac ctcctgggtt cgagcaattc ttgtgcccca gcctcacgct tgggtaattt 46740
tttgtatttt tagtagagat ggggtttcac catgttggcc aggctggtct tgaactcctg 46800
acctcaagtg atctgcccat cttggcttcc caaagtgcta ggattacagg cggagccact 46860
gcgcctggcc cagactgacc ctttacagca tcactcctgt tccttgcata ttcatgcttc 46920
caatttaaga ttttgtccat tacttattac agttgccatg tatgaaggtc cctttttttg 46980
ataattggtt cttttctctt ctacatacta aggcaggggt gggaataaat gtcacagcat 47040
taccactact tgatttgatt ttaatgtgtt actctcttgt actcctacct tattagtttt 47100
agtagtttat ggctgggtgt ggtagctctt gcctgtagtc ccagtacttt gggaggctga 47160
gatggatgga ttgcttgagc ccaggagttt gagaccggcc tgggcaacat gaagaaaccc 47220
tgtctctaca aaaaaaaaaa aaaaaaaaaa gttaaccagg catggtggta tgtgcctgtg 47280
gtcccagcta ccccggaaac tgaggtggga ggatcacttg aatctgggaa attgaggctg 47340
cagtatgagc tgtgattgca ctactgtgcc cagcttgggt gacagagtga gaccttgtct 47400
caaaaaaaat ttttagtagt ttctgtgttt tgtttgtaaa tttaactctg tattgagcca 47460
gaaaagcaca agccctgtaa tttctcattt tagagaaaaa cattcatttt tttccttccc 47520
caaggagttt ctccttcttc ctattttttt ctttaaagag agtttggcag aattccagag 47580
cctaaaataa taaaataaag gtaattaaat gaattctcca gccagcagag accatcttat 47640
cttgaattgc tagtggttta tgctggtccc cctttgacct tctttcactt tttggaggtg 47700
gatgaacttt tccactcaaa actatcttgt cagcctcaca catatttatt aaaggagtga 47760
gtatgcttaa gagacagatt ttttgtgttg gttgaagaaa ccattttgga tatttttctc 47820
tgttgtgact gggactgaat ttttagattg atttatattt cctttgaaga acaaaggaat 47880
atttttctcg aaaatgagaa ttaacaggag cattaataac aacaaaaagt ctgttaaacg 47940
tatttgggga gattatttgt tttcaaagaa taacatttct tgtgtgagtc attatattca 48000
gcaaaatttc ttttgtgata attttatgct gccataaaat tttttgggaa aataacttta 48060
aaaaaacttc aaaaaatttt attttagagg ggcagtagta cagttggaag aggaatttta 48120
agtcaaagaa cagtaaaaca tgaattcttt cagaagacag ttgaaaattt actgtgcatc 48180
aggaacatta cttagctctg aacataaaaa atgatgaagg tactgctctc tgcctttatg 48240
tctttgcgtg tagtctaatg ggagacacaa gtaaataaga aacaatgggt caaggtcatt 48300

atagtagtag ttacacaggt aggtgcatat gcttctagtc tagccagtgg gatgctgtgt 48360

aaattgtagc tatgtggaga agccttgaga tttccctctc tgaaagtaaa attattgcct 48420

tagggcaggg ttagcaaact atagctagcc cctgggtatc tacctgtttt tgtatggctc 48480

atgagcaaag attggtgttt acatttaaaa atagtttaaa caaaataatg ttttgtgata 48540

gatgaatgaa aattatatga aatggaaatc tcagtgttaa taaagtttta ttggaacaca 48600

gccacattaa ttcatttaca tattgtctga ctacttttgt gctgcactag ctgagttgag 48660

ttttttgtag gagagacctt ttgtggccca cgaaacctaa aatatttata atctggcctt 48720

ttattataga aaaagtttgt tgagccttct ttagagtgtt atgagcactg actttttatg 48780

acctcaatca cacatgcagt ttccttaaat tccttattat atgtttaaaa gttaaattag 48840

ctgggcgcag tggctcacgc cagtaatccc agcactttgg gaggctgagg tgggtagatc 48900

acctgtggtc aggagttcga gaccagcctg gccaacatgg tgaaaccccg tctctactaa 48960

aaatacaaaa aattaaccgg gcgtggtggc acatgcctgt aatcctagct actcagaagg 49020

ctgaggcagg agaattgctt gaacccggga ggcagaggtt gcagtgaact gagattgcat 49080

cgttgtactc cagcctgggc gacaagagcg aggctccatc tcaaaaaaaa aaagaagtta 49140

aattttaagt ggctgtataa aggattatga tgatgcctac cttgaatggt aaacttttag 49200

atgtaacttt tcagttattt ctgctgaaaa atgctaaagt gatgtgctat tctattgaaa 49260

gccaagtgta ttgatttgct aggcctgcca taaagtactg cagactgggt ggcttaaatg 49320

acagaaatgt attttctcac agttctggag gctagagttt caagatcaac atgtcagcag 49380

gtttggtttc tcctatcacc cctctccttg gcttgcaatt gttgccttct ctctgtcctc 49440

acagggcctt tcctctgggc atgtattcct ccctggtgtc tcttcttaca aggacatcag 49500

tcatagtgga ttaggccccc ccaccttgta acttcattta accttgatta cctctctaaa 49560

gcctctattt ccaaatacag tctcattgta aagtactggg ggttaggact taacatatga 49620

attttgagag gctacaatca gtgcacaccg ccagggaatt tctgatagaa acttcatttg 49680

tttacacatt aagcagatct tgagggtgtg tactgtgtga cagatcctgt tttacgtatg 49740

tatgtgtgtg tactatgtga cagatcctgt tttagataca tatggtgaac gagacaaaca 49800

agatttgaca cttaagaggc tggtggttta gttgagaaag acagataatt aataggaaaa 49860

taaatatttg agagagtgat aaggactgtg aaggaaatta aactgtgtca agtgttagaa 49920

tgattagtgt gacgggtcgt caggtagggg cttcctgaaa aggtgacctt ttacctggga 49980

gcttaaatgg taagggatag ctagctagtt aagttcccag gcatgtttct agggcctgat 50040

gttgatcaag attggtatgg aaaaggttca aaagaatagt gagtcaatta gggagcttta 50100

taagttgagg ttggagaggt tggtggggca aaatcattaa gggccttgtg ggaattatgg 50160

taaagatttg gcttcctttt gcacaaggaa taaaatctgg agggatttta gcaggggagt 50220

gacatgattt ttctgttcga aagagcactt tggctgctaa ataaagagag gaaactgcag 50280

gtatgagagt agaggatagg gaggcctgtt aggggattat caaagtattg taggtcagag 50340

atattggtgg tttagctaag atcatggcag ggaagagaaa aagaaaggga aataatttga 50400

gagatatttt gggagataaa atccatgtat tatttactaa tggattaagt atgggggctt 50460

aggaagaggg aggaatatct ttggcaatag tgttgtgtaa tactgtccac tgtagcaagt 50520

gaacctatag tgttagcaaa tcagatagat gattgctgca gcagcaagtt acagaatata 50580

atttggaaag gaaaaaagcc ttcaatcatt aaataggaaa acagaaggca ggtaaacaat 50640

gctatggttt aattatgttc aacttacagg cttataacta ctagacaacc aatggggaat 50700

tcatagtttg ccatagagaa tgcctttggg acacataacc tgtttggaat gaaaaagccc 50760

tggagagtat tgtaaaataa caaatgtcag gtttgatgga atgactatgc atgcaattaa 50820

gtgattttct ttagtgctag agttcttttt tcttaggtaa atcattttaa aaaatggacc 50880

agactttgtg aaagtagata tgttacataa aataatgtta gctaaagatg aatggaatag 50940

gttaagagtg tcttcttgaa aatttgaact atatctcttt caaaaagaat tttattacct 51000

ctaggctgga cttgactgaa aagttaaaaa aaaaaaaaga caaaatctta tgattgttga 51060

actcccaatg aggagtcaga gaattgtgag agccagggtc tgttttttgt cacttttctc 51120

tctctctttt tttaagatag catcttgcta tctgcaggct gaagtgcagt ggtgcgatca 51180

cagctgcttg cagctttgaa ctcttaagct caagcgatcc tcccacctca gtgtcctgag 51240

tagttgggac tgcaggtgca caccaccacg cctggcttat ttttgcattt tttgtagaga 51300

cggagtcttg ccgtgttgcc caggctggtc ttgaactcct gggctcaagc catctgccca 51360

cctgggcctc ccaaagtgct gggattacag gcgtgagcca ctgtgcccag ctgccacttt 51420

tttctatgtt tagaaatcta cctctgagcc ttattcccca tccataaggc aggtaaccac 51480

tagatggcta attttgaaag agcgagagca ttcagagatg cacagtaatt ctaagttctg 51540

agtagaatac agtcatcctt tggtatccat gggggattgg ttccaggacc tcccatggat 51600

accaaaatct gtggatgctc aagttcctga tagagaatgg catagcgttt gcatataacc 51660

taagcacatc ttcctgtata ttgtaaatca tgtctagatt acttgtagta ccaaatacaa 51720

tgtaaatgcc ctgtaaatag ttactatact gtattgttta gggaaaaaag tctacatgtt 51780

cagaatagat gtaattttaa aaatctggat aatttccatc tgttgttggt tgaatccaca 51840

gatgcagaac cccattgata tagagggctg actgaataca aaagcaaggc aagtgtgata 51900

gcagccttct tttactttag gattcaggat ttacttcttt acttcaggat caaatatttt 51960

tacttttcga ttttattttt ttgtttgtga agagttggga gttgctgggt ttagttatgc 52020
tactctcttt ttaagggaga atatagcagt cctagaggga tttgcaaact atagctcttg 52080
gggaaacatt caaaattttc cttgtgtaag aaattttatg agtctcgagg gccggatgtg 52140
gttgctcatg cctgtagtcc cagcattttg agaggccaag gctggaggat ctcttgaggt 52200
caggggtttg agactggcct ggccagcatg gtgaaacccc atctctacca aaaaaaaaaa 52260
aaaaaacaaa gcagtgagcc aagattgtgc cactgcactc cagcctgggt aacagagcga 52320
gacgctgtct aaaaaaaaaa aaaaaaaaag aaattctatg agtcttaaat tacatcaagt 52380
aactttggag gcaatagttt aaattctcta aaatactggg cacattagct catgcctgta 52440
agcactttgg gaggctgaga tgggaggatc acttgaggcc agtagttcaa aaccagcctg 52500
ggcaacagag tgagactcca gtgctacaaa aaattaaaaa ttagctgggt gtggtgggca 52560
cacgcctgta gtctaagctt ctctggatgg aggctgaggc gggaggattg cttgggcaca 52620
gaaattcaag gttacagtga gctgtgattg tgccactgca ctccagagtg agaccctatc 52680
tctaaaaata ttaaattaaa aaaaacccat acaactcaaa tttccaggta ctgttattaa 52740
tatgagttat cttgaactgt tgaaaaatta atcacttatt gttgatttct atctgcatat 52800
taaagatctg gcttaagagt cttgcatttg atgaataggc ttttctatgt tatatacaag 52860
aaaaaggaga taaaataatt taactttttt attacattaa aaaacaggac tctgggaatt 52920
gtcactttat gatcagcctt atattttttc cagactgtgt cattgtgtat gcctgtgcca 52980
cccaacggat gagaccactg atcatgttta gctcaaattt atgttggata gaggttatag 53040
gatggtcctt atctgaatgc agaaaggaat gtttattttg ctgttatgtt ttatatggga 53100
taaaatgctt gaaaagtctt ttttttttct gtattttttt ttttgagacg gagtcttgct 53160
gtgtcgccca ggctggagtg cagtggtacg atcttagctc actgcaagct ctgcctccca 53220
cgttcatgcc attctcctgt cagcctcccg agtagctggg actacaggca gccgccacca 53280
cacctggctg attttttgta tttttagtag agacagggtt tcaccacgtt agccagaatg 53340
gtcttgatct cctgacattg tgatctgccc gtctcggcct cccaaagtgc tgggattgca 53400
ggtgtgagcc accacgtcca gctttttttg ttgttgttgt tttctgagac agagtctttg 53460
gctctgtcac cctggctggt tagtagttgg aactataggc gcgtaccgcc atgaccggct 53520
aatttttgta tttttttgta gagatggggt ttcaccatgt tgaccaggct gatcttgaac 53580
tcctgacctc aagtgatcca cctgccttgg cctcccaaag tgctgggatt acaggtgtga 53640
gccaccacat ctgacctact tgaaaattct taaatggtgc cttatacttt ctggtaataa 53700
aagcactatt ttaatgttcg gaactatttt cattatttgc taaccttaca attaaaaaat 53760

ccttttatat ttatctttgg atagatatga ggggacctca aaaagtttgt ggaaaaatgg 53820
aattaaaaga taaaaataca aaatataaac tttattctca atataagctc tatcgagttc 53880
aagaaaattt tgtaagcaat aatgctagcc atttagtcca ctcctaaaga agtcctgtaa 53940
ccatatcagt atgtgcagtc ttttttttgc attattaatt aaagaaaaat gggtgccctt 54000
taaatttttt tttaaggttc ggaaacaaaa agaagtcaga aggagccaat cagcactgtt 54060
tggtggatgc ccagtgattt cccattgaaa ccctagcaaa actgcccatt tgagaggaat 54120
gagcaggaac cttgtggtgg tgaaagtcaa caagattcct tgagcattcc aaaaagcttt 54180
tgtcatgacc ttcgctcttg atgggtttgc ttttgctcta actggatcac tttcatctct 54240
tggtacccat tgctttgttt ttttctttgt cttcaggatc acactggtaa agtcatgttt 54300
catctcctgt taaaattttt caaagaaatt ccacatgctc ttgttgccac ttgtttaaaa 54360
tttgcattga aggcactgct tttgtctgtg gccgatctga gcacaatggt tttagtaccc 54420
atcaagcaga aagtttgttc aacttaaatt tttttgttag atttgtatac gctgaatgaa 54480
tttatatgtc tatggtattg gctgttgttt gtgctgttaa tcattggatc tctacattta 54540
ggttacaaac gggatgaatt ttttcctaaa aaatttatgt tgatagtctg ctactgcagg 54600
cttcatcttc aatatcatct tgtcccttct tacaatgaat tatcctattt ataaactgct 54660
gatttcttag gggcattgtt tctataaagt tttcgtaaag caccagtgat tttaccattc 54720
ttccactcaa acttcaccat aaatttgatg ttttgttctt gcttcaattt tagcagaatt 54780
catgttgctc tgttagggtg cttttcaaac caatgtctta tccttcttag tgcctcaaac 54840
tacatcttgt tcagatgtgt tataacaagt tagtaccagc ttattttggt gcaaaatttt 54900
attgaaattc atgcatagtg ttttcataaa acatatgtta cacgaacttt ttgaagatcc 54960
cttgtacctg tagtccaaaa ttcagaagtt actaaagaat gtaaaataaa aataagtctt 55020
cttcctatgc ttgttttcac ttacccagtt tccttcctca tcttcctgat ggattaaaaa 55080
aaaaatgaca tgatctaaaa aatatgatct attttaggtc taaggacatg tataggttga 55140
aagtgaaagg actgaaagag ttatcacatg caaatagtaa ctaaaagaga gctgggacag 55200
tttgctaata tcagacaaga tagattttta gttaaaaact cttataaaca cttaagaggc 55260
tgggtgtggt ggctcatgcc tgtaatccca gcactttgag aggctgaggt gtgtggatca 55320
cttgaggtaa aacttcatct ttactaaaaa tacaaagatt agccgggcat ggtggtgtgt 55380
gcctgtaatc ccagctactc aggaagctga ggcagaagaa ttgcttgaac cagggagacg 55440
gaggttgcag tgagccgaga tcaagccatt gcactccacc ctgggcgaca gaacaagact 55500
gcctcaaaaa caaaaaacaa aaaacaaaaa aaaccctctt ataagagaca aggaagagca 55560
ttaaatagaa gagtcgatac accaagaaga tataacaatt ataaacatgt atgtactgaa 55620

catcagagct ccaaataacc tgaagcaaat attgacctca ttgagcaaca gacagatcta 55680

aaataataac tggagacttt aatatgccac tttcaataat gggtagaaca actagacaga 55740

tctagtctag ttagaagtag agtggaatta taggcatggt ggctcacgcc tataatccta 55800

acattttacg agactgaagt gggaggattg ctggagctca ggagtttgag accagcttgg 55860

ataaatagtg agaccttgtc tctactaaaa attaaaaaat tagctgggca tgttattgca 55920

cccctgttgt cccagctact tggaaggctg aggtgggaag aggaagattg tttgaaccca 55980

ggagtttgag gttgcaatga actgtgatca tgtccttgca ctctagtctg ggcaacagag 56040

taagaccttg tctcccccac taactccact gccccccccc aaaaaataaa aaaaacaaaa 56100

caaaacaaag aaataaaaga ctttaacacc gctgtaaacc agttgggcct aataggcatg 56160

tatactccac tcagtagcta tttttctcaa gtgcacatag aacattcttc aggatagacc 56220

atatactatg ccataaaatt agtcttaata aatttaaaaa gattgaaatt acacaaagtg 56280

ccttttctaa tcacaatgga atgaaagtgg aaatatgaga atgagaactg gaaaattcac 56340

aaatatgtgg aaattaacac acttaaccat tgggtcaaaa atcacaaggt aaattagaaa 56400

atatcgagac aaaaacacag cataccaaaa cttaggggtt gcaatatata tatcacttca 56460

tacaattggc ttttcaaaat cagttaagac aaaaggtaaa catttatata attacaggta 56520

ctctttcttt tttgttgggc ggtggtgggt ggtacgtgga tttgaattgc tgtttgaggt 56580

cacttgcttc caggcttttg catttctttt aagggggatc ttccaggaac aaaatctgtt 56640

ttattgcttt ttaaaatctg agaaggtctg tttcgccttc agttttgaaa tgtagttttg 56700

ctagatataa gattggttga caggtttttt ttttctttta tttgaacact ttgattatgt 56760

tatctcattg ttttctgtct gccattgttt ctgcttagaa gtcagctgct aatcttactg 56820

ggttccattt taaaataatg agtcgtttgg ttcttgctgc ttttaagatt ttctccttga 56880

ctttgacttt caacattttt actgtgaggc atctgtctgg atgtccttgc ctttctctga 56940

gttaaaatac attgagtttc ctggatgtgt ggtttattgt cttcagtaca tttgtggagt 57000

tttcagccat tatttctttg aatttttttt cccctctacc actttttctc ctctcctttt 57060

agagtaccaa actgtgccta ttggtgtccc acatagctct aagattttcc aatttactgt 57120

tgagcccccc caccctttat ttttaaattt ccattattgt aattttcagc ttcataattt 57180

ttatttggtt cttttttgta gtttacatct cttcattgat ctctatctga ggtgatattg 57240

tcattatgct ttcctttact tttattattt atttattctt tttttagaga cggagtctct 57300

ctctgtcgcc caggctggag tgcagtggcg cgatctcggc tcactgcaaa ctccgcctcc 57360

cgggttcacg ccattctcct gcctcagcct cccgagtagc tggaactaca ggtgcccgcc 57420

accacgcctg gctaattttt tatattttta ttagagatgg ggtttcaccg tgttagccag 57480

gatggtctca atctcctgac cttgtgatct gcccacctcg gcctcccaaa gtgctgggat 57540

tacaggcgtg agccaccgtg cctggcctgc gttttacatt taagtctatg atccattttg 57600

agttaatttt tgtatgaagt gtgacctaaa tttacaggtt gaagtttttt ttttaagttt 57660

tttgtctatg gatgttcaat tatttcagca ccatttattg aaaaggttat ccttccatta 57720

ttgaattgct ttggatattt gtaaacgtgt ttagggaaaa actctcctca aactgtgttt 57780

ttcctctact gtcataccac agcaataatc aacacagaag aagacttctg tgatcacaca 57840

catggggttg ttttccccac acaccaagca gcagacagca gcctgagtgt cctctaattc 57900

agttcgacac tatctccggg tcattggtgt cagatcccac aggttgaggg ctccgtacct 57960

aagactactc ccctaacaca cacaccagtc ataagtcggg gcctccagaa cttctgacca 58020

accagcttca agttggggct cccatggccc cctctttggg ttcattaatt tgctggactg 58080

gcgcacagaa ctcagggaaa cacttattta cgcttactag tttattataa agaatattgc 58140

aaaggttaca gatgaaggga cgtgcagggc tacgtatggg ggaaggggtg tgaaactttc 58200

atgtcctccc tggatgctcc acccaccagg aacctctgtg tgttcagctg tcttgaagtt 58260

cactgaaccc tgtcctcttg ggtttttatg gaagcttcat gacatcaaca ttcctacccc 58320

aagggcatag atgggaccct acatgagaga gtcttaagac ccgcaatcaa aaagtcaggg 58380

gaacattaag tgtggaaagc aggcaggaga aagaggcctg cctctgaggc ctaacacttc 58440

caacattata atgaaagact ggaataaagg ctatgggagt taaggccagg aaccatggaa 58500

gaagaccaat agatatcata gcaccacaat aaaataacag gcatacttgt gtgggtcatt 58560

tctgggttct ctgttctgtt ctgttactct atgtgtgtat tcttttgctc ataccaacac 58620

agtcttgact agctatgtag ttggccttca gtgaggtctt tttcgattgt tttagctatt 58680

ttggagcttt gccttccatg taagttttat aatgagtttt tatctacaga aatccttgct 58740

gggatcttga taggaattgt attaacctat agaacaattt ggacagaatt gacatcttta 58800

ctgagtcagt atattgctga gaacatagta taacatattg ctatttgttt agtttttttt 58860

tttttttttt ttgagaaatg gggtctcatg ctgttgtcca ggctggagtg cagtggtgtc 58920

atcatagttc actgcagcct caaactcctg gactcaagca gttctctcac cacattctct 58980

tcagtaccta gactacacag gtgcatgcca ctaaactcag ctaactttta attttttttt 59040

agtgacatgg tcttgttgcc caggctggtc tcaaactcct ggtcttgaag tgatcctctt 59100

gcctcagtct cttgtatttg tttagatttc tgatttattt catcagcatt ttggaatttt 59160

tagcatatag ggcctatacg tattttgcta agcgtatact taaagtattt aattttctat 59220

ggagcagttg taaatggtat tgttaaaaat ttcagtttct gcgtgtttat tgttaatata 59280

tagaaaagta aatgatggct gggcacagtg gcttatacct ttaatcccag cactttggga 59340
ggccaacgtg agaggatcac ttgagttcag gagttcaaga ccagcctggg caacatagcg 59400
agactccatc actacaagaa attaaaaaaa aaaaaaaaag gccaggtatg gtggtgtgca 59460
cctgtaatcc taggtacttg gcaggtggag ggcaggagga tccgttgagc ccaggagttc 59520
aaggttacag tgacgtatga ttgcaccatt gcactccagc ctgggcaaca gagtgagacc 59580
ctgtgtctct agaaagaaaa gagaagtgat tgatatttga gtgttgatct tgaattcttc 59640
ctgaactcat tatttctaga attttttttt gtagatcctc agtaacattt taagatttgt 59700
tttgaagtgt tttatctttt ttctattgtg atgtattctt atttcataat gaccaaatat 59760
tgtggggttt taggatgaag gctgttgaaa tccatatatc aattttatgc cccatcacat 59820
tgatgatttt tcttatttat aattttttct ttagttgatt ctcctggatt ttatagataa 59880
aagagtcata taatctgcaa atagttacag tttacctttt tttcccccaa tttttatact 59940
catttttttc cctcatttaa ttatctaaga cttccaagat agagttaaat aatgctggta 60000
ataccatttt ctggctttag actcttagac ttgtttgctt tttttttttt tcttcaaggc 60060
attgcatgct agtaggagtt cattttattt cagtaggtag aatgcaaata gagtaaaaca 60120
tatagtaaat ttcagttctc tagtgtgttc tagtattggg gtgttctgtt ttgttaaatt 60180
tatgggaacc tgaaggttga cataaatact tttacttcaa cagttctact gttgaaagtc 60240
aatattatag gccttcctgc tttctgtaga gtggccaata tttgatttct ttgtagattt 60300
tgcagcactt tattttcttc attcttattt tatcatactt gtcaatttag aaaacagtat 60360
tgataggaat gtatattgat ctcaggactt tactggagag ttgattttct tctttttatt 60420
ttaaggagga tccttaaaaa ttattgggga actcttattt gttgtcattt gatttcttct 60480
tctgagatga ggattacaaa acaattctaa ctaatggagc ttttcagttt ttctttttta 60540
ttacgagatg gggtctttgc tttgttactc aggctggagt gtagtaatgc catcagagct 60600
cagtgcaacc tcgaactcct gggcttaagt gatcctccca cttcagcctc ttgagtagct 60660
agtactacag ccatgagcca cgtgccacca cacccggcta attaaaaaaa tttttttttt 60720
tgtagaggtg ggtctcactt tgttgcccaa gctggtttcg aactgctggg ctcaagtgat 60780
cctcctgcca cagctatcca aagtgctggg attacaggtg tgagccactg tgcccgcctg 60840
agcgtgcgtt tatagtctca taaatgagca acattcctcc atatatagct cctgctgatg 60900
aaaagtttgg tgtgctgata tttttctttt cttttctttc tttctttttt ttttttgaga 60960
tggagttgcc aggctggagt gcagtggcat gatctcggct cactgcagcc tctgcctccc 61020
gggttcgagc aattctcctg cctcagcctc ctaagtagct gggattacag gcatgcgcca 61080

ccacacccag ctaatttttt gtatttttag tagagactaa ttttttgtat ttttagtaga 61140
gggtttcacc atgttggcca ggatggtcta gatcttctga ccttgtgatc tgcccgtctc 61200
ggccccccaa agtgctggga ttacaggcgt gagccactgc tcctggcctc tttttttttt 61260
tttttttttt taagataatg tctcactctg ttgcccaggc tggagtgcag tggcgtgatc 61320
ttggctcact gcaaactccc cctcccaggc tcaagtaatc ctctcacctt ggcctcccta 61380
agtgctggga ttacaggcat gagccactgt gcctagccta gtgtggtgat atttcaagaa 61440
cactttacaa agctttattc tacttgctac agcttcagca tcaagatcct gactctgtag 61500
ttttcttgtg atataaacca actacattta gggttcattt tcttatcttt tactcacccc 61560
agtcttaatt ttgttgcaca gtggaaagca catgaacttc atgaggttca gaaactgggt 61620
ataacacagc tgcttctctt tgaatcctaa tgaccttgaa taagttatcc aaactctaat 61680
cctcaaaatg gaggataata atacctcaga aggctatatt taaagatgct gtgaagtacc 61740
acatgtaaat tatctggtac agaatttacc tttgttgatt ctcagtaaat gtttgctgcc 61800
tctcccctga aacaaaatga aatggtgtgt gtgaatagct ttgtgagctt taaataatat 61860
aattaaatag aagccattat tctgtttcct ctttcagaaa tgggcattaa taacataatt 61920
tataatctca atagtatcat gtgcttggcc caggtacatg cccaggtttg gtattcttta 61980
gatttttgtt tcttcttgcc ctacatactt cgaaagccct tggagaaatc ctacagcaaa 62040
ttcacatgat ttatgaaacc aacagttgac taattcttac tctgtttctt tgactaattc 62100
ttagtctatg tatccacata tatgaaacta aatccacctg ttaccttctt tttatggtat 62160
ctgggttctc ctactttgcc ctactccttt ccccactaag aaccttgaac ctcatggaat 62220
ttattagcta gcatataccc tggtctcttc ctgttttggt atttctggtt ggattgactg 62280
tgataatcct tattggatat tcaactaatt gtctaaaata agcttacata tcatgagtta 62340
agaggtatag tgccaaggac ttgaaaagct tgaactccta ggacttactt gctgcagtcc 62400
agtttagaga gaaattaatg atttctgaaa atgttcttag cactcaagag cattgagata 62460
ttatcaaata ttcattaaac atttgtgtgc gtagcacttt ctgggaaccc agatggatgt 62520
aaaacgtgta tgctttggtc ctgagtttct tccttccagg gtagtctagt tagagttcag 62580
aataactcaa aaaagttgaa atgtaagaca ttaagccttt tgaatacaaa atattatgga 62640
taatatgtgc ttataaattt gaagactttg tcctgtttat tttttataaa gtatggtgaa 62700
ttttgttttc ctttacctgt gaactagaaa taaaaatacc ctgtggggtc attgtgatgt 62760
ttaaatgaga ttggatatgt aaagcccagt tataatatat aaacttacat atattctttc 62820
taatacatac aattagtatt tagaattcta atttctaatt gtatactatt ttttaaatta 62880
aacaagtaat atgatacatg gttaaaaaaa atgcaggcag aacaaagtaa gtattcttct 62940

cacttcagac ccgaggttct tccatatgtt cttctgtacc aagagtatgg ttttaaatat 63000
ttttcattaa ttattctaga acatatgcac ttacatatac ttacatattt ttataacata 63060
taagtgggaa catattattc atgttgtctt gtatcctgtg tttttcattt aaaaagagat 63120
ttttggctat attccctgtc aacaaatata gcttttaagt acattattct tttattattg 63180
attgctgagt attctgtcgt ttggatatgt gataatataa ctgatatttt ccccagcact 63240
cctttaatgc attttttcaa aactttcatc ttcttaggat caactcacta tcctgaaggt 63300
ccattctccc aagaagaggg gacagaaaga cagatctatt tgtaagaaag gcttgggtat 63360
cccatgaacg agccaacaga aaaccgattg gggtgcagca ggactccaga gccagatata 63420
aggctcagaa aagggcacca actggatggt acacgaagag gtgataatga cagccaccaa 63480
ggagatttgg agcccatttt agaggcatct gttctatctt cccatcataa aaaagtaagt 63540
caaattggaa gaatgataaa caaaggaaaa aatgtggagg aagtggcaga gggaggatat 63600
ggggaaaaaa aagtgggggt ccattgctgg agggaggatg gggtggtaag aaggaaactt 63660
gcacatttgt atttttttct tctggataac ttgaagtctc ttatctttat tattattatt 63720
actactacta ctactactac tactactact actactacta ctactactat tgagacggag 63780
tctcgctctg tcacccaggc ggagtgcagt agcatgatct cggctcgctg caacctccgc 63840
ctcctgggtt caagcgattc tcctgcctcg actacaggta catgccacca tgccaggcta 63900
atttttgtag ctttagcaga gatgaggttt tgccatgttg gccaggctgg tctcgaactc 63960
cttacctcag gtatcctccc acctcggcct cccaaagtgc tgggattaca ggcatgagtc 64020
atcgtgccca gcttcttatt cttaaattag cttagatatt atttgatttt gattttatgg 64080
ttaatgtctt tctgctgact cttttttttt taatttcgga gacggagtct tgttctgtcg 64140
ccaggctgga gtgcagtggt gcgatctcag ctcactgcaa cctctacctt ccaggttcaa 64200
gtgattatcc tgcctcagcc tcccgagtag ctgggattac aggcacctgc caccacgcct 64260
ggctactttt tgtattttta gtagagatgg agtttcacca tgttggccag gatggtctcc 64320
atctcaacct ccttatccac ctgcctcaac ctcccagagt gctgggatta tagacgtgaa 64380
cgactgcgcc cggcctctgc tgactcttaa aagcgtactt aacctgcttt atcttgttga 64440
agggagaaga cgtggtctat gggtaccata taatctaatt taaccatact ttagctatga 64500
cgttatctgt tatttttgga gaaattcgaa ttgcgtgtta gagctacttt tccagaaatt 64560
gtttacctcc agtgtatacc ttgggagtca gtagagttct gaaagctgaa gcagatttta 64620
ttctttcatt tcctagagaa aagagcaacc tttaggcaac tatacctata gagcagaagt 64680
atcagaaagt cattgttaca gacattttgc tatgtgcttt ggaacttcca aattatatag 64740

catgtgttta atgttttatt actagtacag tgtttctcaa actttgggtt gtacctatta 64800
gtgggttttg aaattaattg tattataaag tgttaaaaat taaagatcaa aatagaataa 64860
aaacattgaa gtgtgtatat agtaagtaat acacatttaa attgttttag ttttatatag 64920
ttatgtatat aaatatgaat aataatatga aatacatttc ttactacggg ttgtggttaa 64980
cattttgaaa gcttctactg ggaacagcag ctgggggaag agtgtttcaa ggtagaggaa 65040
acaagctagt gccaaggtcc aaaggtggga atatgcttat cttccttgcc ttcccctcct 65100
gcctcccacc accacctgcg cctgcttgta tcatctttct cttctttcat cttctccttt 65160
attttattat tataattata tttttttgga gatagagtct cgctctgttg cccaggctgg 65220
agtgcagtgg caccatctcg gctcactgca acctccgcct cccaggttca agtgattctc 65280
ctgtctcagc cttccgagta gctgggacta caggcctgcg caaccacatc cggctaattt 65340
ttgtattttt agtagagacg gggtttcacc atgttggcca ggctggtctt aaactcctga 65400
cttcaagtga tccgccacct tggccccgca aattgctgag attacaagcg tgagccactg 65460
tgcctggcct tctttttttt tttttttttt taaaggacca aaagaaggcc agtgtagtta 65520
gagcatgagg aactagaggg aagagtgaaa tgagataagg tcagtgggcc agtcagagaa 65580
cagattatac aggtttattc agggaattta aattttattt taataaaatt gcctggcacg 65640
tgggcagctt ttggggtgtt taaacatgga gacatttgtg tttttaaaag tatgactctg 65700
gttgtggaat ggattatttg aagacagaat ggaagaggga cccaggtgtt agaatataga 65760
tgagagatga tgtcacttga aacagcatgg tgggagcagc agaaatggag ggaaatggtt 65820
ggattctgga tatattttgt gggtcaaatc aacaagactt cctgatggat tagctgtaga 65880
gtgaggaaaa tagaggaatc aaggctgatt ctctgagctg aactgaaatg gtaaagcaag 65940
agggaagagc gggtttaagg tagggaaatc aagagtggtg ttttggaaat gtgagattca 66000
agattcttgt tagatatctt agaagatata ttgaggagtc agtaactgtg tcacgattag 66060
atttttattt acctctgttt cttaactatt tttaaaactg aactgttatt aaggaatcta 66120
aggaattttc aagcttgaaa gaatttcagg ctagccaatt tgagattcat aatggcaata 66180
aaattctgtt tatcatgcta cattattttt aattacgtac aaagatctaa catgtcaccc 66240
agggaccatt tcacccactg ctctgtttgg tcgccagtct tttgtctttc ttttcagcaa 66300
tggtgaggca gatacccttt tcctcaggga agagaagtct gtggtttgtt gcccttgcca 66360
ataacaaaaa tgttggaaag ttgagtggca aagctgtttc cattggcatc tttcatgtga 66420
accacatcaa aagatccaga gtgcctctct ctgttggtta tcacaccagt tcttcccagg 66480
ttagcaccta cagtcaccat gcataggtta ccattgtcaa acttgatgaa attggtaatc 66540
ttgccaatct ccaaatctgt ctgaatggta tcattcacct tgatgaggga atcagggtag 66600

cggatggtgc cggccatcat gagtcaccag atgagggatt ccttttgtgc ccccagagat 66660
ttttctcact ttgtatttgg cctcctcagg tgtaatagga tgtacagcaa agcgaccctt 66720
ggtgtcatag atgagacgga aattctcccc cgtcttgtca atgctgatga catccatgaa 66780
tccagcaggg taggttatat gagttcggac cttgccatcg atcttaatga acctctgcat 66840
gtaatcttct ttacttcatc tcctgtcagg gcttacttaa gtctgttcct taggaatatg 66900
atggggagac actctctcaa cttgtgggga ctagtggatg gacgaggagc aaacacaccg 66960
gtcaatttat ccagcatcca atgctttgga gctgctgccc acttcagatg cttcttggga 67020
ccacgcgcca tggctgcgtt aggcagggaa agcaaaatat atttttagag tccagaactt 67080
gcccacccaa tcacttttgt gtcagattta cattaattga tttttttatg tgttgtaata 67140
ctatatattt ttgtttcctg tcttcactgg ttgaagtggt atcttctgta aataatattt 67200
gtgttttaat ttcactaata aataattagg tggatgggat ttttcccatc cttatgaatg 67260
aactcattta ttgccctgaa agcctctctt tgattttcct tttatcttga gctaatgcaa 67320
aagttagcag tatgaacaaa agaaccattt tttctttcat ttgttttaat agatttttct 67380
atgaggagcc aaggaaagaa aggatccata ggttgtggaa tgttagagct gaaaaggcct 67440
tccaaaatca tcttgtttgc cttcctcaat ttaccagtga ggaaattgtg tttttattcc 67500
taatgaaaat ttgcgtgtgt atgttgtttc tctttggagt tgaagttgtt tgctgaaaaa 67560
gagaatctat atgcagtact gtcccttgct gccaatttca cggttaatgt atactacaca 67620
tgggagctag aaaagaaggg ggatagataa gagttataga gaactgcagt tttgagactg 67680
aaattaaagg caaaatattg atatcttcac atgatttgag gagttccata tgctgatttt 67740
ttttgttgtt tggagaaagg actcatttct taaaattttc agttttgaag ttgaaacatc 67800
tgaactgtag actgcaacct tctggataat tctagtaata tcttaattgt gtcactttca 67860
cagacattat ctcatttgct tctcaaaact atactctgaa atagagcaga tatgatttct 67920
ttttgaaagt agacgaaaat aggatttaga aaggttagtt taggaatttg cccaaggtaa 67980
catgacaaga tgagagcatg tgctaagtct tctgactgct ctttctgccg aacaaagctg 68040
cctttatgag aattggcacc agattgactt aatgtttctg aattccaaac tcagtccctg 68100
agttgtttat attgacctag aggttagatt caacagttgg tcttgttttt tggcagaagt 68160
tgttgcagac ctcgttttct gttaggccag ggtaattttg cttctcagat gtgttctaac 68220
tttgtaatgg gttttctgtt tattttgaaa gcaaatagaa cgaatatatc atagtacagt 68280
cattcacatt tctaacaatt ttgcatcatt ttctttgttt ttttccctct tttagaagat 68340
gcagatctta actaaattgc ttaggcttca tttaaaacat tatgggagcc cttaggctct 68400

cttaagttgt ggagattggc attcacatgc caatcatgaa taaggatcaa agtaaaatag 68460

gaaaagcagc aggggtgtct cgttttttat taagtggaat tctgaattta taaagtaaga 68520

tttaaggtaa ttatgtgcat tacagactgt ggtatctttt tgtacaagtc tttatttacc 68580

gccaggtttc tttaaatcaa taaacctatt aggagtgccc acctgatcac atattgccct 68640

ttatagaaaa ctacaaatgc caggatttta ttccaggcct gctgaatcag aatctttggg 68700

tcctgggtat attttttttg tttcacaagt aagatgctca ctactgttta aaaacctcct 68760

tcctggccag gcgcagtggc tcacacctgt aatgtcagca ctttgggagg ccgaggcaga 68820

cagatcatga ggtcaggagt ttgagaccag cgtgtccatc atgctgaaac cccgtctcta 68880

cgaaaaatac aaaaattagc tgggtgtggt ggcaggcacc tataatcccg gctactttgg 68940

gaggctgagg taggagaatc gcttgaaccc aggaggtgga ggttgcagtg aaccgagatt 69000

gcaccgctgc actccagcct gggtgacaga gtaagactct gtctcgggga aaacaaaaca 69060

aacaaaacaa aacaaaaaaa acaacctcct tcccgtagga gatagttaag gaaaacaaat 69120

aaacaaaacc caaaccagtg cctaattttt tttgatattt atttatttta aataagtatt 69180

ttatttaaaa tagttaatgt attaaaaact tcttagcaac atgctttgca aagaaatctt 69240

aacacattct ctgaagattg ttagggaaat aaagttagat ttatttactg tttgttaaag 69300

gaaaatatca catttactca cagttgactc ttaaaacatt tttttttgag ataaatttta 69360

cataccataa aattaaccca tttaaagtgt acaatttaat ggttttatta gtcacagggt 69420

tgtacaacca tcacttcatt ctaactttag gacattttta tcatccccta aagaaacctt 69480

aacccatttg ccatcacttc ccatctctac ccccaaccct aggcaacctc tagtctactt 69540

tctgtctcta tggctttgtc taatctggac ttttaaataa atggaagtgt aaggcatgtg 69600

gtgttttgta tctagcttct tttacttagc ataatgtttt taagtttcat caatattgta 69660

gtatgtacca gtacttcatt cctctttatg actgaataat attccattgt atggatatac 69720

aggggttctt tcttatttgt ggtttcacat tctgtggttt cagttaccca tgggtcaact 69780

gagatctgaa aatattaaat ggaaaattcc agaaataatt tctaagtttt aaactttgtg 69840

ctgatctgag tggtgtgatg aaatctctca ccttccttgc cctgtcctgc cttacccagg 69900

aaatgaatca tccctttgtc cagcatatcc atgctgcaga tggctggcta cttagttact 69960

tagtagcctt ctggttataa ggtcacagat cacaagaagc gtgagtgcaa tacactaaga 70020

tattttgagt gagagagaga gcatattcac ataacttttt tttacagtat attgttataa 70080

ttgttctatt ttattattgg ttgttaatct cttactgtac ctaatttata aattaaacta 70140

tgttataggt atgtatatat aggaaaaaac acagcatata tagggtccag cagtatccat 70200

gatttcaggc atccactagg ggtcttagaa catatttcct gtggatgggg ggaaactact 70260

gtactatagt ttatccaata atgagttgat ggacactgga ttgtttccac ttttcgactg 70320
ttgcaaataa tgttgctatt atcattcatg tagaagcttt tgtatggatg tgttttcatt 70380
tctgttgggt atatactcag gagggatttt tctgggttat atgttaactc tgtgtttaac 70440
attttgagaa actaccagac tgtttttcat agcagctgca ccagtttata ttcccaccaa 70500
caatgtgtgg gggttccact ttctccacac ccacactaac tattgttact atattttgaa 70560
tataactatc ctagtgggtg tgaaacgata tttcatcata gtcagttagc tcttgagctt 70620
ttatgaagag acctgcctag gttaacttta gctcaggatt ttaagtatta acctctttga 70680
ggaatttgga aatagaagct aaattcaaac ttactataaa actcaagttg tgtcaatttc 70740
acttcaaatg gtctcctcat catctgttct gccattactt taatctaagc caccaccatc 70800
ttccatctgg aatacacctg acaggacttc tgttttgttt cttgtgctgt ccaatatatt 70860
cttcgcacag ttcctagaga gagctcttaa aaatataaag tttattatgc cattcccatt 70920
taagaaattc atttgtttcc cattgcactt atggtacaat cccaaattct ttctataacc 70980
ttcgggatcc tgcgtgatct cactgctgcc ctatctggcc aacttcattt caggacattc 71040
ttctccatgt tcattgagct gcagccgcat tggccttatt tcagtttctt aaacaggctg 71100
ggctctttcc agcttcagag ccttcaaaca tggctctttc ctctgcctgg agtgttgtta 71160
cacccacacc tctggctaac tgctgctcat ccttcagtat tcaggttaag ttctctactt 71220
ccacttcatt ctatttcaag tttcccattg ttatctttaa ttacctttta ttttccatta 71280
caatgcgtat catgacatat atttgtgtga catatattta tgtttataaa catatatata 71340
aaacatattt tataaaaatt tataattgtt taataattgt ttttgcttgt agactcttca 71400
gttccatggt gtaagaatct tgcctttttg ttttcctctg atatcctcag agtctaacct 71460
ggtctttgag tggcatataa caaatagttg ttgaatgaat ttatcagcaa caaatataaa 71520
gttctgtgtc taagtggttt caagtagttt ctttaaagag atgtacaaaa ggagcacgtg 71580
gggacgtata ctatttagat gcttagggaa gcttaactaa tctacttctt taatcttaga 71640
ataaatgcat tcttatcagg agaataatga tagttcactt ttattgatct tttacaatgg 71700
atgaagtaat tttttgagct catttgaata tgttattcgt tcctaaagta gccttttaga 71760
atagatatta ttagaatctg ttttacagat gaggaaactg aggtttcatg tgaatgtatg 71820
tgtattgtac actgggactc ttgtcatggg gatgatatac tttttctctt caaaggggca 71880
gggttttctt ttcattggtt tattgtaaaa agtgcgatat ggcaatttca atggttattt 71940
gcctttaata cattttaaaa tacattattt ccatagatga taagaattgt ggaaaaagag 72000
accctgtgat aggtgatagt gatccattgt agttgtggaa ttggctctca agtaacatta 72060

aataaaatac ctgtgtgtac agttagatta cgtttacctg tgcataggaa tcccttcctt 72120

atttgaggtg ttggagaggt tagtctcttt ggttccttat gaagcaggat ggatttttag 72180

aaagagtatt agaaactaaa tactttaaaa ttgaaatctc tgcagtagga atctgtattt 72240

ttcacaagtt cccctatgtg attgtgaggc acacttaatg atggagagcc agtgatcaca 72300

gttttctttt cataatgcat atacggaagt tccagaccgg taggaattac atcagggtaa 72360

agctgggcct agaggaggtg ggtgtttctt cttttgtaaa tgcagtatgt aatagtatgc 72420

ctcttgtctc acagggttgt aatgataata acttagtgca tgtaaagcag tggtaacact 72480

gcctggtaca gagaaagcac ttaataaatg ttagatgtag catatcattg gaggtgtata 72540

tttgtgtcaa aaagaaaaga cttcctctgt tatatcaaaa ggacaaagtt aatctttctt 72600

tatttctttt tgtttttatt agatttgtat ttatcttata aaaaatagat tggtggttat 72660

ctgtctccac tcacttgaac atgtttattc tctgcatcca aaatagtcca ggaatggctg 72720

gacctggtgg ctcatgcctg taatcctaac aactttgaga ggctgaggcg ggcagatcgc 72780

ttgagcccgg gagtttgaga ccagcctgga caacatagcg aaaccttatc tctgcaaaga 72840

atacaaaaat tatccgggcc tggtggcatg tacctgtagt cccagctact tgggaggctg 72900

agatgggaga atcacttgag cccaggaatt cgaggctgca gtgagccatg attgtgccac 72960

tgcactccag ccttggcaac agagtaagac cctggctcaa aacaaaaaat tacacatata 73020

atgtatattc caagaatatt gtatttgtgc aatatttaag taaagaaatg aatgattaaa 73080

cctgagtttt agtcctggct gtactgccag ccagatgtgt gactttggcc aattttcttg 73140

atttctttgg gtttcatttt ccttacatta aaatggagat gtttaactgg gtgataaaca 73200

tctaagattt tcttatcctc aaaatttctg tggtccccga cactatatta atactttttt 73260

gtaatgggat aaaaaatgag tgagggtcat agtttacttt tttcataaat caatccagtt 73320

aggtatcttt gcttaagcag ccctgtttaa tgccttcttt gatcactttt atcgtgcctg 73380

gttttattta gtgaagccat ttctgtggac tagtaaataa ttttgaagga aatagtttgg 73440

taaaaggaaa aaagcagggt tttgtactca gagtgaccta aatttgtatc cctgatctct 73500

tctgtatcat tatttttcaa acttctgtgt aagaggaccc ttcaaagaaa aacaacaaaa 73560

accttattaa agtattaaac acacacacac acacacacac acacacacac gtagatatat 73620

tgtaagtgga tagtctgata aacttttata aactagacac tcctgtataa ccagcactcc 73680

tggaggtctc tttattacct cctcatagta actgcctatt cccactcccc caaaggtaac 73740

cattaccctg acttctaatg gcatggatta attttgcttg tttttatact ttatctcatt 73800

gtaatcattc catatgtact cttgtatctg gcttcttttg ctcagtatga tgtttgtgaa 73860

attcatctgt attttctggg tatagctgta gattattcat ttttattggt ctgtgttatt 73920

tctttatatt actgtaccaa tttttatttt atttatatga aggcaatctg ggttgtatct 73980
aacttggggc tattataaat agtgttgcta tgaacattcc agtacttgtt ttttagtgaa 74040
caaatgtatg cacttccatt agctacatac ctgtgagtgg aat[?]actgcg ttaggaagat 74100
gatcccattc tccctttaaa catcaaaaat atttaacaga cctcactaca tgatactcac 74160
attttaaata ttagtttgaa aaaaatacat gatagaaatt ggctatgaat tcagttcatc 74220
ttaagtgaat ttgtattttg tgattcacgc agcacctaca tgattttaaa ttagttacac 74280
ctactttatg aatatgagac atttattcag tctacagatt acatttaatg tgcatataca 74340
cttgcataat ttttgaagta attagccaag atagtggtgt taggttggga attaaggtac 74400
tacttaaagc tgtgtacttt ctcttagctt ctgtttcttc atttaccatg tttgatctag 74460
atttccactt aataaaatgg gaatagaaat atactcattt cagaaaattg ttatgagggt 74520
ttgattaatt tgtaaaaagc acttagtaca atgtctggca tgtagtgtat aataaataat 74580
aattgttact c[?]gtccttcc tttataatag tacttgacta ctccataact gtagtaagtc 74640
ctgataaaca catcttgaag ttatccacat atctcagcca cccttgattt caacagcctt 74700
ttcataaagg tgatttctaa atctatcatc ttagtgcagt cttctttttt ttcccccttt 74760
tttcttctat agccttcaaa tgagaagtgc tttcttcttt attggacttg taaccatttc 74820
aaattgaact aatgttaaac taaggttaac tgtccactcc cccatcagct tttccttcta 74880
ttctaatttt c[?]aatcctaa tttctcttaa atagtaccaa ctttctttca ggtactcaga 74940
cttggttcct agactgtcaa gagtggaagg gatcccatca gtcatttagt ttagtagaca 75000
catggatgct tttaatcctt ctacaaatgt ttttagtttc tttaccatgt gttggcactt 75060
tggacttgag aagacatagt ttttgccctt aagcagcatg tagtttagtt ggagagacag 75120
gtaaatcagt acatttgtgg tgagtgctgt gtggttttat gcacagatta ctatggtagt 75180
aactccagtt gcaaatggat ttagctaaga aaggactaaa ggacatttta gacaaaggga 75240
acagcagatg tgaagattca gaagcagatg taatacgatg cctgtaggga atagtaataa 75300
taatagctaa tgtttattga gtacacactg gcacatactg atgtctgaac ctgaaaaatt 75360
tctagcatag ctggaatgaa aggtcaaggt gtatagtggg gtggaggatg tggtagtaag 75420
taagacaaga cacctggggc ctgaaatcta ggtctttagc ctcctggtcc agtattatgc 75480
catcattcaa atttcttttt gcaatttata tcagtttcaa attttgtgta atgttgtgta 75540
ctggttgaaa tgattagacc attgccccta ggcctctgac cctaaagcct ggactctttt 75600
aactatattt gctaaagtat atagttagga ggcattctgt aagcttttgg acaaaaatat 75660
aagagaggca tttcaaacca ggttgtcctg atctttattt ttatggctcc cctctgcctc 75720

cctgctccct ttctgagctt tcaagatatt aggttgaata aagagatcag cctttatggt 75780
tgtcctactt ttcttctgct ccagattctt ctttataaat tgggagataa ggaactttta 75840
ttcattcaac cttcatattc tgttaaactg gtttacttta accaaaatca agaatgccac 75900
tatataatag tttattgtaa gaaaactaac ttgtaaatag actcttgtca atcacttcat 75960
tttattttca tggagaagcc tgcttaagaa attcaattaa gattagaggc cgggtgtggt 76020
ggctcacgcc cgtaatccca gcacttcggg aagctgaggc gggtggatca cctgaggtca 76080
ggagttcgag accagcctgg ccaacatggt gaaaccccgt ctctactaaa aatataaata 76140
attagccggg cgtggtggca ggcgcctata atcccagcta ctcgggaggc tgaggcagga 76200
gaatcgcttg aacccaggag gcagaggttg cagtgagcca agattgcgcc atcacactcc 76260
agcctggagg acaagagcaa gacttcgtca aaaaaaaaaa aaaaaaaaaa aaaagataga 76320
attgctattt gagaattttt aaaaaactga gtgattttaa agatcttttc cattaagctt 76380
tgatggaagt cagagtatat atatttctag gcaggccaaa atgaatattc atatagacta 76440
gatattatag ttattctttt ggagaatact gggtatacat ccatcaatac ttattcttca 76500
gctatcattt ttttttgtag atacctttgt ttccacaaac ctctttgttt tcatttgtta 76560
gtaatatcag cttgctaatg cccttgctac acatttccaa tttctaaagt taaggctggc 76620
ttcaacctgt caacttgtaa aaagaccata aaaatcacct gttgatcaaa ctaagccagg 76680
tttattacat actccagtaa gggtgaacac tgccctaaag agtcctagaa gtatttcaga 76740
aagaaagtat tggatatgga tatttacagg gttttggggt ctggtttcaa gtagtttaag 76800
gagattttac ttggccctgt tctgagtttg tgatagaatt cttagaccaa gtcttgataa 76860
acaaatattt gtttggtaag tgagcatgtt tgcccagttg agcaatgatt aaggatctaa 76920
ttgagcagtc tgttgttcag ttaaatgaat ttttgagaaa ttcctaaagc taatgatgaa 76980
gttatttact ggtttgcatt cttaccttcc tgggcaagaa tttccgagaa caaataataa 77040
agttctgttg atataggggc cttagatctt agtccttata attaagctgt gtgaatgcag 77100
aaggtctcag ttctcaatga catttataaa ggcagacaga ttttaatcaa aggtttactg 77160
tctccagccc tggatattgt accatttctt gacctttttc atctaattgt ggatggtaga 77220
tagtatcatg attgtgagtc tttgtggcac ttcttgtact tccactaaca tttaaatacc 77280
tcatttatgt tttgctactt tcctaaatgc ccccgaagct ctagatgaac tagtctcttt 77340
ttctcaacat ggcacttttg cttttccctc ttggttattt tcctttatct catctttgca 77400
aaatttttat tcttcatgca ggctttcctg agcctcatca tgtttatctt tgaactagct 77460
ataccttttt ttctatgaag tcatttattt ggctaattta gcactttatt agattgtaag 77520
tccatggaag acagagacta tgttttattc atcttatttt taatcatcta ataaatattt 77580

gttaaactga attactctgt tgaattgagt taggccaaaa ccaataaata cccaataggt 77640
ttgtgaatta ttttcatatt agactgatct taaagacctt ttatctttat cactatggtt 77700
ctctgtcttt ccaaaggtaa agtgtactct ggaaaattct cattttagat tgcagtttgg 77760
acagatcatc aaagtcaaat gttactatgt tttgaaatac agtcagcaaa gttctcacag 77820
atggacttta ctgaaaacag atggcgggag gggggagata tttcaaaatg tctttgtgct 77880
catttcaaag cattgacctc tacaagttta aatatagtta ttcatagaaa aacctggaga 77940
attaagtttt atgcctttat tcttggcagc tttggttttg tttaatctgt cacaatgtca 78000
tgtttcttgt cataattttg ttgagggtgt ggtaaggagt atgacaacag aagcagaaca 78060
tagcatgtat gtgatcattg aatatagata caatttattg ttcagagagg tatctttggg 78120
tactgtggtt tggtggctgt tggatggaaa ttagaaatag taatttcact cataataaaa 78180
tagttatttg gacttaatat tggaagacag agactatttt aaatatagcg tctagcatag 78240
tctttccaag ccatcagtat gtgacatatg gtatacccta gggaaagtat ttttttatcc 78300
tgttttagcc agtgtttaac tctgccactg tattcctact ttatcctctc tctaacccag 78360
ggaactgcat ttaccaccac ccacaccacg tggataccgc attagcaact ccgttaatac 78420
ttgaacacaa taaatagtta attatggtct agtattttaa tttatccttt gagaacatat 78480
ttgtgaatat tcctttatac aagggtagga attggtcata gtacttgata aatcaacaga 78540
tttattaata ctttgccctt ctgaaggaaa tatttttgtt tttgtttgtt tctcttgttg 78600
cccaggctgg agtgcaatgg cacgatctcg gctcactgca acccccacct cctgggttca 78660
agcgattctc ctgtctcaag cctgctgagt atctgggatt acaggcgctc gccacctcac 78720
ctggctgatt cttgtatttg tagtagagac agggtttcac cacgttggcc aggctggtct 78780
cgacctcctg acctcaggtg gtgcacccac gttggcctcc caaagtgctg ggattacaga 78840
tgtgcgacac tgtgcctggc cggaaataat tcttttataa agattctgaa tcttgctgat 78900
atattgggat ggatggaaat atttatcttt actaaagtaa tttttttttt tttttgagac 78960
agggtgtagc tctcttgccc aggctggaat gcaatggcaa gatcacagct cattgcaacc 79020
tcgacctcct ggggctcaag gagtcctccc cctcagcctc ttgagtagct gggactgcag 79080
acaggcacgc aacaccactc ctggctaatt tttatatttt ttgtagagat ggggttttac 79140
catgttgccc aggctggtct cgagctcctg ggctcaagcc atcctcctgt cttggccgct 79200
atcagtagtc ttatatgtaa ccagttgcac ttagcctgat aatcagaatt actgattgtg 79260
agtaatctca ctgattactt actgagtcac attgattcgg agtgcagttt catcatgttt 79320
acttaatggt agtatacatt agccatattg tgttttatgt acatatctgt agttttgaaa 79380

tggctgacag taatttgtat gagaataaag actctttgtt tctgtttctt tggagaatca 79440

aatacatgtt gaatatgtgc aaatatctga gctattttat ataacataat tttatatttt 79500

aaaatcggtc tgctataatc atttcaagct attgcccgtt ttcaggtttt atataatttt 79560

aatggaattt tgtcatttta atttcgtgtt taattaggca cagccctcat taacttattg 79620

ttgaaagtct cctagagcat ttattaattt agccatcatg taagtatgtt ccagaagcca 79680

tggggaagaa gacagacact taaaaatcta gtttaggaaa tgatacgcta aaaatgtaaa 79740

attctattaa acgttaaata aaacagtatt agtcagtaaa agagctgtaa tcagatatta 79800

ctgtcaaatg agaggcacag acttaagtgc taaagaagtc agaagaagta gtcagaagaa 79860

acagaagtca gaagaagcaa ttgctgtgga ttagataggt tatgcagtct ctaatgaaaa 79920

ttggttcttg gtggggtagg ggatgaaaaa ctattaaata ctactatggt ttggaacccc 79980

ccatagggcc acagtacata gatagtatat ctgtgttatt aaaattcatg gggatgagga 80040

aagcaatcta aaatgtccca gggccgggtg cagtggctca tacctgtaat cccaacacac 80100

tggaagctga ggcaggagga ttgcttgagg ccaggagttc aggaccagcc tgagcaacac 80160

agacctcgtc tctacacaca cgcatgtgca cacaacagac acagtggtgc atacctgtag 80220

tgcacgcatg cacacacacg tgcgcacagt ggtgtacacc tgtagcccca gctattcagg 80280

aggcttaggt ggaggattgc ttgaggccag gaggttgagg ctgcagtgag ccatgatcgc 80340

accactgtac tccaggagcc tgggtgacag agcaagaccc tggggtctca aagaaaaacg 80400

ctgtctcaaa ctaaaaaaaa aaaaaggtcc cggggcttgt gggtagcagt aatgaaaggt 80460

tgaggaacac gagtttagga tagttaaaga agggttttaa ataggaacta tgaaggttag 80520

tcattgaagg atagatggta agttttaggt aaacaaagat ggagaggtta ttttattcaa 80580

gatggttttc ttggagatta tgtgttttta taaaatcaga ctgggtgtat aacatgtctc 80640

acatgccgca tcatagcctc tagttagtgt cttataaact gaagaatagg aaaattaaaa 80700

tctcagaatt tccttttaaa caagatcaaa tttaaagtgt tgtagtagat tacccattgc 80760

ctctgtgttc attcttacca agtagttttt tgattagagg tagaggattt attcatttct 80820

ttgtgcagtc atgttagcag tactgtttat ctaaacagac tcttggttac atacggaatt 80880

atggttctct tgtgctgtct tattcattga gttaaagaat tatagaattt ttttagagat 80940

gtagaagact gtagagattg tttaaactcc tccattgata catagggaga aaatggacct 81000

gcagtgaaga tccattgatg ttagtggtgg agctggcctc accactttgg tctcctgact 81060

tgcagtctac tgcccttctt ctcacaatat cattttatct aaaccagtat gaaccttggt 81120

ctggaggcag aaattttaat tttagtaatt tatctcttta gaccatacca gattgtcact 81180

tctttttcca tttctctaga caaatcagtc attttctcca gaatgtggaa aggcagtggg 81240

ttttggagtt tgacttttcc acttcagctt taaccttgga caacttactt aacttctttg 81300
cctaatgctc ttatatgtta aatgggaatg agaatactta cttaataagg tggttgttag 81360
gattaagtta gatgacctat atataaagcc ttttcctctg tgttggatat atttaaaaaa 81420
tttattaaat gtttttctcc cctttctttc tctgccacta gagtacttac cacattgtgt 81480
atcacagtta gttattttat gtgtcccagg tgcgagtctt ggaggttttc cccttagtaa 81540
tttctcaact ttatgtaata ttattatggg gtctatgaca taatttttaa aaaacaaacc 81600
agtaaagcca ggaatttgct actgaaacag agatgctgct atagtagaca gttaattcat 81660
ctgtcagaga agaattgagt actagtgttc atttttaata ctaataattc tataataaac 81720
tgacttacta tattgttgat tcttgtctaa ggccttgttt tgaccttaag aatttcatta 81780
taattccaca agggaaatgt attgattgtg tcatttggct cccagtctct gtccttgact 81840
gtttctgtga ttgccttgca gagctctgag gaacatgaat acagtgatga agctcctcag 81900
gaagatgagg gctttatggg catgtcccct ctcttacaag cccatcatgc tatggaaaaa 81960
atggaagaat ttgtttgtaa ggtaagagtg cagtttcttg tcattgtcat gctatatatt 82020
tagcccttcc aaaacagaaa cctttaaaga gagtggtagt actatagcct ttggtttgct 82080
ttggggaaga tctgtggcaa gatggggaca gaagtggtct tgggactcta atcctgttga 82140
agacagaata catttgttgg cttgtagtag ccatgataaa gctctgataa aactaggata 82200
tccagctggg aaacaaacat ttcctaatgt aggttaacag agtaaatgtc ttattggtgg 82260
tttagacaaa cagaatgaaa catagtcaga agtggttgac gcctggagat atgaaaatgc 82320
cctccgcagg tcccttaaat ctgttctgct gagtgactgc aatggcaaat gtactgttct 82380
ttctagggat tctgtgagta atcaggctac tcttgagaga gattttccag tttccatgta 82440
catctggtat tttcagttca gctggtatac attctgtctc ttaaggtttt tcttccagtc 82500
cattgtcttg attccctagg acctattatg ggattctttt tttttttttt tttttctaaa 82560
atagtaatta gaattgagtt tgcctgctta gagaaaatag acacactggt aaacttgtag 82620
gtacttgatc aatcagatca ataagggccc tggcaggtag aatctaatta atgtaagtcg 82680
atcagtctgt gtgtatacgt atgtgtatgt aaattgattt attaaacatt tctggagaac 82740
tctttattat ttagagagaa atttttgtga cacttagctt ttcatggctt ttatgaaacc 82800
aaaacttttt atgtacatgg tcttagatcc aggagactaa atataggttt tcttacattc 82860
acaataatgc tggaaataat tgggatcagt ggacttagaa tgctattact ttggaccagg 82920
gtatgaaaag agaacattgt gatctgaaca tagacatcct ttccaagact aagggtccag 82980
tgggttttca gggggagttt aaacatttat gggtctgtaa tagaaaattt tagccagttt 83040

gaaaatgagt gaagaaacaa ctgaacagaa caatagattc attctagtga tcactgtctc 83100

aggagaatta gcacaaaggg atacaaaaga ttattttgtt tttaaaatgc aaatttggtg 83160

tgaagaaatc tctcaaagtt ggcaaattta gtatctgggc cattagggga tttcaatatt 83220

tagagatcag cggggtacgg tagctgactt aactgccttt ttgtcctctc cctctctgaa 83280

cagagaacaa agaataaaac aaataaaact ctaaatttaa cagcaaatca gagaataaat 83340

agggcaggac atttgtttgc tacaaactat gatcttctga aggaaaagga gtgttgtaaa 83400

tatcccttag cagtttgttg atgacagcag aatgaagagg catcctgtta cacagtgttg 83460

tctggtgtgg ctcaaccctg ccatggcagt gcaactgacc acacttgaaa accagccgtg 83520

tctagtggca cagggattct atttcttgga ctggaagcaa gtaaacagag acagtttaat 83580

atgaaatgtg ctgtggataa tttagtcttt agagtttcca cgtttgatct tggtcactct 83640

tttttcccct agacttcagt gtttgctatt taccagcata accaggtcca tgtgggaata 83700

ggatgtatgc tatggggagt ttatttgttt ttgaaattta tttattgttg agtctcgctc 83760

tgtcgctagg ctggagtgca gtggcacgat cttggctcac tgcaacctcc aactcccggg 83820

ttcaagtgat tctcctgcct cagcctccca agtagctggg attacaggca tgtgccacca 83880

tgcccggcta atttttgtat ttttagtaga gatggggttt caccatgttg gccaggatgg 83940

tctccatctc ctgacctcgt gatctgcccg cctcggcctc ccatagtgct gggattacag 84000

gcatgtgcca ccatgcccgg ctaatttttg tatttttagt agagatgggg tttcaccatg 84060

ttggccagga tggtctccat ctcctgacct cgtgatctgc ccgcctcggc ctcccatagt 84120

gctgggatta caggcgtgag ccactgcgtc cagctgtttt tttaagaaat gtttttgtgc 84180

attttatggt ctaatatatc cacataccca gtggattatt actaagatat ttgaaggtag 84240

gcagtgtctt tgtcaagctt ataaataaat atgtctttgg ctaagccgtg atcttatttt 84300

tttattctag gggtaaaaag aggataccta tttgtgttgg aatttggctg actgtatatg 84360

actccagctt gagaatgagg gtgcagtgtc tgttgaatgt gtcactaatt gcagtgtaat 84420

taagaaagta tagctccttg tctcagtatt tagaccacta gaacatttgt ttagaaatac 84480

cagggaaaat cactctgaaa ttatatgcat atataaccaa taagaaactc acatattctt 84540

tttgaatgta aaaaaggaac atatgactct tttgtctgtg tggggtagca gagtagcagt 84600

ttggatacag cagagctgtg tgcacattgt gaattgcctt gaagatgtaa gggttgattt 84660

ctgtcttcct ctccctctca gaaaataaaa ttcccccaaa gtaaggtaat agcaacattc 84720

tgtaaggtta atctcaacaa tagtggtctt ttcccaaatt caaatttgat gcaaaaaatt 84780

cagtatcttg gtctctctct gacactaaaa gaagcagact gcagaggttg acagacttcc 84840

agcagttcca acagaaggga tactggatcc ctgatcccct ttctctttct ctccataaag 84900

tcctgtggaa gatctcatac agctactttt gtaagtggta gtactattag cagcagatgt 84960
taaaaatata gattagtctt ttggcattat tttattttat tgtatgttag aagcaagcat 85020
agaatggcca cattagcacc agtaaccaca gaccccacgt tttgagttgg aaagtgaggg 85080
aggagaggtt ttgtatggct gggggcagga agagtaggga agaggcaggg ggctagtttg 85140
gttagagtgt taagagggac acatctgttc ttaaattgtt agtcctcact tcctttggat 85200
gtggagatgt tacacagagg attagttctt ttacaaatat ctgtaaggaa gtaagagagc 85260
agagaacaag aaacaggttg tggagcaaaa ggaatgggca cagtagctgg tgagtaagtg 85320
gcagtctttc catagagtag agagagagcc aagtttccat ggcttaccca tattgtcaca 85380
agaccctgta gtgggtaggg tggaggtcat tgtctgaagg tgaatcttgc tgatttgcag 85440
cctggattct tgctttatag ggtaatagac tggaagaact gctgagccca cattagctgg 85500
aagataaacc atgtgcctgc catttttcct tacatctttc tgtctagcag aagcaagtta 85560
gtcaagatta agtgatcctt gtggtgtgag ctctcggggc tatgagacat taaaagaagc 85620
agagtgcaga ggttgacaga cttcctacag aggggatatc ggattcctga tcccttttcc 85680
tctagaatct gatgcgggag ggaccatgag gaaacgggag gaaagggctc tgagggactg 85740
aaagggtggt tagggctgag cttctttacc atctctctgc agcatgagaa ggaagatgga 85800
ttagtcacta actgtctgac aggagatagc ccctggggat agtcatgtac tggctttgag 85860
tagatgccag ggattactaa ctaattcatg aaatgctttg tgtcattttg tttgatcttc 85920
atgatttccc tatgaagtaa ctgaagcgct attttacaaa tgaaggagct gcaggttgga 85980
ggttttttaa ttaattaaat ctcatatagc tagttaatgc caggggtcca caattttctg 86040
actcctgtgc tctttctttt ctgcctcatc atatttgcat agtgggaaga gaagaaagag 86100
taagatggag ggagagattt tagcaaaagg cacaaaagta tgattctatg taattctgtt 86160
tttaccaata ggaatattaa atataaaaat gttcatatta acaccacagg gatttatcag 86220
aggtaaagtc tgttatatag gtaggatgaa ggaggtcagt atgagtgaga ctttggaacc 86280
ttcatcccac cattagccag gttgaaacaa agtttgaaac cactctttta gggcatttcc 86340
tataaatttg tttactcttt ccttttccta aggcccccac ttaaaagtat attttttctt 86400
tctttttttt tttttttttt tttgaggcag agttttgctc ttgttgccca ggctggagtg 86460
caatggcgtc atctcagctc accacaacct ccgcctcccg ggttcaagca attctcctgc 86520
ctcagcctcc cgagtaggtg ggattacagg catgtgccac cacacccggc taattttttg 86580
tatttttagt agagatgggg ttcgagctcc tgacctcagg tgatccaccc accttggcct 86640
cccagagtgc tgggattaaa gccttagcca ccatgcccgg caaaaatata tttttcatca 86700

gtctttaaag aactcagaat ataagacatt atacattttg agatgtttgc ctttttaaaa 86760
atgaattgtt tttcccatta taaaacagtg tgataagaca cagttgttag gggtcagtgt 86820
tggtaaattg acaactaaaa tcacaaagat gtttgataat acctctctgc caggtatggg 86880
aaggtcggtg gcgagtgatc cctcatgatg tactaccaga ctggctcaag gataatgact 86940
tcctcttgca tggacaccgg cctcctatgc cttctttccg ggcctgtttt aagagcattt 87000
tcagaataca cacagaaaca ggcaacattt ggacacatct cttaggtatg taatgtcagt 87060
gatgtaatga gctggtgatt cactttcttc ctttttattt tcatgtattt gagggtaagc 87120
acagaacttc agaaatgtat ttggatttgc cattttgttt tctgaatttc taatgatgaa 87180
ttttctgact ggtttactcg tagtttatcc tggtttgcac tatgattttg ttataactta 87240
agttatattt ctttctacta gattttcaga tcttgtcctc aaaccaggcc aagtaaaagt 87300
tgctatacaa aatgtggttt catattgtag tgaccactga gaagaaagta ttatttaata 87360
gaagtaatct gatcttttaa aatgcattac ttatatgcac tgctaatttt cagtttggcc 87420
tgaagtagaa tatggactgt aatttttctc ccataatgat tgtatattaa agaaagaata 87480
ctgtagagga aagaatagtc tttccaacaa atggtgctgg gataactgag ccatatgtaa 87540
aagaatgatc ccgaactcac atatgcaaaa gtgaacccaa aatggatcaa agacccaaac 87600
ataagagcaa aaactataaa atccttagga gaaaacatat ataaatcctt gtaagtttgg 87660
attaggtgac tgtttctgag aattgccacc aaagaaaaaa tagataaatt ggacttcatc 87720
aaaattaaaa acgttggtat atcaaagtgc attatcaaga aagtgaaaag acaacccata 87780
gagaagagaa tatttacaaa tcagctagtt ggtaaggatc taataaataa ctcctacaac 87840
tcaacaataa agacaaccga ttttaaaaat gggtaaaaca tctgaataga tgttcttcca 87900
gagaagatct aaaatggcca ataagcacat gaaaagatgt ttgtcttcgt taggcattta 87960
ggaaaacgca aatcaaatgc tttgagaaat gcagtgagaa atctcaacac ttcacaccca 88020
ctaagatggc tataatcaag aaaatgaaaa ataacaagtc ttggcaagga tgtggggaaa 88080
ttggaacttt catacgttgc cagtaggaat gtgaaatggg gcagtcccca tggaaaagtc 88140
tggcagttcc tcaaaaagtt acatagagtt accagaaatc aactcctagt atattaccca 88200
agatgactga aaacatgttc acataaaaac atgaacacag aagtttgcag caacattatt 88260
cataatagct aaaaagtaga agcaacccag atgtctatcg actggtaatg gagaaacaaa 88320
atatggtgta ttcatacact ggagtattat ttagctgcaa aaagggatga agcactgata 88380
catgccacag catgggtgag ccttgaaaac atttgctaag tgaaagaagc cagacacaga 88440
aggtcacatg tcgtttgatt ctacatgcat gaaatgtcca gaacaggaaa atccataaag 88500
acagtagatt agtggttgtt tagggctgta aggagtgggg aatgggggat gacagcagat 88560

acagtacttc tgtttggggt gatgaaaata ttttggaatt agttagtggt gatggttgta 88620
caaccttgtg aatatactag tttctggcct tactccattt gatttgcatt tcttatcaca 88680
catttgattt ccatggtgcc tctaagttcc tgcctgctag tatagtgaga ggagagtaga 88740
gggtcaaatc aagtttgttg ctgacttcta attcaagggt aatttttaat taaaatgata 88800
aaacaattac ttagcaaacg ttgaagttga aatttctgtg cctgccttgt gttaggtggc 88860
ccctccctaa gcaataggaa catggcatct ttctgtgtag aacagtgtaa gaattggtta 88920
ggaaatggcc tgagtaggca ttttgtcctt aactcttgaa tctcctgaac cagctgatga 88980
gggccagaga aacagcatga cactccctga cagctattgg tagaggaaag ctgtatttct 89040
tggcagggtt taggggtatt ggaattcatt gtaaaaacaa aagcaaaagc acaatggacc 89100
tttgaaagct ggactatgag gtatgtgcca aaaactgtat aaatgaaact attctacatc 89160
tagaggtaga agtggcttca ttatatcttc taagaagtag cagcagtgtc tgatttcttt 89220
ttagattatg cccttagata agtttttata ttctgtatat tcagtttatg aagctgaagt 89280
atttctgata ccaacaatgt tggaagtaaa ccacaatttt ggataggctt cagttatttg 89340
gaagattttt ctcattgaac ttttgattca gatattgttg taatgcagaa ggattgaaaa 89400
cagaatcagt tataatactg tctcttctaa agggtgatat tttatctatt ttctgtgctc 89460
tttttcctag gttgtgtatt cttcctgtgc ctggggatct tttatatgtt tcgcccaaat 89520
atctcctttg tggcccctct gcaagagaag gtggtctttg gattattttt cttaggagcc 89580
attctctgcc tttctttttc atggctcttc cacacagtct actgccactc agagggggtc 89640
tctcggctct tctctaagta agtatctgta aagtccgtat tttggccaat gatttagagg 89700
tagtgcgtta gggaaaaaca ttcagcagag ttggcagaat tcttaatatc atctcatgca 89760
aacttttttt tttttaaaaa ttaaagagca acccagtttg gctcttaggt gtcgttgatg 89820
gcttatgtca tgagggatta attggaacct gtagtggcca gtagttttta aattactgca 89880
ttaaatggat tttttttttc tgtcatagac tggattactc tggtattgct cttctgatta 89940
tgggaagttt tgttccttgg ctttattatt ctttctactg taatccacaa ccttgcttca 90000
tctacttgat tgtcatctgt gtgctgggca ttgcagccat tatagtctcc cagtgggaca 90060
tgtttgccac ccctcagtat cggggagtaa gagcaggtaa gagcacgggg aggttctaca 90120
ttcgacattc atttattcac tagttaaatt cactatttat taaagttcta ccatttgcca 90180
aacattatgc tgtgtatttg tgatgccaag atgaagaaat catagttcct attgttgggg 90240
gtttttttat gtagtagtcg tagtagttgt tgttgttagt agtgtttatt aattgtctta 90300
aagcattagg gttgaggggt catggtgagt atgaggtgat ataatttggg actgttactt 90360

acttgaattt atggctaaac accatccaga aagcggtagt ctataacttt tgtaaacaat 90420

gatttctctt actcattgtg gagtgtactt tttgtagtga ttcttttttt gttgtcattt 90480

tctgttttgt tttatatttt tattttataa tggataatct gcttaaggct tcacccccat 90540

gctgattatg ttttgttaaa gtgggcacca tatctccctt ctgatgtagg gataaagggg 90600

ctttttatat ttaggataat gcctgtttag ctggagatca tagagaaagc cacaagcccc 90660

tcagtgcctt ggctgagtct tcttgtagga catggaatta gcagagatgt tct[?]gtctcc 90720

tactcagtgt caagctcagt gcccactgag aatgaacaag gagtgttttt cctttcgtcc 90780

ttaaaccccc aaaaccattt agta[?]taaaa tctaaaccct ggatcatccg ggggtacctg 90840

gaaggttaga cacagaagta acctcatgtc agtgaccact gttaaaacac tctgccgtcc 90900

tcctttttgt gttcatatga gcaaatgagc attggtcagg gtcagctttc aacagtactt 90960

gagtagtaca gtggtgttaa gcctgcttga gcacatgaga taagaatgtc tttgctgagt 91020

acagctgagc a[?]tctgttat ttacatacat cagaagaacc aaattcctga gcctttgact 91080

tagagaatga taggcagaaa ccattagcag cctttgctta gcatgtagag gaaaagaaaa 91140

agtgaatagt gcaaaagtct agcaaatctt tccttagagg agcagaggta ggaaatagtt 91200

tttctttagg tctgcttggt tcttacctgt tgtgagagca gcattgccat tttcctcttc 91260

tcttttagat gtccatttta tttgtgagta tgcatcccat agctgtgctt acatatattg 91320

ctgtatacca gcagtttatt tggtaatgtc caaagcaagt aataggttct ttatctgttg 91380

agaaagctta ccaaatgaca gcttgttttg caagggtcat tgttaactct gtgtcaaaat 91440

gtaggagcag ttactggcag aggcagggag ctcctgtgtt gctt[?]ggctg atacacgggt 91500

ttcaaagtgg atttagatca gtggacttca catttttttt tcca[?]catga cattttctat 91560

tacagtcctg aacacgtgta tctatacata agttcatgta tata[?]aaatt acaaaacttt 91620

aacaaaatag tatttaatcc tccacgtatg ttacagtctg atattttaaa ttttattcta 91680

gttcatttaa aaaaatgctg gttgagactc atttaattga tct[?]agaact taggtcatat 91740

ccagaagttt aaaaaaacat tagatgcttc tggaggcagt aaggcagtag ggctgtggct 91800

gagtgatttg ctagactttt gtacttgatt gctatttagg ggtctgggag cttatagtat 91860

tct[?]tcccat cccccacccc cattaggtca tttgttaaag gcatttaaca atttttaagt 91920

gccc[?]agttat aggttataga tttagattaa atttttttctt ctttttttctt ttctttttctt 91980

tttctttctt tctttctttt tttttttttt tttttttttt ttgagatgga gtcttgcttt 92040

gtagcccagg ctggagtgca gtggtgccat cttggctcct gacctcaagt gatctgcctg 92100

cctcggcctc ccaaagtgct gggattacag gtgtgagctg agccactgcg cccagcctta 92160

gattcctatt aaaaataggt gatgccaggt gatgtgatca ttcatcgcct ctccacccca 92220

acaacgcctg ctgcttcaaa gagaagatgg gatacgtaaa attgcttgta ttgtaacctg 92280

tgtctgtaga aggacaggct aggacagttt aaataaactc ctggagctgt cctttccgaa 92340

aacatttagc aacattaatt caaaaccctt gaagactgct gacttactaa gctaagcctc 92400

tcttagtgag gctcattgct tttttttttt ttttttgaga cggagtttca ctcctgttgc 92460

ccaagctggg gtgcaatggc gtgatctcgg ctcactgcag cctctgcccc cggcttcagg 92520

tgattctcct gcctcagcct cccgagtagc tgggattaca ggagtgcgct accacgcctg 92580

gctacttttt ggatgtttag tagaaatggg gtttcaccct gttagccagg ctggtcccaa 92640

agtgctggga ttacaggtgt gagccactgc acccggctgg ctccttgcat ttgtaaatct 92700

ttagaacata gctgtgttgt cttgtgtgac agtgtcccag agtttgaaga accttctttt 92760

tcctttaaaa ctggaagcta attttatatg ctactttgaa atattcagtt tacagagtta 92820

atggtgctgt gctgtaatac tgacttctgg ctctttgttt ctcttctgca ttactttact 92880

ctcttcttgt gactcctagg agtgtttttg ggcctaggcc tgagtggaat cattcctacc 92940

ttgcactatg tcatctcgga ggggttcctt aaggccgcca ccatagggca gataggctgg 93000

ttgatgctga tggccagcct ctacatcaca ggagctgccc tgtatgctgc ccggatcccc 93060

gaacgctttt tccctggcaa atgtgacatc tgggtaagta tgtcggggtg actgagtgtg 93120

taggtatctg ctcatgagtt attggcatcc tgcagagtga tgcaatagaa aaagttttag 93180

acacaaagtc aggagagttg tatcctggtc ctaactccct attgagcttt ctctggacaa 93240

taagtacaat aataactaac atttagtgag cacttccttg gtgctcagca tggtacagag 93300

tgctttgcac ggattacctc attacataca ttagccctat gtggtatgat aactcttgtt 93360

aatgtcttca ttttacaaag caggagacta aggcttaggg aggtgaggcc acttgtccaa 93420

gccatgtggc tatttaagtg gtagaacagg aatttgaatt aggctagttt taatatcaga 93480

atctgtgctc tttttgacaa tggcaagtca ttttgccatt ctttgtctca gtttccttat 93540

ttatgaaatg aggatgatag accagatttt caccagagcc ctttttagga ccagatttct 93600

atcagggtct ttttttatac cgaaatttag tgatttgtaa tgggagactg agtgtatgta 93660

tgtctggtgg aagaagaggg attgtgaaga agagggaact ggattgaact ggattgtggt 93720

cattggtaag agaggaggaa agagaagagg gaaaagaaaa tataaggact gatatagata 93780

gaaaaactta taaccaaaga gggcctgaaa tattttcaac aagacaacat tggaaatacg 93840

cttaatgtga aatctgacct ttgtaattct gttcctcagc ctattgctga ttattataga 93900

ctaacctgat gatctcatct ctttaaagtt tagtttagct atctgtaaaa tgaagatgat 93960

cttgaactta agcaatcttt ctagcttcaa attagctaat ttatctttct gattcatcat 94020

catcatttta acattggtat ggaggggttt ttgcccttta tcccctttct ttgggggaaa 94080
ttaatcccct ttggtttcct aggaaacatc tcttgggatg tgacttgatt tcttctagtt 94140
tttgcttcta aaatcagtgt ttttctcttg ataaatactg ctttgccact gtactttttc 94200
attttgagat tataatgaca gaatagtgta atttttgaag tgcagcgagc aattgattac 94260
ccttcttgag atgtggaata gcattcaaca gaacaggtaa aatcagaaat aatgtcactt 94320
gcttgtagac cagcttctga cttgagaagt aaaagggcct gtttgcttag tttttgacac 94380
attatgtagt tcatgaaatt tgagaatcgt agcactgaag gaaactctgg ggaccatatg 94440
ttgtgacatt ccacacacca tcctggaagc ctctttagtt tttctaacag agagcaacat 94500
ttaaatgacc atagctctag tcctcttgtc catgacaaaa tgtccatcag cagtgagtag 94560
gaaaaggact gtatgtgtaa tcatagccca tggctaggtg tcaagggatc cttgggacct 94620
ttgtggcctg ttagcaagag agtctgtgtg tcatttgcca ctttagcaag agagtctgtg 94680
tgtcatttgc cactggtttc agtgttaagt ttgtagattt ttatgtgtag agcttagtgg 94740
cagcaccttg gtttctttgg gaacagctgg ctttcagctc agcagaatgt gttacaccta 94800
atggatgttc caggatctgt ggacgccttg aatttgagct ctgtcacatc cattccctgc 94860
cttctgctca gcataaacca ggattaaacg atcctaagaa tctttagcag tcttaataat 94920
gtatgggttt ctctaccccc ttctcttctt ttttcccctc cagtttcact ctcatcagct 94980
gtttcatatc tttgtggttg ctggagcttt tgttcacttc catggtgtct caaacctcca 95040
ggagtttcgt ttcatgatcg gcgggggctg cagtgaagag gatgcactgt gatacctacc 95100
agtctccagg gactatgacc ctaaaccagg gcctgcggca cttgcgggcc tccctgctgg 95160
ctactgatgc cagtaccaga ggagccccaa aactttgaca gcctcgtggg ctttgtgacg 95220
gcccagtggc tctgcgtggt acatgactga gaagagaaaa acaaaaataa atcatacctc 95280
aaaggatgga gtgcatcaat tgggagaaaa ggagacatag cccaaaccct ggcttattct 95340
tgggatctac tgattgcggg ctctgcaaga cccttggcaa actggcttct gatccatatc 95400
atatttattt gtagaagatg gcgaaacagt ttagctggtg gttctttctt ctccctttct 95460
ctctctctat gacaataata caaaccaatt taagtgaaca tttatatccg ataaggggtg 95520
ggagtgtgat tttaaatgct cttttgggag aacaaagaaa ttaatgtaaa taagatttct 95580
aactgtttaa ataagacttt atataaatgt ttaaaacata ggggtaaggg agggagggag 95640
aatttttgta tagaatgaaa catgcaagta ccacacactg tttgaatttt gcacaaaaag 95700
tgactgtagg atcaggtgat agccccggaa tgtacagtgt cttggtgcac caagatgcct 95760
tctaaaggct gacatacctt ggaccctaat ggggcagaga gtatagccct agcccagtgg 95820
tgacatgacc actccctttg ggaggcctga ggtagagggg agtggtatgt gttttctcag 95880

tggaagcagc acatgagtgg gtgacaggat gttagataaa ggctctagtt agggtgtcat 95940

tgtcatttga gagactgaca cactcctagc agctggtaaa ggggtgctgg aggccatgga 96000

ggagctctag aaacattagc atgggctgat ctgattactt cctggcatcc cgctcacttt 96060

tatgggaagt cttattagag ggatgggaca gttttccata tccttgctgt ggagctctgg 96120

aacactctct aaatttccct ctattaaaaa tcactgccct aactacactt cctccttgag 96180

ggaatagaaa tggacctttc tctgacatag ttcttggcat gggagccagc cacaaatgag 96240

attctgacgt gtccaggttt ctcctgagct catctacata gattggtaga cccttccttt 96300

ggattaggaa agatgagttt tacctctggt acactgtctt ggtaagcctg gatgtgacag 96360

acacctcggc tctccttgaa taagaaagcc agcagaactc ttaaagccag ttgtagtacg 96420

cagttgtcag cactcactga acctcacttt acagggataa gagtggtgtg gcattttaaa 96480

tacaatggta tgttattgcc agggagtgag gtacaagacg atggctcatg tcacaggcct 96540

acctgatacg gtgtcagaga aagtggtggg gaaaggatct ggttcatgga attctgatct 96600

tggcccatag gtgaaccacc aaaatagtgc tcgagtctta ggttactgtc atcaaagact 96660

tgggatgact ccattatatc ctggggttgt gggtattaga actaaatatg gaggtcctga 96720

gcatggggac tggcgtcctc agtaggtgtt tgggaatatg ggaagggtct cctatttatt 96780

caatagagtt ttctcagtta ttttcctccc ttgcccttgc aatctccagc aaaaggtggg 96840

atctaggaag aaagaatcca gtgtagaagt tgagaagaac ttgaacgttt tggttctgga 96900

taaggtcact gtcctaggtg ctaggtggac cgagcaaaag actcagtgga tgaactggtg 96960

cagtgcctga cagaataaag aacagtatta atccctttga gaaagcatag tccagcagga 97020

cagtggccat ttggacagaa gcccacttag tttcttggga gcaacagcac gtatcagaag 97080

ccagacttgc tcttcggtca tgcactttgg gatacagcgt ataggtgcag ccctgtcaca 97140

acaccaacag aagtagcagc ctctgggtgc agtcacccac accccaaagc tggaaggatc 97200

tggttcaaca tagcacaaac ccttaggaaa aatgaaatta acatcactga tgtgtaatcc 97260

agtaaaatct ccctttttcg ggtgtgtatg tgggcatgtg cccatttcta tgtgtgtgtc 97320

tacgtgcagc tcactaccaa cagcctcatg tgcacttgac ctgacagtgc tcgctgagaa 97380

ctctcaccag gttggcgcct gaatgcctta ctctcagcag tcagaggctt gcttgctctg 97440

tgcagatttt taattttctt ttttggccct aggctggttg ggacctctac agcttcattc 97500

tttcaccatt aaatagtggc ctttttcagt attttccctc ttccccttta taaattatgc 97560

taaagccaca aagcacattt ttggggatca tagaaggttg gggttccaga aaggcatctg 97620

tgtgatggtt ccattgatgt gggatttccc tacttgctgt attctcagtt tctaataaaa 97680

agaaccaaat gaaatatgac ctgtcgtgtg tttttttatt gccgttcagc ttacctcccc 97740

ttggcttttg gcacttctct gaagctctaa tttagacact gtgatttaag aaatttttg 97799

<210> 11
<211> 16994
<212> DNA
<213> Homo sapiens

<400> 11
tggtactggc caagttttct ctaaaggata aagcccgaac tcagcccctc agacagtgaa 60

taagaagttt ctttttttct ttctttcttt cttttttttt tttctcgaga cggagtctcg 120

ctctgtcgcc caggctggag tgcagtggtg agatctcggc tcactgcaag ctccgcctcc 180

cgggttcaca ccattctcct gcctcagcct cccaagcagc tgagactaca ggtgcccacc 240

accacgcccg gctaattttt ggtatttttt tagtagagac ggggtttccc tgtgttagcc 300

aggatggtct cgatctcctg accccctgat ctgcccacct cggcttccca aagtgctggg 360

attacaggcg tgagccacca cgcccggcca agaagtttct tttctgtatt tttacttttt 420

tttatttttt gagacggagt ctcgctctgt cacccaggct agagtgcagt ggcccaatct 480

cggctcactg caacctccgc ctcatgggtt cacgccattc tcctgcctca gcctcccaag 540

tagctgggac tacaggcgcc caccatcacg cctggctaat tttttgtatt tttagtagag 600

acggggtttc accgtgttag ccaggatggc ctcgatctcc tgacctcgtg atccgcccac 660

ctcggcctcc caaagtgctg ggattacagg cgtgagccac ggcgcctggc ccaagaagtt 720

tctttaaggc agctccagct ttttcagtca aagtgaaatg gtcctgaaat ttgcatatgc 780

aatgtttacc tttctttttc attaaacgag agagggaacc aaaacaaaaa tacagtttga 840

ccatttaaaa tgttggaaga gctgccaact tggtagcact atctagagtg gctccattct 900

gccatctagt ggaagggaaa agacaaattt agaccaaaat acttccatct ttaatttaca 960

aaaaagtgtg ttcttttgtt ttttgttttt taaagttggg gtcttgctct gttacctagg 1020

ctggagtgca agcagtgtga tcatagctca acagcctgga gctcctgggc tgaagccatc 1080

ctcccacttc aaccttcctg gtcactggga ttacaggcat gagccgcagc accaggctgt 1140

gttttctgat tagcataatt taaattatct actaaatttg ctcgttaata gattcacttt 1200

gtaaagaaaa accacacagt attctaaaca ctaagcagta aaactaaatt tcccaaaaca 1260

tttatattta taaactatga atttcaaaga tctgcagagg aaagagagaa cgttcttgag 1320

ctcttattat cataaatttt cctgcccctc cctttcccgt ttatattatc catttctaca 1380

catactcttt acgtttattt tggcacatag actgggagaa gcccagagtc tctgaggatt 1440

atatctttcc caaaatactg agcaaaatca gtgggtgagt aagatcattc cattcatttt 1500

tcaaaaatgt acactcactt aaaatgttga tgtatccggg catggtggct caagcctgta 1560

atcccagcac tttcagaggc tgaggcaggc ggatcacaag gtcaagagat tgagactatc 1620

ctggtcaaca tggtaaaacc ccgtctccac taaaaataca aaaattagct gggcatggtg 1680

gtgtgtgcct gtagtcccag ctacttggga ggctgaggca agagaatcac ttgaacctgg 1740

gaggcagagg ttgctgtgag ctgagatcat gccactgcac tccagcctgt tgacacagtg 1800

agacttcgtc tcaaaaacaa acaaacaaaa aagttgatgt aaccaaggta tttgttaagg 1860

cctgtgtgct tgaatttgtt ttttgctggt gtatgtatgc atgtttgctt tgttttgatt 1920

tctatggaca ttatctaggt atttatgatg atttccattg cctaggatga agacatctgc 1980

agccaaaact ccctgaaaag ttagtaacag tagcatacct gaagtgactg gtgctattct 2040

gaggctgtta ctcttttttt gaaacagggt cttgctctgt cgcccaggct gagcgcgatg 2100

acatgatttc ggctcactgc agccttgacc tcccagactc aagcaatcct cctgcctcgg 2160

cctcccaatg tgctgggatt acagcacagg ccaccacacc cggctaattt ttaaagtttt 2220

ttttagagac ggggtctccc tatgttgccc aggctggtct caaactcctg ggctcaagag 2280

atcctcttgc cttgacctcc caaagtgttg ggattacagg tgtgagccaa cacatctggc 2340

tggttattac tcgtattact agcaaatgtt atttattgag tttctatggt gtactagaca 2400

ctgttctttt tttttttttt tgagatggag tctcgctcta tcccccaggc tggagtgcaa 2460

tggcacaatc tcgacacact gcaacctcta cctcccaggt ccaagcgatt ctcctgtctc 2520

agcctcccaa gtagctaggt ttataggtgc ccgccaccat gccaagctaa tttttgtatt 2580

tttttttagt gcagacaggg tttcaccatg ttggccaggc tggtctcgaa ctcctaacct 2640

caggtgatcc acccatctca gcctcccaaa gtgctgggat tacaagcgtg agacaccctg 2700

ccttggccag acactgttct tgatgtacat tttgctaatc cttccactaa atctccaaag 2760

tagatatcat tgtcttcact ttagaaataa aaaaatgatg cttatgacca cctaccagca 2820

aatcttgatg cgccagggac tggggggaat aatcattgac tagctggcta ttatgagtca 2880

cagtttccat tcctgcaaaa tgagaatttt tttgacttag gggtggggtg ttctctaagc 2940

tttttgcctg attctaattc ttctgctaga agctgtgggt ttacagaggt gaaaaagata 3000

gcccttccac caaacatata ttctattata tatatcttat ctatatatat tatatatatt 3060

ctattatatg tatcttatct atacatatta tatattctat tatatatatc ttatctctat 3120

atattatata tattctattt atcttatctc tatatattat atatattcta ttatatatcc 3180

cttccaccaa aaatatattc ttttatatta ataaaatata aacccagggt ccaatggaaa 3240

agacttaagg gcagacacag agtgggatgt ggtttgtgtt aggagagggc tgaagggaca 3300

tcatggcagg catttgtgtg acctcccaca aaacaccctc ctccactctg acctcacttt 3360

gttcttcctt ccctttgtcc ttattatttc ctttctgctt ctctttcttt tctgtctccc 3420

tctgccctgc cctagaacgg tgcctttcta attgtgggtt ataacacatt attagtaggt 3480

cataaaatca attcagtgga tgtggaccag cattttaaaa agggaattaa tagaatataa 3540

actccttaaa aggcgtcact catactcagg ataatactgc tgtgtaaaat ttgttttaat 3600

atgtgtcagc atgtgtacac tgtgtcaaga tgtaacactt acatcctatt ttaagaccct 3660

gctgtagact aaaccaggac ccccatggtg ctccaggcgt ccccaccctc acctgtggca 3720

ccaggagctc ctgctgtcac tggcccagct gggtcctgtg tctttctctg cgttgtcagg 3780

gcctgcgctg gcagtccacc tacagggagg tggaatccgg ggcttgacgg cactgggagg 3840

atccttcatc tttccaggct caagcaaatc ctcgcctgga ggcggggaat gggctagatg 3900

acccctgaaa cagtccagtc cagcatctga gagctcctct gggtccagcc ctgtgccgcc 3960

tttccacgct tccctgcagg gccctgggga gagcagagaa agcagctgcc agctccaggc 4020

agtcatttta ccccaggtgc cacttaccct gcggaggggc tggaaccagc taatcatttt 4080

accccaggtg gcacttaccc tgcggagtgg ctggagcttc tcaggttttt cagttcctgt 4140

gtcacctccc agccctgccc accacatgct gtgggtgtct atggctgacg aacgcccctg 4200

agccaggcca acccccagcc tcccagtggg tggctctgtg gtccctggca gccccggcat 4260

catccggagc acagaggcca tggacactca ggggatggtc aaggtcgtag ctccctccgt 4320

tcttccgttc ctccctcaca gagctttcag caagatagat ggtcattctt ctgtcctcac 4380

tgctcccggc ataaggggac cccagggctg gatgacttcc ctaaagagga atctctcagg 4440

gtttcctgca gccgctccca agtcccgtct ggaggatgaa ggtggcggac acatccctga 4500

cgcagtttct cctctgccct ccacccctca cccatcccac ctcctggact aggaactcaa 4560

ccaggacttg cttcaacctg agaagatggt gtccagcccc cttggagcct gccagcatcc 4620

ggcgggattt cagaggggcc tccagggagg cgctgcccag ctgcccccag tgttgctcac 4680

acagccccca ctgatctgct ctggggaggc cacggcccca gaacgcggct tgtggggggc 4740

ctggggcagc acgccgggag acgtcacttg tccgctgcct gctcacccag ataactgcag 4800

ctccctccac tgccgcctac agccctcccg ccatgcctct gcccagaagg ccctccctct 4860

ccagtcctgt ccgcccctca aggcctgccc aaacctccct gaattctcgc ctgagcagac 4920

actttcctag cacggctccc gccgcgtgga ttactcagcc gagcctcagt ctggggctgc 4980

cctgtgctgg gagctttatg cagatgctcg gctccctacc ctggcttgtg agccgtgtgg 5040

cgcagaaacc gcatctcact cagagcgagg gtaaagagca gcagggctgg agaagccagc 5100

tgccctgcca caggccaaga ggacttggcc aagtgaccat tgctgtctgt ttccccaact 5160

gtaaaggggg ctgagaccct tacagggttg tgaggcttaa actttgacaa acgcctacca 5220

tcccaccagg cacagggcag ttgctgagta tttggcggtg gctgctgttc cccacagtgt 5280

ccaacacctc ccggggatgt aacaggtgca ccacgggcaa gactgatggg tttatcagtt 5340

cactgcggag agaggcaggg atggatgcac ttgcttgttc ctcttatttt cccttatggg 5400

gagcagtgac acttggcaga ggctgccagg gagatgggaa atggacatta attacgggaa 5460

agaaaaggag gcaagaaggg gaggagggtg ggaaatagac aggggaggaa gaagggacgg 5520

aggcatggtt agaagacagg caagcaaaaa agacgactgt tttcttggac gaacagggag 5580

gtagggccat gaaaagcatg tggacgcttc ttctcagtga attaaggcat ttcagcgctg 5640

gaaagagcct gaaaaaatca gactcccttc acagcccagc ggagtgcagg ccctgggaga 5700

gcaggagtta gagatgggga ggaccccgcc tgcaacaggc actgcctgct acaccctgcc 5760

atgggcagag gggctgtcct cccaccccag agaggcccca tcctttcctt tctttcccca 5820

aggctgctcc agcagggagt ttagctccat ggacaccttt gaaggctatg aaggtgcaga 5880

agacatggaa aaggtgagca gcgctctgaa cgtaatttct ttctttcttg tttatttcat 5940

cttcttcctc cttcctattc atcagctcct cctgcctact gtccctcgcc tccatccctc 6000

tccccctctt caggattctc acccataagg acctggttaa gccagggaac cgaggtcact 6060

agtcctcaaa gtggctctga ccctgctagc ccggcatgtc cccacgtggc tgtctccttg 6120

gcggtgcgct ggtgtccggt cataactggg gctcccgctg agagccccgt ggcccaggtg 6180

ggagggctgg ggtgggcctc agcctgccag cggaagtcac cacattgccc ggcctggcca 6240

cagcctccag caataactct gagggtgcac ccgtcccccc ccccgccccc ccttccagct 6300

cgcggggcct tttccagttg gatgacagtc acgaaacctg ttcctccaaa tccgctttcc 6360

tggagaggag agaaagcacc cagggagaaa aagaagaacg cgatgaggaa gcaacgtgag 6420

tgtgagcgag gaaaactaag cgcctctgtg ggccacggcg tctctgactt tcttctcgga 6480

attgcagggc gaccggcagc accaagaagg agcgtgctgg ggcgtccacg ccggctgact 6540

cctgcggggg cgacggctgg tttccaggcg agggcgcggc gcccgaggac ctccaccccg 6600

gagagctgcc ctccctgcgg tcgccccggc tccccggctc cagagcgccc gcattcccga 6660

gagaggcgat gtgggggccc gggccctgcc cagctacgca gaaagcagcc ggggccgcgg 6720

cggcggcaga aaggggacgc ccagggccgc ctcccgggag cccgaggggt gccctgcgtg 6780

ctcgtctagc tcccgccgcc cggcgaagga cctcgcggga cggggacccc tagtgcgcct 6840

gcgcgagcgc ggcgggcgga ggcagacgct cgcggaggcc acgcctccta aggctcggcc 6900

gagcctagga ggagccggag ctggctcggg gctgctccca cccgccagcg ccggctgccc 6960

agcctggcag tggaccgggt gtgcaagcca cttagcacct gacaggttaa ttcagagggg 7020

gtggttccaa ggtaactgct gagagattta cgtttggatt tgttagttta aaaattagtt 7080

ttccagttac ccgggctgga gtgcgatggc gcaatctctg ctcactgcat cctccgcctc 7140

acaggttcct gcaattctcc tgcctcagcc tcccgagaag ctgggattac aggcatgtgg 7200

caccacgccc ggctgatttt tgtattttta gtatagatgg gggtttcacc atcttggtca 7260

ggctggtatc gaactcctga cctcaggtaa tccgcccgcc tcggcctccc aaagtgctgg 7320

gattacaggt gtgagccact gcggcctgcc gattttcaga attttcgtca gaggtttgca 7380

tctgtttagt tttttagtta tcttcagact tgtcatgttt tcttttcata agttgcaaat 7440

taacttttct gtttttaatt tttacttact tagttttaag gtgtatttac tttcagaatt 7500

tcttaggttt aacattgctg tcagtcaggg tccaatcact gaaatagacc ccacaccaat 7560

tattttaaca gaaaatttaa tataaggaat tgtcacacaa ctattggaga actaaaaagg 7620

caaaaaaaaa aaaaaaaagg atgatattga gatatcatgg agatttcaac tgcaagaagc 7680

agctactacc tcaagagcta agggaacaaa aagaagttgg aatgatcaaa actcagaagt 7740

ctgaagatgg ccccagggct ggggttgttc tttgcggagg agacactgct cagagcctgg 7800

ggtctgggag ctggatgcag aactcgggag gggacatggc cagctcgtgc tggtgtctct 7860

gaatgggcat catcaggctg gttatgggag tgctgggaaa atgacaagct ggaaccaact 7920

gctgctactg acaccacctg tcattctcag agtggagacc tgttgctaga acgctgacag 7980

gaacccagga gcacaggaag gagcccatcg tgaccctgca ccacagagca ggggcggggg 8040

tggggctggg gggcaggggg atgtaaaagc tgaggaccat cccagcacac ttgctttgga 8100

gttacgtaga gctccgttag gattcagtca gttttggaat ggttaggggt gatttcattt 8160

tttcactaca tgtgggatct ggcagaatct tcagtctcct ggtcttgttt ctggaatgga 8220

gtgggtcacg aacacctcac aggagtctct cccaggagac ttgagaggat cactttagaa 8280

tttaagaaaa caaaaataac atgtctcact ttgttcacaa agaagccctt tgacaaagtg 8340

gccaaaaacg ggcagagcct aaggaacaga ctcttaggga ggggtccttg gaacagtatc 8400

ctgcagagcg ttcctctttc aaattcttta gaattttacg ctgttttgtg gggccaccct 8460

gtcccactcc ctttcctaac aacacagtgc ctttcttgct ttgaagttgt aaactgtttt 8520

tttaattgtt tgcacaataa aataaagttc ctcccaataa gggacagact cttagggagg 8580

ggtccttggg acagtatcct gcagagcgtt cctctttcaa attctttaga actttacgct 8640

gttttgtggg gccaccctct cccactgccc ttcctaacaa cacagtgcct ttcttgcttt 8700

gaagttgtaa actgtttttt taattgtttg cacaataaaa taaagttcct cccaataagg 8760

gacagactct tagggagggg tccttggaac agtgtcctgc agagtgttcc tctttcaaat 8820

tctttagaac tttatgctgt tttgtggggc caccctctcc cactgccctt cctaacaaca 8880
cagtgccttt catgttttga agttgtagac tgtttgcata ataaaataaa gttcctccca 8940
aatcaataat ctgtaatctt aggttcttct acctgtatta tccttcagcc cacatttttc 9000
catttgacca tcagaaagtt tacatatgta tgtacatatg tatgtacgta tgtatgtatg 9060
tacacattcc ctttattcaa tacctatttc ctgagcactc tccttccctg aggacgggtc 9120
tggcaccagg tagagaccag ggaggtatga agatgagtga agcattcagt ccagtggggg 9180
agacagatgt ggaaacagaa tcacaacacg atgtggcaaa ttctctggga gcacagaaca 9240
ccatcactga ctcttcctga tacagtcagg aaggggtgac actggaactg ggtcttacag 9300
gcaagtgtct tctcctggtg gaggatgaga agggcaccct ggcggaggga atagcatgag 9360
ctcaggtaca ggggcacgga ggagctgtgc atggcaggta ggacgagttg atgggggctg 9420
cagggttgca gggttcagaa aggcactaag cttaagggct gaccctggtg cttgacacat 9480
cctgcctcat ttgttcctgc agcaagtctg caagatagta gggagcaacc tcaggagtcc 9540
catgtttctc ctaacacagt tgacatttct ggatatctta agcgccttcc caactttctc 9600
cttctcccac ccttattttc atttgtctat tgaagatgag tccctccttc cttcgccttt 9660
tcactcctgc ttcttaaaca tatgtagaga tgaaggaggg ttgataccag caagctgtga 9720
aacacaggag acttcagctg aaaaaggagg tggagaagag cagagcagag gagaccagct 9780
tgtgggaatg gctgcataag ttctgcagac cagcatggtg aggagtgggc tccgggcatc 9840
accaaccatg actgcctcca actatattca gcaaccattg taaggtcatg aggatgcctt 9900
tcccaaaggg ggaacctgct gtagatccaa cccaggtttg tgaatgtaac agactggaat 9960
ttttaaaagc acacagagcc ctttgctgca ctaacagcaa tggacatgtc taccaggtcc 10020
aaaaaaaaaa atggtcctac acaatctgcg gtggtgtatt ttcacacttt taataataca 10080
cacctatgca ttccgtcttg gggtgtgtgt gtctgtgtgt gtgtgtgtat gtgactaact 10140
taacacactg aacgcattac aatgcattaa aaagttcttc agcagacttc tctgctttag 10200
cctctgctat ggtctaaatg tttatgtccc cgccaaattc acttgttgaa atcctaaccc 10260
ccagggtgat gatggtgtta gaaggtgggg acatagggag gcgatcgggt catgagggca 10320
gagccttcat gaatgggatt attatcctta taaaggagga gagacttcct tgccccttcc 10380
actgtgtaag ggcacagtga ggaagcacca cctatggacc ggaaagaagg ccttcgctac 10440
tggtgtcttg atcttggact tcccagcctc cagaactgta agcaataagt ttctgttgtt 10500
gttgttgttt ttctgagact gagtctcgct ctattgccca ggctggagtg cagtggtgtg 10560
atctcggctc actacaacct ctgactcctg ggttcaagtg attctcatgc ctcagtttcc 10620
caattagctg ggattacagg tgcgcgccgc cacgccaggc taatttttgt atttttagta 10680

gagagggggt ttcaccatgt tggccaggct ggtcttgaac tcctgacctc aagtagtcca 10740
cccgtctcgg cctcccaaag tgctggaatt acaggcatga gccagtgcgt ccggccagtt 10800
tctgttgttt ataagccacc cagttcatgg tattttctta tagcagctca aatgggctac 10860
aacagcatct ctgattatcc tataacaata tatccgtttc tctatgggaa tccatttctg 10920
tgcgagaata tgtaagaacc tcagatgttt catgccattg ctgaacagag gagtctcctg 10980
ggttctggac acttgccttg ggaagcacat cacttggttc ctctcaaacg catttgacca 11040
ctgaatagcc agccactcac tatggaaaca cagcccgaag cactctcaat cttttggacc 11100
tcagcccttt tctggatcta tgaaaatgtc catactggat ggtgctggca ggttgcgaag 11160
tgcccaaacc tcccacgctt cacaggagga accctctgca aatcttacat ggcaggtggg 11220
tgaccagagg agccattcca ataccagtca ttgctggatg tttctggggt gctgtcccag 11280
gtgctgtgga gcatatctac tctgtctagt ctgtctttcc attccagtcc agtggatatt 11340
gtgtcttctt cttcaggtcc ttgttcactg tagctgctca ggaaatagtt gtgaaatgga 11400
ttgttgttgt ggtacattct taaagtccct tgaaagtaag aaaataaacg aatgactctt 11460
ctgtcggtaa aactttggtc atcttttcac ttgtttagat gagaaagaat acagaacaat 11520
gagattatag tctcctaaaa cttttggaaa aattcttcat gtcctctata gtgttataat 11580
tttgctattg tcccccaaag ggactcaggt aacatgcttt ggatggaatg gtgggattaa 11640
aggcaccttt gattctgcac atttggtggt gttgtgaata caaactaaac actagctgtg 11700
cagtatgcaa tatttaaagg aagtagaaat catccaaatc agcatttcca gagtttttca 11760
gaacaagcaa gagaatacaa gagatcagca gttttctccc tcaagttgtt ctataggaaa 11820
aaaaaaattt atccaggctc aaagaaattc tataggaagg aaatctgcat acttttgcta 11880
accccatcat ttcccagact ctttgaattc agtaggaaga ccgcttgctc cacacagtta 11940
ttcaaggtcc ccaagattct tctatctggg ttttccatca tgccctagaa caatgtcttt 12000
atctgcatgg tcaaaactga cttacagtcg tatctgtggt cacagatagg ggaaggggac 12060
tgaggattac atgtgcaata ttttaagact caacctgcaa gtggcacaca tcactttctc 12120
tcacattcca ttagtgaaaa cttaatcatg tggtcactcc tcccttcaag gaggggtagg 12180
aattgtggtc cctaatgggg aggccatttc tacattattt ctgacagcta caagtctgtt 12240
aatgtgtatc atagttgtcc caccagcctg caacagagca ggggctagaa gccaggtctt 12300
ccaactccta gtccagagtt cccattacca cactagtatt tctgtacagt aagtcctcac 12360
ttaatgttat cagcaggttc ttggaaactg caattttaag tgaaatcatg tataatgaaa 12420
tcaattttga aatttctcat caatgttgta actgagtaac gttatttgag gacctgctta 12480

cataattttg cttgaagtcc aagtttccaa gaacctattg atgacattaa gtgaggactg 12540

actgactgtg ttcagccaac atatatggag tcctcctgtg tgccaggctc tatgtcaata 12600

tgagagtgga aagtcctaca aaacagaatc ccctaattat atatttactc tagtatcaag 12660

ctttattaat ggataaacgt tcagatagct ttgtaaagct atcccaaatt atctgatagc 12720

tttgcatgat gttcctttca tttgccaaga acatacatat tcttggtgag ggattaacac 12780

atataatgaa cagagtagag aatcctgaaa gagatcccaa tgcatttgaa aaatgggtat 12840

atgaaaaagt catttaaaaa atagaaccaa aaagataaat atacattaaa aaggagggtg 12900

ttgagtccac taactaagaa tatatatata tatatatata tatatatata tatatatata 12960

tatatagcta gatttccctt taattccctc ttgaagaagg aattgcttat gcaagactcc 13020

aaagctgaga accagattgt ctttcagttg tcatgcagcc tggaaagccc ccaccttatg 13080

taccaataga ttgtgaagca atcaaagact actacataca ctttaaagcc aaccagaaag 13140

actccttccc ttccaagtat cttttacccg attggaacta gaatatgtca ccatgaaaga 13200

acactcacca aagattggct taagtcttcc tccttttagt ctgtaaaact tactcttcta 13260

ggctggcgca gtggctcacg cctataatcc cagcactttg ggaagctaag gcgggtggat 13320

cacctgaggt caggagttcg agaccagcct ggcaaacatg gtaaaacccc gtctttacta 13380

aaaatacaaa aattagctgg gagtagtggc aggtgcctgt agtcccagct gctcaggagg 13440

ctgaggcagg agaatcgctt caacctggga agcggagctt gcagtgagct gagatcgtgc 13500

tactgcactc cagcctgggc aagagtgaga ctccatctca aaacaaacaa acaaacaata 13560

agacaaaaaa acttactctc ctttcaaacc ccttccaacc tctactataa tgaaaatgat 13620

ggcagctgac tcccttataa cagtaagttt tgaataaaca gcctttgatt aattctattt 13680

tgtttgccaa agaaaataat taaagtttgg taaaataaaa taattaaaag aaaaagaaat 13740

catggacact ctagaagaaa acatttattt gagctttact gctgaaaaag aaaacatatt 13800

ttaaaaagta accttgactg cacagcaaaa gaaactatca acaaagtaag cagacaacct 13860

acagaatgga agaaaatatg cacaaactat gtatctgaca aaggtctaat attcagaatc 13920

tataaggaac ttaaatcaac aagcaaaaaa caaataaccc catttatggc ctttgcttac 13980

tttttgggca gtcctcaaaa gaagacatcc aagtggcaaa caatcatatg aaaaaatgct 14040

tatcatcact aatcatcaga gaaaagcaag tcaaaaccac agtgaagtat catcacacac 14100

cagtcaaaat ggctattatt aaaaataaca gatgttggca gtgctgcaga gaaaagggat 14160

tgcttatgca ctgttggtga gaatgtaaat tcatttagct actgtggaaa gcaggagatt 14220

tctcaaagaa ctaaaaatat atatatcatt caaaccaaca attccattac tagatatata 14280

tccaaaggaa aataaattgt tctatcaaaa agacacatgc acctgtatgt tcatcgcaat 14340

attattcgca atagcaaata catggaatca accaaggtgt taatcaacag aggagtggat 14400
aaagaaaatg tggtacatat acaccatgga atactataca gtcagaagaa agaatgaaat 14460
catggccttt gcagcaacat ggatgcagct agggaccatt atcctaagta aactaatgca 14520
gaaacagaaa agcaaatgcc acatgttctc atttataagt gggagccaag tgagtacaca 14580
tggacatgaa atggaaacaa tagacactgg agactacaag agaagggagg gggacaggga 14640
ttgaaaaact acctattggg tactatgctc agtacttgag tgatgggttc aatcgtatgc 14700
aaatttcagc atcatgcaat atacccttgt aacaaacctg cacatgtacc acccctgaat 14760
ctaaaataaa agttgaaaaa aacaaaaaca aaaaaacccc ttggaatggg aggagtcttt 14820
tcagaccaaa catgagaccc agaagacaaa aagtaaactg ttaataagtt aggctgcata 14880
gcaagccaaa atttttatat gaaacatttt ttaaagtgaa aagatagata aattttcaaa 14940
acatataaca aagaattaat ttctttggta tacaatgagc ttttaaaaat taataagaat 15000
aattcaatga gaggttgaaa tgggtacaaa tatagagtta ggtagaagca taagacctaa 15060
gtgtttgata gatcagtagg gtgactatag ttcaagataa tctattgtac ctgtcaaaat 15120
agctagaaga gaacaattca aatgttccta gcattaaaaa agataaatat tgccggtgat 15180
gaatacccta attacttttt ttttttccag acagagtctc attctgtcac ccaggctgaa 15240
gtgcaatggc gcaatcttgg ctcactgcaa cctccgcctc ctgggttcca gtgattctcc 15300
tgtctcagcc tcccaagtag ctgggattac aggggcccac agccatgcct ggccaatttt 15360
tgtattttta gcagagatgg ggtttcacca tgttggtcag actggtcttg aactcctgat 15420
ctcaggtgat acacccacct cggcctccca aagtgctggg atgacaagcg ttagtcactg 15480
tacccgacct ccttagatct ttacacatta tatgaatgtg tcaaattatt acatgtatcc 15540
caaaaatatg tacattatgt gttaataaaa agattttcaa aattaataag aataattcaa 15600
taaggaaaat ggacaaggga agaaacacac aattccagga aaaaatacaa ataatcaata 15660
aatatatgat acatttaatc acatataata gaagaaatac aaataaaaat aaggcacttt 15720
taacacaatc tattagaata ccagatattt aaatttgaca gaattgagta ttgttgagga 15780
tatagagaaa tgggcactct catcctacat tgggagaaat ataaatcaat actggattct 15840
tgagattcag tttacaataa gtacaattta agatgtgcat atctctttac cctgcaattc 15900
cacttctaga aatttatcct aaatatttac ttacaaatga gcaaagacat atatgtacag 15960
ggatgttcag atttttatgt cgggtttttg tacggacata gttttttttt tttttttttt 16020
tttttttgag atggagtttc actctgtcgc ccaggctgga gtgcagtggc gtgatctcgg 16080
ctcactgcaa cctccgcctc ccaggttcaa gcaattctcc tgcctcagcc tcccaagtag 16140

ctgggattac aggcatgcac caccacacct gggtagtttt tctattttta gtagagacag 16200

agtttcacca tgttggccag gctggtctcg aactcccggc ctcaggtgat ttgcccgcca 16260

tggcctccca aagtgatggg attacaggca tgtgccaccg agcctggcta tatggacata 16320

gtttttagtt ctttgggata aatgcccaga agtgttactg ctatttaatc tttaaagaaa 16380

ctgccaaact acttttcaga gtggctgcat tattttccat tcccaccagc aaagtatgag 16440

taatctattt ttacgcattc tttcccaaat ttagtatttt cacttttttt tttttttttt 16500

tttttttttt tttttttttt tttttgaggc agggtcttgc tcagttgcct aggctggagt 16560

gtagtggcac tatctcggct cactgcaacc cctgcctccc aggcttaagc gatcctcaca 16620

tctcagcaac ctgagtagct ggggctacag gtgagtgcta ccactcatgg gtaatttttt 16680

ttgtattctt agtagaggtg gagttccgcc atgttgccca ggctggtctc aaactcctga 16740

cctcaagcga tccacctgcc tctgcctccc aaagtgctgg ggttacaggc gtgcaccact 16800

gtgcctggcc attctcacta tttttaattc aaaatagttt gtgttctcta ttctccacat 16860

ctttttgttc tttttaatca tctaaagtcc atagtttgta ttaggattaa cttactgttg 16920

tacattctct ctctctctct cttttttttt taatgagatg gagtttctct cttttttgcc 16980

caggccaagc gcaa 16994